# EUROPEAN PATENT SPECIFICATION

(11) **EP 1 814 871 B1**
(45) Date of publication and mention of the grant of the patent: **02.03.2011**
(21) Application number: 05817959.9
(22) Date of filing: 17.11.2005
(51) Int. Cl.: C07D 333/38, A61K 31/381, A61Q 19/08, A61P 17/00

(54) **COMPOUNDS THAT MODULATE PPARY TYPE RECEPTORS, AND USE THEREOF IN COSMETIC OR PHARMACEUTICAL COMPOSITIONS**
PPAR-REZEPTOREN MODULIERENDE VERBINDUNGEN UND IHRE VERWENDUNG IN KOSMETISCHEN ODER PHARMAZEUTISCHEN ZUSAMMENSETZUNGEN
NOUVEAUX COMPOSES DE MODULATION DES RECEPTEURS DE TYPE PPARG, ET UTILISATION DANS DES COMPOSITIONS COSMETIQUES OU PHARMACEUTIQUES

(30) Priority: 19.11.2004 FR 0412326
(43) Date of publication of application: 08.08.2007
(73) Proprietor: GALDERMA RESEARCH & DEVELOPMENT, 06410 Biot (FR)
(72) Inventor: BOITEAU, Jean-Guy, F-34130 Saint-Aunes (FR); CLARY, Laurence, F-06480 La Colle sur Loup (FR); MILLOIS BARBUIS, Corinne, F-83700 Saint-Raphael (FR)
(74) Representative: Allab, Myriam
(86) International application number: PCT/EP2005/013533
(87) International publication number: WO 2006/053791

(56) References cited:
- WO-A-02/12210
- WO-A-2004/024939
- WO-A-2004/113331
- FR-A- 2 833 949
- FR-A- 2 847 251
- FR-A- 2 847 580
- FR-A- 2 848 553

## Description

The invention relates to, as novel and useful industrial products, a novel class of compounds that are modulators of receptors of Peroxisome Proliferator-Activated Receptor type of subtype γ (PPARγ). The invention also relates to a process for preparing them and to their use in pharmaceutical compositions intended for use in human or veterinary medicine, or alternatively in cosmetic compositions.

The activity of receptors of PPAR type has been the subject of many studies. Mention may be made, as a guide, of the publication entitled "Differential Expression of Peroxisome Proliferator-Activated Receptor Subtypes During the Differentiation of Human Keratinocytes", Michel Rivier et al., J. Invest. Dermatol 111, 1998, pp. 1116-1121, in which is listed a large number of bibliographic references relating to receptors of PPAR type. Mention may also be made, as a guide, of the report entitled "The PPARs: From orphan receptors to Drug Discovery", Timothy M. Willson, Peter J. Brown, Daniel D. Sternbach and Brad R. Henke, J. Med. Chem., 2000, Vol. 43, pp. 527-550.

PPAR receptors activate transcription by binding to elements of DNA sequences, known as peroxisome proliferator response elements (PPRE), in the form of a heterodimer with retinoid X receptors (known as RXRs).

Three subtypes of human PPARs have been identified and described: PPARα, PPARγ and PPARδ (or NUC1).

PPARα is mainly expressed in the liver, while PPARδ is ubiquitous.

PPARγ is the most extensively studied of the three subtypes. All the references suggest a critical role of PPARγ in regulating the differentiation of adipocytes, where it is greatly expressed. It also has a key role in systemic lipid homeostasis.

It has been described in particular in patent application WO 96/33724 that PPARγ-selective compounds, such as a prostaglandin-J2 or -D2, are potential active agents for treating obesity and diabetes.

Moreover, the Applicant has already described PPARγ compounds and/or the use thereof in document FR 2 773 075, which describes the use of PPARγ activator compounds in the preparation of a pharmaceutical composition, the composition being intended to treat skin disorders associated with an anomaly of epidermal cell differentiation.

One of the aims of the present invention is to propose a novel class of PPARγ-modulating compounds that show very good specific affinity for PPARγ.

Thus, the present invention relates to compounds corresponding to the general formula (I) below: 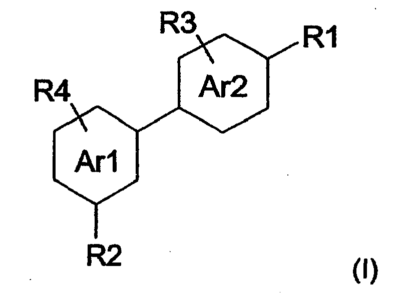 in which:
- **R1** represents a radical of formula (a) or (b) below: 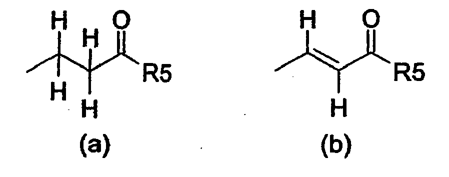 R5 having the meanings given below,
- **R2** represents a radical of formula (CH₂)ₘ-NR₆-CQ-(NH)ₙR₇; Q, R6, R7, m and n having the meanings given below,
- **R3** and **R4,** which may be identical or different, represent a hydrogen atom, a halogen atom, a linear or cyclic alkyl radical of 1 to 12 carbon atoms that may be interrupted with oxygen, fluorine or nitrogen atoms, a hydroxyl radical, an alkoxy radical containing from 1 to 10 carbon atoms, a polyether radical, an aralkyl radical or an aryloxy radical;
- **R5** represents a hydroxyl radical, a radical OR8 or a hydroxylamine radical;
   R8 being defined below,
- **R6** represents a lower alkyl radical containing from 1 to 4 carbon atoms;
- **R7** represents an alkyl radical containing from 1 to 12 carbon atoms, an aryl radical, an aralkyl radical, a heteroaryl radical or a heterocyclic radical;
- **R8** represents an alkyl, aryl or aralkyl radical;
- **m** and n may take the values 0 or 1;
- **Q** represents an oxygen or sulfur atom;
- **Ar1** and **Ar2** may be identical or different and represent an aromatic radical of formula: 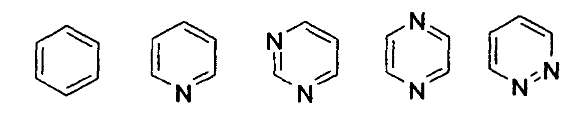 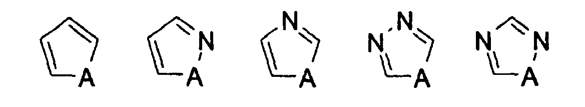
which, when it is an aryl radical, may be mono- or disubstituted with a halogen atom, a CF₃ radical, an alkyl radical containing from 1 to 12 carbon atoms, an alkoxy radical containing from 1 to 7 carbon atoms, a nitro function, a polyether radical, an aryl radical, a benzoyl radical, an alkyl ester group, a carboxylic acid, a hydroxyl radical optionally protected with an acetyl or benzoyl group or an amino function optionally protected with an acetyl or benzoyl group or optionally substituted with at least one alkyl containing from 1 to 12 carbon atoms;
and which, when it is an heteroaryl radical,is optionally substituted with at least one halogen, an alkyl containing from 1 to 12 carbon atoms, an alkoxy containing from 1 to 7 carbon atoms, an aryl radical, a nitro function, a polyether radical, a heteroaryl radical, a benzoyl radical, an alkyl ester group, a carboxylic acid, a hydroxyl optionally protected with an acetyl or benzoyl group or an amino function optionally protected with an acetyl or benzoyl group or optionally substituted with at least one alkyl containing from 1 to 12 carbon atoms
- **A** represents a sulfur or oxygen atom or a radical N-R9;
- **R9** represents a hydrogen atom or an alkyl radical containing from 1 to 12 carbon atoms;
it being understood that when Ar1 or Ar2 is an aryl radical, then Ar2 or Ar1 is necessarily a heteroaryl radical,
and the optical and geometrical isomers of the said compounds of formula (I), and also the salts thereof.

In particular, when the compounds according to the invention are in the form of salts, they are salts of an alkali metal, in particular a sodium or potassium salt, or salts of an alkaline-earth metal or salts of organic amines, more particularly of amino acids such as arginine or lysine. In the case of compounds containing nitrogen heterocycles, the salts may be mineral or organic acids.

According to the present invention, the term "hydroxyl radical" means an -OH radical.

The term "halogen atom" means a fluorine, chlorine or bromine atom.

According to the present invention, the term "alkyl radical containing from 1 to 12 carbon atoms" means a saturated or unsaturated, linear or cyclic, optionally branched, hydrogenated or fluorinated radical containing from 1 to 12 carbon atoms, which may be interrupted with a hetero atom, and the alkyl radicals containing from 1 to 12 carbon atoms are preferably methyl, ethyl, isopropyl, butyl, isobutyl, tert-butyl, hexyl, heptyl, octyl, decyl, cyclopentyl, cyclohexyl or methylenecyclopropyl radicals.

The alkyl radicals containing from 1 to 4 carbon atoms will preferably be methyl, ethyl, n-propyl, i-propyl, c-propyl, methylcyclopropyl, n-butyl, i-butyl or t-butyl radicals.

The term "alkoxy radical containing from 1 to 7 carbon atoms" means a methoxy, ethoxy, isopropyloxy, methylcyclopropyloxy, isobutoxy, tert-butoxy, pentyloxy, isopentyloxy, hexyloxy, heptyloxy, benzyloxy, aryloxy or phenoxy radical, which may be optionally substituted with an alkyl radical containing from 1 to 12 carbon atoms or an alkoxy radical containing from 1 to 5 carbon atoms.

The term "polyether radical" means a polyether radical containing from 1 to 6 carbon atoms interrupted with at least one oxygen atom, such as methoxymethoxy, ethoxymethoxy or methoxyethoxymethoxy radicals.

The term "aralkyl radical" means a benzyl, phenethyl or 2-naphthylmethyl radical, which may be mono- or disubstituted with a halogen atom, a CF₃ radical, an alkyl radical containing from 1 to 12 carbon atoms, an alkoxy radical containing from 1 to 7 carbon atoms, a nitro function, a polyether radical, an aryl radical, a benzoyl radical, an alkyl ester group, a carboxylic acid, a hydroxyl radical optionally protected with an acetyl or benzoyl group or an amino function optionally protected with an acetyl or benzoyl group or optionally substituted with at least one alkyl containing from 1 to 12 carbon atoms.

The term "aryl radical" means a phenyl, biphenyl, cinnamyl or naphthyl radical, which may be mono- or disubstituted with a halogen atom, a CF₃ radical, an alkyl radical containing from 1 to 12 carbon atoms, an alkoxy radical containing from 1 to 7 carbon atoms, a nitro function, a polyether radical, an aryl radical, a benzoyl radical, an alkyl ester group, a carboxylic acid, a hydroxyl radical optionally protected with an acetyl or benzoyl group or an amino function optionally protected with an acetyl or benzoyl group or optionally substituted with at least one alkyl containing from 1 to 12 carbon atoms.

The term "heteroaryl radical" means an aryl radical interrupted with one or more hetero atoms, such as a pyridyl, furyl, thienyl, isoxazolyl, oxadiazolyl, oxazolyl, isothiazolyl, quinazolinyl, benzothiadiazolyl, benzimidazolyl, quinoxalyl, indolyl or benzofuryl radical, optionally substituted with at least one halogen, an alkyl containing from 1 to 12 carbon atoms, an alkoxy containing from 1 to 7 carbon atoms, an aryl radical, a nitro function, a polyether radical, a heteroaryl radical, a benzoyl radical, an alkyl ester group, a carboxylic acid, a hydroxyl optionally protected with an acetyl or benzoyl group or an amino function optionally protected with an acetyl or benzoyl group or optionally substituted with at least one alkyl containing from 1 to 12 carbon atoms.

The term "heterocyclic radical" preferably means a morpholino, piperidino, piperazino, 2-oxo-1-piperidyl or 2-oxo-1-pyrrolidinyl radical, optionally substituted with at least one alkyl containing from 1 to 12 carbon atoms, an alkoxy containing from 1 to 7 carbon atoms, an aryl radical, a nitro function, a polyether radical, a heteroaryl radical, a benzoyl radical, an alkyl ester group, a carboxylic acid, a hydroxyl optionally protected with an acetyl or benzoyl group or an amino function optionally protected with an acetyl or benzoyl group or optionally substituted with at least one alkyl containing from 1 to 12 carbon atoms.

Among the compounds of formula (I) above that fall within the context of the present invention, mention may be made especially of the following compounds alone or as a mixture:
1. 3-(5-{3-[(Methyloctanoylamino)methyl]phenyl}thiophen-2-yl)acrylic acid
2. 3-(5-{3-[(Methyloctanoylamino)methyl]phenyl}thiophen-2-yl)propanoic acid
3. 3-(5-{3-[(Methyloctanoylamino)methyl]phenyl}furan-2-yl)-acrylic acid
4. 3-(4-{5-[(Methyloctanoylamino)methyl]thiophen-3-yl}-phenyl)acrylic acid
5. 3-(4-{5-[(Methyloctanoylamino)methyl]thiophen-3-yl}-phenyl)propanoic acid
6. 3-{4-[6-(3-Heptyl-1-methylureido)pyrid-2-yl]phenyl}-propanoic acid
7. 3-{6-[3-(3-Heptyl-1-methylureido)phenyl]pyrid-3-yl}-propanoic acid
8. 2-[4-(2-Carboxyethyl)phenyl]-4-(3-heptyl-1-methylureido)pyridinium acetate
9. 3-{5-[3-(3-Pentyl-1-methylureido)phenyl]pyrimidin-2-yl}acrylic acid
10. 3-{5-[3-(3-Pentyl-1-methylureido)phenyl]pyrimidin-2-yl}propanoic acid
11. 2-[2-Butoxy-4-(2-carboxyethyl)phenyl]-6-(1-methyl-3-pentylureido)pyridinium hydrochloride
12. 2-[2-Butoxy-4-(2-carboxyethyl)phenyl]-6-(1-methyl-3-heptylureido)pyridinium hydrochloride
13. 2-[4-(2-Carboxyethyl)-2-ethoxyphenyl]-6-(3-heptyl-1-methylureido)pyridinium hydrochloride
14. 3-(3-Butoxy-4-{5-[(methyloctanoylamino)methyl]thiophen-3-yl}phenyl)propanoic acid
15. 3-(3-Butoxy-4-(5-{[(4-methoxybenzoyl)methylamino]-methyllthiophen-3-yl)phenyl]propanoic acid
16. 3-(3-Butoxy-4-(5-{[(3-methoxybenzoyl)methylamino]-methyl}thiophen-3-yl)phenyl]propanoic acid
17. 3-{5-[4-(3-Heptyl-1-methylureido)pyrid-2-yl]furan-2-yl}propanoic acid
18. 3-[5-[3-(3-Heptyl-1-methylureido)phenyl]-4-(2,2,2-tri-fluoroethoxy)furan-2-yl]propanoic acid
19. 3-(5-{3-[3-(3,5-Dimethoxyphenyl)-1-ethylureido]phenyl}-3-methylfuran-2-yl)propanoic acid
20. 3-(5-{6-[3-(4-Ethoxyphenyl)-1-ethylureido]pyrid-2-yl}-furan-2-yl)propanoic acid
21. 3-(2-Methyl-4-{6-[1-methyl-3-(4-methylpentyl)ureido]-pyrid-2-yl}phenyl)propanoic acid
22. Methyl 3-{4-[6-(1-ethyl-3-naphthalen-2-ylureido)pyrid-2-yl]-2-fluorophenyl}propanoate
23. 3-(4-{6-[3-(4-Butoxyphenyl)-1-methylureido]pyrid-2-yl}phenyl)-N-hydroxypropionamide
24. 3-{3-Cyclopropylmethoxy-4-[6-(1-methyl-3-pentylureido)-pyrid-2-yl]phenyl}propanoic acid
25. 3-{4-[6-(1-Ethyl-3-phenylthioureido)pyrid-2-yl]-3-propoxyphenyl}propanoic acid
26. 3-{3-Ethoxy-4-[6-(3-heptyl-1-methylureido)pyrid-2-yl]-phenyl}propanoic acid
27. 3-{4-[6-(3-Hexyl-1-methylureido)pyrid-2-yl]-3-isopropoxyphenyl}propanoic acid
28. 3-[4-[6-(3-Heptyl-1-methylureido)pyrid-2-yl]-3-(4,4,4-trifluorobutoxy)phenyl]propanoic acid
29. 3-{3-(2-Dimethylaminoethoxy)-4-[6-(1-methyl-3-pentylureido)pyrid-2-yl]phenyl}propanoic acid
30. 3-{3-(3-Hydroxypropoxy)-4-[6-(1-methyl-3-pentylureido)-pyrid-2-yl]phenyl}propanoic acid
31. 3-{4-[6-(1-Ethyl-3-phenylthioureido)pyrid-2-yl]-3-fluorophenyl}acrylic acid
32. 3-{4-[6-(3-Hexyl-1-methylureido)pyrid-2-yl]-3-trifluoromethylphenyl}propanoic acid
33. 3-[6'-(1-Methyl-3-phenethylureido)[2,2']bipyridinyl-5-yl]propanoic acid
34. 3-{2-[6-(3-Hexyl-1-methylureido)pyrid-2-yl]thiazol-4-yl}propanoic acid
35. Ethyl 3-{2-[6-(3-hexyl-1-methylureido)pyrid-2-yl]-thiazol-5-yl}propanoate
36. 3-(3-{6-[1-Methyl-3-(6-methylheptyl)ureido]pyrid-2-yl}isoxazol-5-yl)propanoic acid
37. 3-{4-[2-(3-Heptyl-1-methylureido)pyrimidin-4-yl]phenyl}-propanoic acid
38. 3-{3-Cyclopropylmethoxy-4-[2-(3-heptyl-1-methylureido)-pyrimidin-4-yl]phenyl}propanoic acid
39. 3-(4-{2-[1-Ethyl-3-(4-propoxyphenyl)ureido]pyrimidin-4-yl}-3-fluorophenyl)propanoic acid
40. 3-{2-Fluoro-4-[2-(1-methyl-3-naphthalen-2-ylureido)-pyrid-4-yl]phenyl}-N-hydroxypropionamide
41. 3-[2'-(3-Hexyl-1-methylthioureido)[2,4']bipyridinyl-5-yl]propanoic acid
42. 3-{4-[2-(3-Hexyl-1-methylureido)pyrid-4-yl]-3-propoxyphenyl}propanoic acid
43. 3-(3-Benzyloxy-4-{4-[1-methyl-3-(5-methylhexyl)ureido]-pyrid-2-yl}phenyl)propanoic acid
44. 3-{2-[4-(3-Hexyl-1-methylureido)pyrid-2-yl]thiazol-5-yl}acrylic acid
45. 3-{5-[3-(3-Hexyl-1-methylureido)phenyl]pyrid-2-yl}-acrylic acid
46. 3-{5-[5-(3-Heptyl-1-methylthioureido)thiophen-3-yl]-pyrid-2-yl}acrylic acid
47. 3-{4-[2-(3-Heptyl-1-methylureido)thiazol-4-yl]-3-propoxyphenyl}propanoic acid
48. 3-(2-Fluoro-4-{5-[(heptanoylmethylamino)methyl]thiophen-3-yl}phenyl)propanoic acid
49. 3-(4-{5-[(Heptanoylmethylamino)methyl]thiophen-3-yl}-3-isobutoxyphenyl)acrylic acid
50. 3-(3-(2-Cyclopentylethoxy)-4-{5-[(methyloctanoylamino)-methyl]thiophen-3-yl}phenyl)propanoic acid
51. Methyl 3-(3-isobutoxy-4-{5-[(methylnonanoylamino)-methyl]thiophen-3-yl}phenyl)propanoate
52. 3-{6-[5-({Ethyl-[2-(2-pentylphenyl)acetyl]amino}methyl)-thiophen-3-yl]pyrid-3-yl}propanoic acid
53. 3-(4-{4-[(Methylnonanoylamino)methyl]thiazol-2-yl}-3-propoxyphenyl)propanoic acid
54. 3-(2-Chloro-4-{4-[(methylnonanoylamino)methyl]thiophen-2-yllphenyl)propanoic acid
55. 3-(2-Fluoro-4-{4-[(methylnonanoylamino)methyl]thiophen-2-yl}phenyl)acrylic acid
56. 3-(4-{4-[(Heptanoylmethylamino)methyl]thiophen-2-yl}-1-methyl-1H-pyrrol-2-yl)propanoic acid
57. 3-(4-{4-[(Heptanoylmethylamino)methyl]thiophen-2-yl}-furan-2-yl)propanoic acid
58. 3-{5'-[(Heptanoylmethylamino)methyl][3,3']bithiophenyl-5-yl}propanoic acid
59. Phenyl 3-{5'-[(heptanoylmethylamino)methyl]-3-propyl-[2,3']bithiophenyl-5-yl}propanoate
60. 3-(5-{5-[(Heptanoylmethylamino)methyl]thiophen-3-yl}-4-propylfuran-2-yl)acrylic acid
61. 3-{3-Butoxy-4-[6-(3-heptyl-1-methylureido)pyrid-2-yl]phenyl}propanoic acid
62. 3-(3-Benzyloxy-4-[6-(3-heptyl-1-methylureido)pyrid-2-yl]phenyl}propanoic acid
63. 3-{3-Benzyloxy-4-[2-(3-heptyl-1-methylureido)pyrimidin-4-yl]phenyl}propanoic acid
64. 3-{3-Butyloxy-4-[2-(3-heptyl-1-methylureido)pyrimidin-4-yl]phenyl}propanoic acid
65. 3-{3-Butoxy-4-[4-(3-heptyl-1-methylureido)pyrid-2-yl]phenyl}propanoic acid
66. 3-{3-Benzyloxy-4-[4-(3-heptyl-1-methylureido)pyrid-2-yl]phenyl}propanoic acid
67. 3-{3-Benzyloxy-4-[5-(3-heptyl-1-methylureido)pyrid-3-yl]phenyl}propanoic acid
68. 3-{3-Butoxy-4-[5-(3-heptyl-1-methylureido)pyrid-3-yl]phenyl}propanoic acid
69. 3-(5-{3-[(Methyloctanoylamino)methyl]phenyl}-4-propylthiophen-2-yl)propanoic acid
70. 3-(3-Benzyloxy-4-{5-[(methyloctanoylamino)methyl]thio-phen-3-yl}phenyl}propanoic acid
71. 3-(4-Benzyl-5-{3-[(hexanoylmethylamino)methyl]phenyl}-thiophen-2-yl)propanoic acid
72. 3-{4-Cyclopropylmethyl-5-[3-(1-methyl-3-pentylureido)-phenyl]thiophen-2-yl}propanoic acid
73. 3-{5-[3-(1-Methyl-3-pentylureido)-4-trifluoromethylphenyl]thiophen-2-yl}propanoic acid
74. 3-(5-{3-[3-(4-Butoxyphenyl)-1-ethylureido]phenyl}-thiophen-2-yl)propanoic acid
75. 3-{5-[3-(3-Heptyl-1-methylureido)-4-trifluoromethylphenyl]furan-2-yl}propanoic acid
76. 3-{2-Butoxy-4-[6-(1-methyl-3-pentylureido)pyrid-2-yl]phenyl}propanoic acid
77. 3-{2-(4-Methoxybenzyloxy)-4-[6-(1-methyl-3-pentyl-ureido)pyrid-2-yl]phenyl}propanoic acid
78. 3-{2-(3-Methoxybenzyloxy)-4-[6-(1-methyl-3-pentyl-ureido)pyrid-2-yl]phenyl}propanoic acid
79. 3-[2-Cyclopropylmethoxy-4-(6-{3-[2-(4-methoxyphenyl)-ethyl]-1-methylureido}pyrid-2-yl)phenyl]propanoic acid

According to the present invention, the compounds of formula (I) that are more particularly preferred are those having at least one of the following characteristics:
- R3 is an alkoxy radical of 1 to 7 carbon atoms;
- R5 corresponds to a hydroxyl radical;
- the sequence -(CH₂)ₘ-NR₆-CQ(NH)ₙR₇ has m = 0, n = 1;
- Q is an oxygen atom;
- R7 represents an alkyl radical of 1 to 8 carbon atoms;
- at least Ar1 or Ar2 is a group of pyridine type.

The subject of the present invention is also processes for preparing the compounds of formula (I), in particular according to the reaction schemes given in **Figures 1, 2, 3** and **4.**

**Figure 1:** The boronic acid or ester **2** may be obtained from compound **1** by using standard conditions, for example by reaction with tert-butyllithium followed by an addition to the trimethyl borate or reaction with pinacoldiborane in the presence of a palladium catalyst, for instance diphenylphosphinoferrocenepalladium dichloride. Compound **1** may also be directly coupled with an isocyanate, a thioisocyanate or an acyl chloride (in this case n=0) under standard conditions to give compound **3.**

The boronic acid or ester **4** may be obtained from compound **3** by using the standard conditions, for example by reaction with tert-butyllithium followed by an addition to trimethyl borate or reaction with pinacoldiborane in the presence of a palladium catalyst, for instance diphenylphosphinoferrocenepalladium dichloride.

The compounds **5** may either be purchased commercially or may be synthesized from the corresponding aldehyde thereof using a Wittig reaction.

A Suzuki type palladium coupling between the boronate **4** or **2** and compound **5** (chosen from an aryl bromide, iodide, chloride or triflate) allows the compounds having the aryl-aryl sequence **7** or **9,** respectively, to be obtained.

A Suzuki type palladium coupling between the boronate **4** or **2** and compound **6** (chosen from an aryl bromide, iodide, chloride or triflate) allows the compounds having the aryl-aryl sequence **8** or **11,** respectively, to be obtained.

Compounds **10** and **12** may be obtained from compounds **9** and **11** by deprotection under standard conditions, for example by treatment with a strong acid, for instance trifluoroacetic acid if P is a Boc group.

Compounds **10** and **12** may be coupled with isocyanates, thioisocyanates or acyl chlorides (in this case n=0) under the standard conditions to give the compounds **7** and **8** respectively.

Compounds **8** and **11** may be obtained via hydrogenation of compounds **7** and **9** under standard hydrogenation conditions, for instance: hydrogen catalysed with palladium-on-charcoal.

The acid functions of compounds **13** and **14** may be obtained from compounds **7** and **8** respectively: via saponification if R8 is an alkyl chain, using a base, for instance sodium hydroxide.

Compound **14** may be obtained via hydrogenation of compound **13** under standard hydrogenation conditions, for instance: hydrogen catalysed with palladium-on-charcoal.

Compounds **15** and **16** may be obtained from the esters **7** and **8,** respectively, via treatment with hydroxylamine.

**Figure 2:** A Suzuki type palladium coupling between a commercial arylboronic acid containing a formyl group and compounds **1** or **3** (chosen from an aryl bromide, iodide, chloride or triflate) allows the compounds having the aryl-aryl sequence **18** or **17,** respectively, to be obtained.

Compound **18** may be coupled with isocyanates, thioisocyanates or acyl chlorides (in this case n=1) under standard conditions to give compound **17.**

Compounds **7** and **9** may be obtained via a Wittig reaction starting with compounds **17** and **18,** respectively, for example via the action of methyl triphenylphosphoranylideneacetate.

### Figure 3

A Suzuki type palladium coupling between the boronic acids or esters **4** or **2** and aryl dihalides chosen from bromides, iodides and chlorides allows the compounds having the aryl-aryl sequence **19** or **20,** respectively, to be obtained.

Compounds **7** and **9** may be obtained from compounds **19** and **20,** respectively, via a Heck type palladium coupling with alkyl or aryl acrylates.

### Figure 4

The boronates **21** and **22** may be obtained by treating compounds **5** and **6,** respectively, with pinacoldiborane, in the presence of a palladium-based catalyst, for instance diphenylphosphinoferrocenepalladium dichloride.

A Suzuki type palladium coupling between compound **21** and compounds **3** and **1** (chosen from an aryl bromide, iodide, chloride or triflate) allows the compounds having the aryl-aryl sequence **7** and **9,** respectively, to be obtained.

A Suzuki palladium coupling between compound **22** and compounds **3** and **1** (chosen from aryl bromide, iodide, chloride or triflate) allows compounds having the aryl-aryl sequence **8** and **11,** respectively, to be obtained.

The compounds according to the invention show modulatory properties on receptors of PPAR type. This activity on the PPARα, δ and γ receptors is measured in a transactivation test and quantified via the dissociation constant Kdapp (apparent), as described in Example 6.

The preferred compounds of the present invention have a dissociation constant of less than or equal to 5000 nM and advantageously less than or equal to 1000 nM.

Preferably, the compounds are modulators of receptors of specific PPARγ type, i.e. they have a ratio between the Kdapp for the PPARα and PPARδ receptors, and the Kdapp for the PPARγ receptors, of greater than or equal to 10. Preferably, this ratio PPARγ/PPARα or PPARδ/PPARγ is greater than or equal to 50 and more advantageously greater than or equal to 100.

A subject of the present invention is also, as medicaments, the compounds of formula (I) as described above.

A subject of the present invention is the use of the compounds of formula (I) to manufacture a composition for regulating and/or restoring skin metabolism lipid.

The compounds according to the invention are also particularly suitable in the following fields of treatment:
1) for treating dermatological complaints associated with a keratinization disorder relating to differentiation and to proliferation, in particular for treating common acne, comedones, polymorphs, rosacea, nodulocystic acne, acne conglobata, senile acne and secondary acne such as solar, medicinal or occupational acne,
2) for treating other types of keratinization disorder, in particular ichthyosis, ichthyosiform conditions, Darier's disease, palmoplantar keratoderma, leukoplakia and leukoplakiform conditions, and cutaneous or mucous (oral) lichen,
3) for treating other dermatological complaints with an inflammatory immuno-allergic component, with or without a cellular proliferation disorder, and in particular all forms of psoriasis, whether cutaneous, mucous or ungual psoriasis, and even psoriatic arthritis, or alternatively cutaneous atopy such as eczema, or respiratory atopy or gingival hypertrophy,
4) for treating all dermal or epidermal proliferations, whether benign or malignant, whether or not of viral origin, such as common warts, flat warts and epidermodysplasia verruciformis, oral or florid papillomatoses, T lymphoma and proliferations which may be induced by ultraviolet light, in particular in the case of basal cell and spinocellular epithelioma, and also any precancerous skin lesion such as keratoacanthomas,
5) for treating other dermatological disorders such as immune dermatitides, such as lupus erythematosus, bullous immune diseases and collagen diseases, such as scleroderma,
6) in the treatment of dermatological or systemic complaints with an immunological component,
7) in the treatment of skin disorders due to exposure to UV radiation, and also for repairing or combating ageing of the skin, whether light-induced or chronological ageing, or for reducing actinic keratoses and pigmentations, or any pathology associated with chronological or actinic ageing, such as xerosis,
8) for combating sebaceous function disorders such as the hyperseborrhoea of acne, simple seborrhoea or seborrhoeic dermatitis,
9) for preventing or treating cicatrization disorders or for preventing or repairing stretch marks,
10) in the treatment of pigmentation disorders, such as hyperpigmentation, melasma, hypopigmentation or vitiligo,
11) in the treatment of lipid metabolism complaints, such as obesity, hyperlipidaemia, non-insulin-dependent diabetes or syndrome X,
12) in the treatment of inflammatory complaints such as arthritis,
13) in the treatment or prevention of cancerous or precancerous conditions,
14) in the prevention or treatment of alopecia of various origins, in particular alopecia caused by chemotherapy or radiation,
15) in the treatment of immune system disorders, such as asthma, type I sugar diabetes, multiple sclerosis or other selective dysfunctions of the immune system, or
16) in the treatment of complaints of the cardiovascular system, such as arteriosclerosis or hypertension.

A subject of the present invention is also a pharmaceutical or cosmetic composition comprising, in a physiologically acceptable medium, at least one compound of formula (I) as defined above.

The composition according to the invention may be administered enterally, parenterally, topically or ocularly. The pharmaceutical composition is preferably packaged in a form that is suitable for topical application.

Via the enteral route, the composition, more particularly the pharmaceutical composition, may be in the form of tablets, gel capsules, sugar-coated tablets, syrups, suspensions, solutions, powders, granules, emulsions or lipid or polymer vesicles or nanospheres or microspheres to allow controlled release. Via the parenteral route, the composition may be in the form of solutions or suspensions for infusion or for injection.

The compounds according to the invention are generally administered at a daily dose of about 0.001 mg/kg to 100 mg/kg of body weight in 1 to 3 dosage intakes.

The compounds are used systemically at a concentration generally of between 0.001% and 10% by weight and preferably between 0.01% and 1% by weight relative to the weight of the composition.

Via the topical route, the pharmaceutical composition according to the invention is more particularly intended for treating the skin and mucous membranes and may be in the form of ointments, creams, milks, salves, powders, impregnated pads, syndets, solutions, gels, sprays, foams, suspensions, stick lotions, shampoos or washing bases. It may also be in the form of suspensions of lipid or polymer vesicles or nanospheres or microspheres or polymer patches and hydrogels to allow controlled release. This topical-route composition may be in anhydrous form, in aqueous form or in the form of an emulsion.

The compounds are used topically at a concentration generally of between 0.001% and 10% by weight, preferably between 0.01% and 1% by weight relative to the total weight of the composition.

The compounds of formula (I) according to the invention also find an application in the cosmetic field, in particular in body and hair hygiene and more particularly for regulating and/or restoring skin lipid metabolism.

A subject of the invention is therefore also the cosmetic use of a composition comprising, in a physiologically acceptable support, at least one of the compounds of formula (I) for body or hair hygiene.

The cosmetic composition according to the invention containing, in a cosmetically acceptable support, at least one compound of formula (I) or an optical or geometrical isomer thereof or a salt thereof, may usually be in the form of a cream, a milk, a lotion, a gel, suspensions of lipid or polymer vesicles or nanospheres or microspheres, impregnated pads, solutions, sprays, foams, sticks, soaps, shampoos or washing bases.

The concentration of compound of formula (I) in the cosmetic composition is between 0.0001% and 2% by weight relative to the total weight of the composition.

The pharmaceutical and cosmetic compositions as described above may also contain inert or even pharmacodynamically active additives as regards the pharmaceutical compositions, or combinations of these additives, and especially:
- wetting agents;
- flavour enhancers;
- preserving agents such as para-hydroxybenzoic acid esters;
- stabilizers;
- humidity regulators;
- pH regulators;
- osmotic pressure modifiers;
- emulsifiers;
- UV-A and UV-B screening agents;
- antioxidants, such as α-tocopherol, butylhydroxyanisole or butylhydroxytoluene, superoxide dismutase, ubiquinol or certain metal-chelating agents;
- depigmenting agents such as hydroquinone, azelaic acid, caffeic acid or kojic acid;
- emollients;
- moisturizers, for instance glycerol, PEG 400, thiamorpholinone and derivatives thereof, or urea;
- antiseborrhoeic or antiacne agents, such as S-carboxymethylcysteine, S-benzylcysteamine, salts thereof or derivatives thereof, or benzoyl peroxide;
- antibiotics, for instance erythromycin and its esters, neomycin, clindamycin and its esters, and tetracyclines;
- antifungal agents such as ketoconazole or polymethylene-4,5-isothiazolidones-3;
- agents for promoting regrowth of the hair, for instance Minoxidil (2,4-diamino-6-piperidinopyrimidine 3-oxide) and its derivatives, Diazoxide (7-chloro-3-methyl-1,2,4-benzothiadiazine 1,1-dioxide) and Phenytoin (5,4-diphenylimidazolidine-2,4-dione);
- non-steroidal anti-inflammatory agents;
- carotenoids, and especially β-carotene;
- antipsoriatic agents such as anthralin and its derivatives;
- eicosa-5,8,11,14-tetraynoic acid and eicosa-5,8,11-triynoic acid, and esters and amides thereof;
- retinoids, i.e. RAR or RXR receptor ligands, which may be natural or synthetic;
- corticosteroids or oestrogens;
- α-hydroxy acids and α-keto acids or derivatives thereof, such as lactic acid, malic acid, citric acid, glycolic acid, mandelic acid, tartaric acid, glyceric acid or ascorbic acid, and also the salts, amides or esters thereof, or β-hydroxy acids or derivatives thereof, such as salicylic acid and the salts, amides or esters thereof;
- ion-channel blockers such as potassium-channel blockers;
- or alternatively, more particularly for the pharmaceutical compositions, in combination with medicinal products known to interfere with the immune system (for example cyclosporin, FK 506, glucocorticoids, monoclonal antibodies, cytokines or growth factors, etc.).

Needless to say, a person skilled in the art will take care to select the optional compound(s) to be added to these compositions such that the advantageous properties intrinsically associated with the present invention are not, or are not substantially, adversely affected by the envisaged addition.

Another subject of the invention relates to a cosmetic process for beautifying the skin, characterized in that a composition comprising at least one compound of formula (I) as defined above is applied to the skin. Regulation and/or restoration of the metabolism of the skin lipids makes it possible to obtain skin whose surface appearance is embellished.

Several examples of the production of active compounds of formula (I) according to the invention, and also biological activity results for such compounds and various concrete formulations based on its compounds will now be given, by way of illustration and with no limiting nature.

### Example 1: 3-(5-{3-[(Methyloctanoylamino)methyl]-phenyl}thiophen-2-yl)acrylic acid

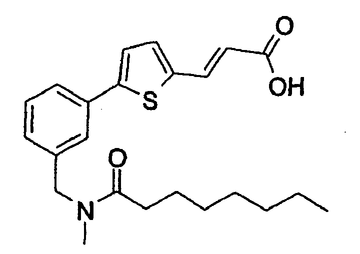

### a. N-Methyloctanamide

25 g (0.37 mol) of methylamine hydrochloride are dissolved in 125 mL of dichloromethane, and 115 mL of triethylamine are then added. At 0°C, 70 mL (0.41 mol) of octanoyl chloride are added slowly. The reaction mixture is stirred for 3 hours at room temperature. The mixture is filtered and 100 ml of water are then added to the filtrate. The organic phase is separated out by settling, dried over sodium sulfate and evaporated. 61 g of N-methyloctanamide are obtained in quantitative yield.

### b. N-Methyl-N-(3-bromobenzyl)octanamide

5 g (31.8 mmol) of N-methyloctanamide (1a) are added at 0°C to a suspension of 1.4 g (35 mmol) of sodium hydride (60% in grease) in 60 mL of tetrahydrofuran. The reaction mixture is stirred for 30 minutes at room temperature and a solution of 8.9 g (35 mmol) of 3-bromobenzyl bromide in 15 mL of tetrahydrofuran is then added. The mixture is stirred for 16 hours at room temperature. The reaction is stopped by adding 100 mL of water and is then extracted with ethyl acetate. The organic phases are combined and dried over sodium sulfate. The solvents are evaporated off and the residue is then chromatographed on silica gel (75/25 heptane/ethyl acetate). 7.4 g of N-methyl-N-(3-bromobenzyl)octanamide are obtained.
Yield = 71%.

### c. Octanoylmethyl-[3-(4,4,5,5-tetramethyl-[1,3,2]dioxaborolan-2-yl)benzyl]amide

1.0 g (3.98 mmol, 1.3 eq) of pinacoldiborane is added to a mixture of 1.0 g (3.06 mmol, 1 eq) of N-methyl-N-(3-bromobenzyl)octanamide and 900 mg (9.18 mmol, 3 eq) of potassium acetate, in the presence of 111 mg (0.15 mmol, 5 mol%) of diphenylphosphinoferrocenepalladium dichloride (PdCl₂dppf) in 10 mL of dimethylformamide. The mixture is stirred for 2 hours at 80°C. The reaction is stopped by adding 20 ml of water and is then extracted with ethyl acetate. The organic phases are combined and dried over sodium sulfate. The solvents are evaporated off and the residue is then chromatographed on silica gel (80/20 heptane/ethyl acetate). 1.0 g of octanoylmethyl-[3-(4,4,5,5-tetramethyl[1,3,2]dioxa-borolan-2-yl)benzyl]amide is obtained in the form of an oil. Yield = 88%.

### f. Methyl 3-(5-bromothiophen-2-yl)acrylate

4.2 g (12.5 mmol, 1.2 eq) of methyl triphenylphosphoranylideneacetate are added to a solution of 2.0 g (10.4 mmol, 1.0 eq) of 5-bromothiophene-2-carbaldehyde in 15 mL of toluene. The reaction mixture is stirred for 1 hour at 80°C. The reaction is stopped by adding 20 ml of water and is then extracted with ethyl acetate. The organic phases are combined and dried over sodium sulfate. The solvents are evaporated off and the residue is then chromatographed on silica gel (80/20 heptane/ethyl acetate). 2.1 g of methyl 3-(5-bromothiophen-2-yl)acrylate are obtained. Yield = 82%.

### g. Methyl 3-(5-{3-[(methyloctanoylamino)methyl]phenyl}-thiophen-2-yl)acrylate

200 mg (0.81 mmol, 1.0 eq) of methyl 3-(5-bromothiophen-2-yl)acrylate and 334 mg (0.89 mmol, 1.1 eq) of octanoylmethyl-[3-(4,4,5,5-tetramethyl[1,3,2]dioxaborolan-2-yl)benzyl]amide are dissolved in 5 ml of a 5/1 mixture of dimethylformamide and of 2M potassium phosphate solution. 93 mg (0.08 mmol, 10 mol%) of tetrakis(triphenylphosphine)palladium are added and the reaction mixture is then stirred for 2 hours at 80°C. The reaction is stopped by adding 30 mL of water and is then extracted with ethyl acetate. The organic phases are combined and dried over sodium sulfate. The solvents are evaporated off and the residue is then chromatographed on silica gel (80/20 to 60/40 heptane/ethyl acetate). 220 mg of methyl 3-(5-{3-[(methyloctanoylamino)methyl]phenyl}thiophen-2-yl)acrylate are obtained. Yield = 65%.

### h. 3-(5-{3-[(Methyloctanoylamino)methy]phenyl}-thiophen-2-yl)acrylic acid

212 mg (5.3 mmol, 10 eq) of sodium hydroxide are added to a solution of 220 mg (0.53 mmol, 1 eq) of methyl 3-(5-{3-[(methyloctanoylamino)methyl]phenyl}-thiophen-2-yl)acrylate in 4 ml of 9/1 tetrahydrofuran/methanol. The reaction mixture is stirred for two hours at room temperature. The reaction is stopped by adding 20 ml of water and 3 ml of acetic acid and is then extracted with ethyl acetate. The organic phases are combined and dried over sodium sulfate. The solvents are evaporated off and the residue is then chromatographed on silica gel (90/10 dichloromethane/methanol). The oil obtained is crystallized from pentane. 155 mg of 3-(5-{3-[(methyloctanoylamino)methyl]phenyl}thiophen-2-yl)acrylic acid are obtained. Yield = 73%. m.p. = 102-104°C.
**¹H NMR** (CDCl₃, 400 MHz): 0.88 (m, 3H); 1.31 (m, 8H); 1.72 (m, 2H); 2.42 (m, 2H); 2.98&3.00 (2s(rotamers), 3H); 4.60&4.66 (2s(rotamers), 2H); 6.24&6.26 (2d(rotamers), *J* = 15.6 Hz, 1H); 7.16-7.56 (m, 6H); 7.86 (2d(rotamers), *J* = 15.6 Hz, 1H).

### Example 2: 3-(5-{3-[(Methyloctanoylamino)methyl]-phenyl}thiophen-2-yl)propanoic acid

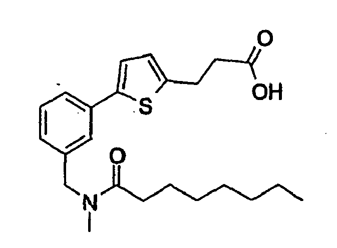

A solution of 80 mg (0.20 mmol, 1 eq) of 3-(5-{3-[(methyloctanoylamino)methyl]phenyl}thiophen-2-yl)acrylic acid (prepared as in 1h) in 2 mL of methanol is stirred for 2 hours at room temperature in the presence of 50 mg of 10% Pd/C under a hydrogen atmosphere. The palladium is filtered off and the solvents are then evaporated off. The residue is crystallized from pentane/dichloromethane. 71 mg of 3-(5-{3-[(methyloctanoylamino)methyl]phenyl}thiophen-2-yl)propanoic acid are obtained. Yield = 89%. m.p. = 67-68°C.
**¹H NMR** (CDCl₃, 400 MHz): 0.88 (m, 3H); 1.28 (m, 8H); 1.70 (m, 2H); 2.41 (m, 2H); 2.78 (m, 2H); 2.96&2.98 (2s(rotamers), 3H); 3.18 (m, 2H); 4.57&4.63 (2s(rotamers), 2H); 6.82 (m, 1H); 7.05-7.14 (m, 2H); 7.28-7.48 (m, 3H).

### Example 3: 3-(5-{3-[(Methyloctanoylamino)methyl]-phenyl}furan-2-yl)acrylic acid

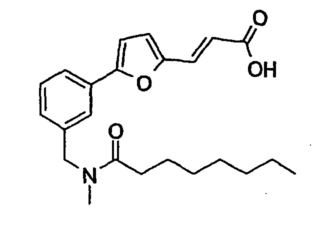

### a. Methyl 3-(5-bromofuran-2-yl)acrylate

4.58 g (13.7 mmol, 1.2 eq) of methyl triphenylphosphoranylideneacetate are added to a solution of 2.0 g (11.4 mmol, 1.0 eq) of 5-bromofuran-2-carbaldehyde in 15 mL of toluene. The reaction mixture is stirred for 1 hour at 80°C. The reaction is stopped by adding 20 ml of water and is then extracted with ethyl acetate. The organic phases are combined and dried over sodium sulfate. The solvents are evaporated off and the residue is then chromatographed on silica gel (80/20 heptane/ethyl acetate). 2.0 g of methyl 3-(5-bromofuran-2-yl)acrylate are obtained.
Yield = 77%.

### b. Methyl 3-(5-{3-[(methyloctanoylamino)methyl]phenyl}-furan-2-yl)acrylate

278 mg (1.20 mmol, 1.5 eq) of methyl 3-(5-bromofuran-2-yl)acrylate and 300 mg (0.80 mmol, 1.0 eq) of octanoylmethyl-[3-(4,4,5,5-tetramethyl[1,3,2]dioxa-borolan-2-yl)benzyl]amide (prepared as in le) are dissolved in 5 ml of a 5/1 mixture of dimethylformamide and of 2M potassium phosphate solution. 46 mg (0.04 mmol, 5 mol%) of tetrakis(triphenylphosphine)palladium are added and the reaction mixture is then stirred for 2 hours at 80°C. The reaction is stopped by adding 30 ml of water and is then extracted with ethyl acetate. The organic phases are combined and dried over sodium sulfate. The solvents are evaporated off and the residue is then chromatographed on silica gel (80/20 to 60/40 heptane/ethyl acetate). 250 mg of methyl 3-(5-{3-[(methyloctanoylamino)methyl]phenyl}furan-2-yl)acrylate are obtained. Yield = 78%.

### c. 3-(5-{3-[(Methyloctanoylamino)methyl]phenyl}furan-2-yl)acrylic acid

300 mg (7.5 mmol, 12 eq) of sodium hydroxide are added to a solution of 250 mg (0.63 mmol, 1 eq) of 3-(5-{3-[(methyloctanoylamino)methyl]phenyl}furan-2-yl)acrylate in 4 mL of 9/1 tetrahydrofuran/methanol. The reaction mixture is stirred for two hours at room temperature. The reaction is stopped by adding 20 ml of water and 3 ml of acetic acid and is then extracted with ethyl acetate. The organic phases are combined and dried over sodium sulfate. The solvents are evaporated off and the residue is then chromatographed on silica gel (90/10 dichloromethane/methanol). The oil obtained is crystallized from pentane. 140 mg of 3-(5-{3-[(methyloctanoylamino)methyl]phenyl}furan-2-yl)acrylic acid are obtained. Yield = 58%. m.p. = 96-98°C.
**¹H NMR** (CDCl₃, 400 MHz): 0.88 (m, 3H); 1.28 (m, 8H); 1.71 (m, 2H); 2.42 (m, 2H); 2.98&3.00 (2s(rotamers), 3H); 4.61&4.67 (2s(rotamers), 2H); 6.43 (2d(rotamers), *J* = 15.6 Hz, 1H); 6.76 (m, 2H); 7.18 (2d(rotamers), *J* = 7.6 Hz, 1H); 7.37-7.69 (m, 4H).

### Example 4: 3-(4-(5-[(Methyloctanoylamino)methyl]thio-phen-3-yl)phenyl)acrylic acid

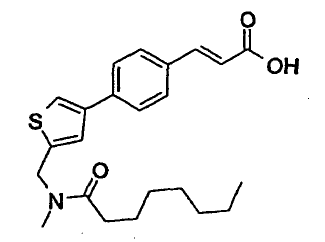

### a. Octanoyl(4-bromothiophen-2-ylmethyl)methylamide

880 mg (13 mmol, 5 eq) of methylamine hydrochloride and 500 mg (2.61 mmol, 1 eq) of 4-bromothiophene-2-carbaldehyde are dissolved in 5 ml of methanol in the presence of 2 g of anhydrous magnesium sulfate. The reaction mixture is stirred for 1 hour at room temperature. 327 mg (5.2 mmol, 2 eq) of sodium cyanoborohydride are added and the reaction mixture is stirred for 4 hours at room temperature. The reaction is stopped by adding 20 ml of water and is then extracted with ethyl acetate. The organic phases are combined and dried over sodium sulfate. The solvents are evaporated off and the residue is then dissolved in 4 ml of tetrahydrofuran in the presence of 0.5 mL of triethylamine. 0.45 mL (2.6 mmol, 1 eq) of octanoyl chloride is added and the reaction mixture is then stirred for 0.5 hour at room temperature. The reaction is stopped by adding 20 mL of water and is then extracted with ethyl acetate. The organic phases are combined and dried over sodium sulfate. The solvents are evaporated off and the residue is then chromatographed on silica gel (80/20 heptane/ethyl acetate). 300 mg of octanoyl(4-bromothiophen-2-ylmethyl)methylamide are obtained. Yield = 35%.

### b. Ethyl 3-[4-(4,4,5,5-tetramethyl[1,3,2]dioxaborolan-2-yl)phenyl]acrylate

1.1 g (4.3 mmol, 1.1 eq) of pinacoldiborane are added to a mixture of 1.0 g (3.9 mmol, 1 eq) of ethyl 4-bromocinnamate and 1.1 g (11.7 mmol, 3 eq) of potassium acetate in the presence of 142 mg (0.19 mmol, 5 mol%) of diphenylphosphinoferrocenepalladium dichloride (PdCl₂dppf) in 10 mL of dimethylformamide. The mixture is stirred for 2 hours at 70°C. The reaction is stopped by adding 20 ml of water and is then extracted with ethyl acetate. The organic phases are combined and dried over sodium sulfate. The solvents are evaporated off and the residue is then chromatographed on silica gel (90/10 heptane/ethyl acetate). 1.15 g of ethyl 3-[4-(4,4,5,5-tetramethyl[1,3,2]dioxaborolan-2-yl)phenyl]acrylate are obtained in the form of an oil. Yield = 98%.

### c. Ethyl 3-(4-{5-[(methyloctanoylamino)methyl]thiophen-3-yl}phenyl)acrylate

300 mg (0.90 mmol, 1. 0 eq) of octanoyl(4-bromothiophen-2-ylmethyl)methylamide and 326 mg (1.08 mmol, 1.2 eq) of ethyl 3-[4-(4, 4, 5, 5-tetramethyl[1,3,2]dioxaborolan-2-yl)phenyl]acrylate are dissolved in 4 ml of a 5/1 mixture of dimethylformamide and of 2M potassium phosphate solution. 52 mg (0.04 mmol, 5 mol%) of tetrakis(triphenylphosphine)-palladium are added and the reaction mixture is then stirred for 2 hours at 80°C. The reaction is stopped by adding 30 mL of water and is then extracted with ethyl acetate. The organic phases are combined and dried over sodium sulfate. The solvents are evaporated off and the residue is then chromatographed on silica gel (80/20 to 60/40 heptane/ethyl acetate). 250 mg of ethyl 3-(4-{5-[(methyloctanoylamino)methyl]thiophen-3-yl}phenyl)acrylate are obtained. Yield = 65%.

### d. 3-(4-{5-[(Methyloctanoylamino)methyl]thiophen-3-yl}phenyl)acrylic acid

230 mg (5.8 mmol, 10 eq) of sodium hydroxide are added to a solution of 250 mg (0.58 mmol, 1 eq) of ethyl 3-(4-{5-[(methyloctanoylamino)methyl]thiophen-3-yl}phenyl)acrylate in 4 mL of 9/1 tetrahydrofuran/methanol. The reaction mixture is stirred for 2 hours at room temperature. The reaction is stopped by adding 20 mL of water and 3 mL of acetic acid and is then extracted with ethyl acetate. The organic phases are combined and dried over sodium sulfate. The solvents are evaporated off and the residue is then chromatographed on silica gel (90/10 dichloromethane/methanol). The oil obtained is crystallized from pentane. 80 mg of 3-(4-{5-[(methyloctanoylamino)methyl]thiophen-3-yl}phenyl)acrylic acid are obtained. Yield = 35%.
m.p. = 175°C.
**¹H NMR** (CDCl₃, 400 MHz): 0.88 (m, 3H); 1.33 (m, 8H); 1.70 (m, 2H); 2.38&2.48 (2t(rotamers), *J* = 7.6 Hz, 2H); 3.05&3.06 (2s(rotamers), 3H); 4.71&4.76 (2s(rotamers), 2H); 6.47&6.49 (2d(rotamers), *J* = 15.9 Hz, 1H); 6.76 (m, 2H); 7.28 (m, 1H); 7.44 (m, 1H); 7.60 (m, 4H); 7.80 (2d(rotamers), *J* = 15.9 Hz, 1H).

### Example 5: 3-(4-{5-[(Methylootanoylamino)methyl]thio-phen-3-yl}phenyl)propanoic acid

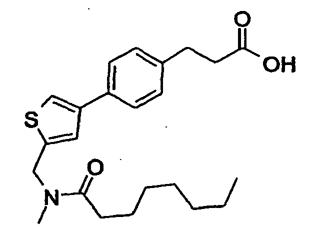

### a. Ethyl 3-[4-(4,4,5,5-tetramethyl[1,3,2]dioxaborolan-2-yl)phenyl]propanoate

A solution of 8 g (26.4 mmol) of ethyl 3-[4-(4,4,5,5-tetramethyl[1,3,2]dioxaborolan-2-yl)phenyl]acrylate in 30 mL of methanol is stirred for one hour at room temperature in the presence of 400 mg of 10% palladium-on-charcoal under a hydrogen atmosphere. The catalyst is filtered off and the solvents are then evaporated off. 7.8 g of ethyl 3-[4-(4,4,5,5-tetramethyl[1,3,2]dioxaborolan-2-yl)phenyl]propanoate are obtained. Yield = 96%.

### b. Ethyl 3-(4-{5-[(methyloctanoylamino)methyl]thiophen-3-yl}phenyl)propanoate

430 mg (1.29 mmol, 1 eq) of octanoyl(4-bromothiophen-2-ylmethyl)methylamide and 470 mg (1.55 mmol, 1.2 eq) of ethyl 3-[4-(4,4,5,5-tetramethyl[1,3,2]dioxaborolan-2-yl)phenyl]propanoate are dissolved in 4 ml of a 6/1 mixture of dimethylformamide and of 2M potassium phosphate solution. 15 mg (0.064 mmol, 5 mol%) of palladium acetate and 45 mg (0.129 mmol, 10 mol%) of dicyclohexyl 2-biphenylphosphine are added. The mixture is stirred for 2 hours at 90°C. The reaction is stopped by adding 30 mL of water and is then extracted with ethyl acetate. The organic phases are combined and dried over sodium sulfate. The solvents are evaporated off and the residue is then chromatographed on silica gel (80/20 to 70/30 heptane/ethyl acetate). 245 mg of ethyl 3-(4-{5-[(methyloctanoylamino)methyl]thiophen-3-yl}phenyl)propanoate are obtained. Yield = 44%.

### c. 3-(4-{5-[(Methyloctanoylamino)methyl]thiophen-3-yl}phenyl)propanoic acid

100 mg (2.5 mmol, 4 eq) of sodium hydroxide are added to a solution of 245 mg (0.57 mmol, 1 eq) of ethyl 3-(4-{5-[(methyloctanoylamino)methyl]thiophen-3-yl}phenyl)propanoate in 4 mL of 9/1 tetrahydrofuran/methanol. The reaction mixture is stirred for two hours at room temperature. The reaction is stopped by adding 20 mL of water and 3 mL of acetic acid and is then extracted with ethyl acetate. The organic phases are combined and dried over sodium sulfate. The solvents are evaporated off and the residue is then chromatographed on silica gel (90/10 dichloromethane/methanol). The oil obtained is crystallized from pentane. 85 mg of 3-(4-{5-[(methyloctanoylamino)methyl]thiophen-3-yl}phenyl)propanoic acid are obtained. Yield = 37%.
**¹H NMR** (CDCl₃, 400 MHz): 0.89 (m, 3H); 1.32 (m, 8H); 1.70 (m, 2H); 2.37&2.48 (2t(rotamers), *J* = 7.2 Hz, 2H); 2.71 (t, 2H, *J* = 7.6 He); 2.99 (m, 2H); 3.02 (s, 3H); 4.74&4.69 (2s(rotamers), 2H); 7.18-7.34 (m, 4H); 7. 50 (d, *J* = 3.2 Hz, 2H).

### Example 6: 3-{4-[6-(3-Heptyl-1-methylureido)pyrid-2-yl]phenyl}propanoic acid

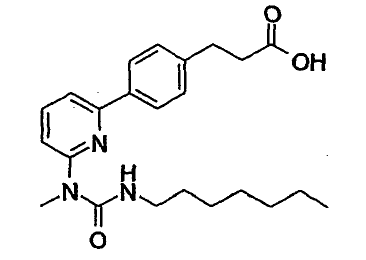

### a. tert-Butyl (6-bromopyrid-2-yl)carbamate

62 g (284 mmol) of di-tert-butyl dicarbonate diluted in 200 ml of dichloromethane are added dropwise to a solution of 49.2 g (284 mmol) of 2-amino-6-bromopyridine, 43.4 ml (312 mmol) of triethylamine and 3.5 g (28.4 mmol) of 4-dimethylaminopyridine in 400 ml of dichloromethane. The reaction medium is stirred at room temperature for 18 hours. After addition of water and extraction with dichloromethane, the organic phase is dried over magnesium sulfate, filtered and evaporated. The residue obtained is purified by chromatography on a column of silica eluted with a 95/5 heptane/ethyl acetate mixture. 39 g (50%) of tert-butyl (6-bromopyrid-2-yl)carbamate are obtained in the form of a white solid.

### b. tert-Butyl 6-bromopyrid-2-yl-N-methylcarbamate

To a solution of 39 g (14.3 mmol) of tert-butyl (6-bromopyrid-2-yl)carbamate in 400 ml of dimethylformamide are added portionwise 6.9 g (17.2 mmol) of 60% sodium hydride in oil. After stirring for 20 minutes at room temperature, 17.8 ml (28.6 mmol) of methyl iodide are added dropwise. The reaction medium is stirred at room temperature for 18 hours, taken up in water and extracted with ethyl acetate. The organic phase is dried over magnesium sulfate, filtered and evaporated.

### c. Ethyl 3-{4-[6-(tert-butoxycarbonylmethylamino)pyrid-2-yl]phenyl}acrylate

1.25 g (4.13 mmol, 1.2 eq) of ethyl 3-[4-(4,4,5,5-tetramethyl[1,3,2]dioxaborolan-2-yl)phenyl]acrylate and 1.0 g (3.48 mmol, 1 eq) of tert-butyl (6-bromopyrid-2-yl)-methylcarbamate are dissolved in 15 ml of a 6/1 mixture of dimethylformamide and of 2M potassium phosphate solution. 200 mg (0.173 mmol, 5 mol%) of tetrakis(triphenylphosphine)palladium are added. The mixture is stirred for 3 hours at 90°C. The reaction is stopped by adding 30 mL of water and is then extracted with ethyl acetate. The organic phases are combined and dried over sodium sulfate. The solvents are evaporated off and the residue is then chromatographed on silica gel (80/20 to 70/30 heptane/ethyl acetate). 850 mg of ethyl 3-{4-[6-(tert-butoxycarbonylmethylamino)pyrid-2-yl]phenyl}acrylate are obtained. Yield = 64%.

### d. Ethyl 3-{4-[6-(3-heptyl-1-methylureido)pyrid-2-yl]phenyl}acrylate

300 mg (0.78 mmol) of ethyl 3-{4-[6-(tert-butoxycarbonylmethylamino)pyrid-2-yl]phenyl}acrylate are dissolved in 5 mL of dichloromethane. 2 mL of trifluoroacetic acid are added. The reaction mixture is stirred for 3 hours at room temperature. The reaction is stopped by adding 30 mL of saturated sodium hydrogen carbonate solution and is then extracted with ethyl acetate. The organic phases are combined and dried over sodium sulfate. The solvents are evaporated off and the oil obtained is then dissolved in 5 ml of dichloromethane in the presence of 1 ml of triethylamine. 630 µL (3.9 mmol, 5 eq) of heptyl isocyanate are added along with 100 mg of dimethylaminopyridine. The reaction mixture is stirred for 16 hours at reflux. The reaction is stopped by adding 30 mL of water and is then extracted with ethyl acetate. The organic phases are combined and dried over sodium sulfate. The solvents are evaporated off and the residue is then chromatographed on silica gel (80/20 to 70/30 heptane/ethyl acetate). 170 mg of ethyl 3-{4-[6-(3-heptyl-1-methylureido)pyrid-2-yl]phenyl}acrylate are obtained. Yield = 51%.

### e. 3-{4-[6-(3-Heptyl-1-methylureido)pyrid-2-yl]phenyl}-propanoic acid

170 mg (0.4 mmol) of ethyl 3-{4-[6-(3-heptyl-1-methylureido)pyrid-2-yl]phenyl}acrylate are dissolved in 5 ml of methanol. 50 mg of 10% palladium-on-charcoal are added. The reaction mixture is stirred for 3 hours at room temperature under a hydrogen atmosphere. The catalyst is filtered off and the solvents are then evaporated off and the oil obtained is used directly in the following reaction. 170 mg of sodium hydroxide are added to the solution of ethyl 3-{4-[6-(3-heptyl-1-methylureido)pyrid-2-yl]phenyl}propanoate in 5 ml of 9/1 tetrahydrofuran/methanol. The reaction mixture is stirred for two hours at room temperature. The reaction is stopped by adding 20 mL of water and 3 mL of acetic acid and is then extracted with ethyl acetate. The organic phases are combined and dried over sodium sulfate. The solvents are evaporated off and the residue is then chromatographed on silica gel (7/3 to 1/1 heptane/EtOAc). 110 mg of 3-{4-[6-(3-heptyl-1-methylureido)pyrid-2-yl]phenyl}propanoic acid are obtained. Yield = 69% over two steps.
m.p. = 108°C
**¹H NMR** (CDCl₃, 400 MHz): 0.87 (t, *J* = 7.0 Hz, 3H); 1.20-1.36 (m, 8H); 1.61 (m, 2H); 2.76 (t, *J* = 7.7 Hz, 2H); 3.06 (t, 2H, *J* = 7.7 Hz); 3.38 (q, *J* = 5.5 Hz, 2H); 3.47 (s, 3H); 6.94 (d, *J* = 8.4 Hz, 1H); 7.35 (m, 3H); 7.76 (t, *J* = 7.8 Hz, 1H); 7.81 (d, *J* = 8.0 Hz, 2H); 10.48 (s, 1H) .

### Example 7: 3-{6-[3-(3-Heptyl-1-methylureido)phenyl]-pyrid-2-yl}propanoic acid

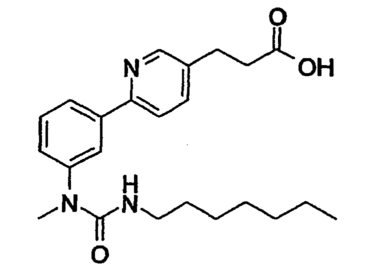

### a. tert-Butyl (3-bromophenyl)carbamate

To a mixture of 94 g (549 mmol) of 3-bromoaniline and 1 1 of dichloromethane are added portionwise, at room temperature, 120 g (549 mmol) of di-tert-butyl dicarbonate. After stirring for 18 hours, the reaction medium is poured into ice-water and then extracted with dichloromethane. The organic phase is separated out by settling of the phases, dried over magnesium sulfate and evaporated. 138 g of tert-butyl (3-bromophenyl)carbamate are obtained in a yield of 98%.

### b. tert-Butyl (3-bromophenyl)-N-methylcarbamate

To a solution of 114 g (447 mmol) of tert-butyl (3-bromophenyl)carbamate in 800 ml of DMF are added portionwise 19 g (475 mmol) of sodium hydride (60% in oil) and the reaction medium is stirred until the evolution of gas has ceased. 29.3 ml (470 mmol) of methyl iodide are added dropwise and stirring is continued for 18 hours. The reaction medium is poured into ice-water and extracted with ethyl acetate. The organic phase is separated out by settling of the phases, dried over magnesium sulfate and evaporated. 115 g of tert-butyl (3-bromophenyl)-N-methylcarbamate are obtained in a yield of 95%.

### c. (3-Bromophenyl)methylamine

5 ml of trifluoromethanesulfonic acid are added to a solution of 3.6 g (12.7 mmol) of tert-butyl (3-bromophenyl)-N-methylcarbamate in 15 mL of dichloromethane. The reaction medium is stirred for 1 hour at room temperature (RT). The reaction is stopped by adding 50 ml of saturated sodium hydrogen carbonate solution and then extracted with ethyl acetate. The solvents are evaporated off and the residue is then chromatographed on silica gel, eluting with 1/1 heptane/ethyl acetate. 2.14 g of oil are obtained. 90% yield.

### d. N-Methyl-3-aminophenylboronic acid

37.6 g (202 mmol, 1 eq) of (3-bromophenyl)methylamine (obtained as in 1c) are dissolved in 300 mL of tetrahydrofuran. The reaction mixture is cooled to -70°C and 166 mL (242 mmol, 1.2 eq) of 1.5 M methyllithium are then added slowly, while keeping the temperature at -70°C. The reaction mixture is stirred for 1 hour at -70°C. 306 mL (444 mmol, 2.2 eq) of 1.46 M tert-butyllithium are added, while keeping the temperature at -70°C. The reaction mixture is stirred for 45 minutes at -70°C. 103.5 ml (808 mmol, 4 eq) of trimethyl borate are added at -65°C and the reaction mixture is then warmed to room temperature. The reaction is stopped by adding 1 L of 1N hydrochloric acid. The pH is adjusted to pH 5 and the reaction medium is then extracted with n-butanol. The organic phases are combined and dried over sodium sulfate. The solvents are evaporated off and the residue is then chromatographed on silica gel (70/30 heptane/ethyl acetate). 11.3 g of N-methyl-3-aminophenylboronic acid are obtained. Yield = 40%.

### e. [3-(5-Bromopyrid-2-yl)phenyl]methylamine

2.2 g (9.5 mmol, 1.2 eq) of 2,5-dibromopyridine are dissolved in 3 mL of an 8/2 mixture of dimethylformamide/2M potassium phosphate. 1.2 g (7.9 mmol, 1 eq) of N-methyl-3-aminophenylboronic acid are added along with 456 mg (0.39 mmol, 5 mol%) of tetrakis(triphenylphosphine)palladium. The mixture is stirred for 3 hours at 90°C. The reaction is stopped by adding 30 mL of water and is then extracted with ethyl acetate. The organic phases are combined and dried over sodium sulfate. The solvents are evaporated and the residue is then chromatographed on silica gel (80/20 to 70/30 heptane/ethyl acetate). 1.07 g of [3-(5-bromopyrid-2-yl)phenyl]methylamine are obtained.
Yield = 52%.

### f. tert-Butyl [3-(5-bromopyrid-2-yl)phenyl}methyl-carbamate

1.07 g (4 mmol, 1 eq) of [3-(5-bromopyrid-2-yl)phenyl]methylamine are dissolved in 10 mL of dichloromethane in the presence of 1 mL of triethylamine and 200 mg of dimethylaminopyridine. 1.7 g (8 mmol, 2 eq) of tert-butyl dicarbonate are added and the reaction mixture is stirred overnight at 40°C. The reaction is stopped by adding 30 ml of water and is then extracted with ethyl acetate. The organic phases are combined and dried over sodium sulfate. The solvents are evaporated off and the residue is then chromatographed on silica gel (70/30 heptane/ethyl acetate). 1.18 g of tert-butyl [3-(5-bromopyrid-2-yl)phenyl}methylcarbamate are obtained. Yield = 81%.

### g. Ethyl 3-{6-[3-(tert-butoxycarbonylmethylamino)-phenyl}pyrid-3-yl}acrylate

1.18 g of tert-butyl [3-(5-bromopyrid-2-yl)phenyl}methylcarbamate (3.25 mmol, 1 eq) are dissolved in 10 mL of dimethylformamide and 1 ml of triethylamine. 110 mg (0.48 mmol, 15%) of palladium acetate and 296 mg (0.96 mmol, 30%) of o-tolylphosphine are added, along with 1 mL of ethyl acrylate. The reaction mixture is stirred overnight at 80°C. The reaction is stopped by adding 30 ml of water and is then extracted with ethyl acetate. The organic phases are combined and dried over sodium sulfate. The solvents are evaporated off and the residue is then chromatographed on silica gel (70/30 heptane/ethyl acetate). 852 mg of ethyl 3-{6-[3-(tert-butoxycarbonylmethylamino)phenyl}pyrid-3-yl}acrylate are obtained. Yield = 68%.

### h. Ethyl 3-[6-(3-methylaminophenyl)pyrid-3-yl]propanoate

A solution of 852 mg (2.23 mmol) of ethyl 3-{6-[3-(tert-butoxycarbonylmethylamino)phenyl}pyrid-3-yl}acrylate in 10 mL of methanol is stirred for 4 hours at room temperature in the presence of 100 mg of 10% palladium-on-charcoal under a hydrogen atmosphere. The catalyst is filtered off and the solvents are then evaporated off. The oil obtained is dissolved in 15 mL of dichloromethane. 2 mL of trifluoroacetic acid are added and the reaction mixture is stirred for 4 hours at room temperature. The reaction is stopped by adding 100 mL of saturated sodium hydrogen carbonate solution and is then extracted with ethyl acetate. The organic phases are combined and dried over sodium sulfate. The solvents are evaporated off and the residue is then chromatographed on silica gel (70/30 heptane/ethyl acetate). 710 mg of ethyl 3-[6-(3-methylaminophenyl)pyrid-3-yl]propanoate are obtained. Yield = 68%.

### i. Ethyl 3-{6-[3-(3-heptyl-1-methylureido)pheny]pyrid-3-yl}propanoate

572 µL (3.5 mmol, 2 eq) of heptyl isocyanate are added to a solution of 500 mg (1.76 mmol, 1 eq) of ethyl 3-[6-(3-methylaminophenyl)pyrid-3-yl]propanoate in 10 mL of a 4/1 tetrahydrofuran/triethylamine mixture. The reaction mixture is stirred for 24 hours at 40°C. The reaction is stopped by adding 30 ml of water and is then extracted with ethyl acetate. The organic phases are combined and dried over sodium sulfate. The solvents are evaporated off and the residue is then chromatographed on silica gel (70/30 heptane/ethyl acetate). 488 mg of ethyl 3-{6-[3-(3-heptyl-1-methylureido)phenyl]pyrid-3-yl}propanoate are obtained. Yield = 65%.

### j. 3-{6-[3-(3-Heptyl-1-methylureido)phenyl]pyrid-3-yl}propanoic acid

200 mg (5 mmol, 4 eq) of sodium hydroxide are added to a solution of 488 mg (1.14 mmol, 1 eq) of ethyl 3-{6-[3-(3-heptyl-1-methylureido)phenyl]pyrid-3-yl}propanoate in 6 mL of 9/1 tetrahydrofuran/methanol. The reaction mixture is stirred for 3 hours at room temperature. The reaction is stopped by adding 20 ml of water and 3 ml of acetic acid and is then extracted with ethyl acetate. The organic phases are combined and dried over sodium sulfate. The solvents are evaporated off and the residue is then chromatographed on silica gel (70/30 to 50/50 heptane/ethyl acetate). The oil obtained is crystallized from pentane. 442 mg of 3-{6-[3-(3-heptyl-1-methylureido)phenyl]pyrid-3-yl}propanoic acid are obtained. Yield = 94%. m.p. = 118°C.
**¹H NMR** (CDCl₃, 400 MHz): 0.85 (t, *J* = 6.8 Hz, 3H); 1.25 (m, 8H); 1.40 (m, 2H); 2.74 (t, *J* = 7.4 Hz, 2H); 3.05 (t, *J* = 7.3 Hz, 2H); 3.17 (q, *J* = 6.0 Hz, 2H); 3.32 (s, 3H); 4.42 (m, 1H) ; 7.29 (m, 1H); 7.52 (t, *J* = 8.0 Hz, 1H); 7.66 (m, 2H); 7.86 (m, 2H); 8.63 (m, 1H).

### Example 8: 2-[4-(2-Carboxyethyl)phenyl]-4-(3-heptyl-1-methylureido)pyridinium acetate

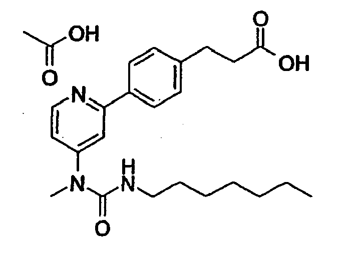

### a. tert-Butyl (2-chloropyrid-4-yl)carbamate

1.69 g (7.78 mmol, 1 eq) of tert-butyl dicarbonate dissolved in 50 ml of dichloromethane, in the presence of 95 mg of dimethylaminopyridine, are added to a solution of 1 g (7.78 mmol, 1 eq) of 4-amino-2-chloropyridine in 30 mL of dichloromethane in the presence of 1.19 mL (1.19 mmol, 1.1 eq) of triethylamine. The reaction mixture is stirred overnight at room temperature. The reaction is stopped by adding 100 ml of water and is then extracted with dichloromethane. The organic phases are combined and dried over sodium sulfate. The solvents are evaporated off and the residue is then chromatographed on silica gel (70/30 to 50/50 heptane/ethyl acetate). The oil obtained is crystallized from pentane. 1.40 g of tert-butyl (2-chloropyrid-4-yl)carbamate are obtained. Yield = 80%.

### b. tert-Butyl (2-chloropyrid-4-yl)methylcarbamate

A solution of 17.5 g (76.7 mmol, 1 eq) dissolved in 100 ml of dimethylformamide is added to a suspension of 3.57 g (92 mmol, 1.2 eq) of sodium hydride in 100 mL of dimethylformamide at 0°C. The reaction mixture is stirred for 30 minutes at room temperature. 9.26 ml (148 mmol, 2 eq) of methyl iodide are added to the reaction mixture and the reaction medium is then stirred for 2 hours at room temperature. The reaction is stopped by adding 100 mL of water and is then extracted with ethyl acetate. The organic phases are combined and dried over sodium sulfate. The solvents are evaporated off. 18 g of tert-butyl (2-chloropyrid-4-yl)methylcarbamate are obtained. Yield = 97%.

### c. Ethyl 3-{4-[4-(tert-butoxycarbonylmethylamino)pyrid-2-yl]phenyl}propanoate

500 mg (2.06 mmol, 1 eq) of tert-butyl (2-chloropyrid-4-yl)methylcarbamate and 750 mg (2.47 mmol, 1.2 eq) of ethyl 3-[4-(4,4,5,5-tetramethyl[1,3,2]dioxaborolan-2-yl)phenyl]propanoate (obtained as in 5a) are dissolved in 10 mL of an 8/2 mixture of dimethylformamide/2M potassium phosphate. 23 mg (0.123 mmol, 5 mol%) of palladium acetate and 71 mg (0.24 mmol, 10 mol%) of dicyclohexyldiphenylphosphine are added. The mixture is stirred for 4 hours at 90°C. The reaction is stopped by adding 30 ml of water and is then extracted with ethyl acetate. The organic phases are combined and dried over sodium sulfate. The solvents are evaporated off and the residue is then chromatographed on silica gel (80/20 to 70/30 heptane/ethyl acetate). 415 mg of ethyl 3-{4-[4-(tert-butoxycarbonylmethylamino)pyrid-2-yl]phenyl}propanoate are obtained. Yield = 52%.

### d. Ethyl 3-[4-(4-methylaminopyrid-2-yl)phenyl]propanoate

415 mg (1.08 mmol, 1 eq) of ethyl 3-{4-[4-(tert-butoxycarbonylmethylamino)pyrid-2-yl]phenyl}propanoate are dissolved in 10 mL of dichloromethane. 3 mL of trifluoroacetic acid are added and the reaction mixture is stirred for 4 hours at room temperature. The reaction is stopped by adding 100 mL of saturated sodium hydrogen carbonate solution and is then extracted with ethyl acetate. The organic phases are combined and dried over sodium sulfate. The solvents are evaporated off. 270 mg of ethyl 3-[4-(4-methylaminopyrid-2-yl)phenyl]propanoate are obtained. Yield = 88%.

### e. Ethyl 3-{4-[4-(3-heptyl-1-methylureido)pyrid-2-yl]phenyl}propanoate

270 mg (0.63 mmol, 1 eq) of ethyl 3-[4-(4-methylaminopyrid-2-yl)phenyl]propanoate are mixed with 0.6 mL (3.7 mmol, 6 eq) of heptyl isocyanate and then irradiated in a microwave machine for 2 times 30 minutes, while keeping the temperature constant at 100°C. The reaction mixture is chromatographed on silica gel (90/10 heptane/ethyl acetate). 260 mg of ethyl 3-{4-[4-(3-heptyl-1-methylureido)pyrid-2-yl]phenyl}propanoate are obtained. Yield = 64%.

### f. 2-[4-(2-Carboxyethyl)pheayl]-4-(3-heptyl-1-methylureido)pyridinium acetate

100 mg (2.5 mmol, 4 eq) of sodium hydroxide are added to a solution of 260 mg (0.61 mmol, 1 eq) of ethyl 3-{4-[4-(3-heptyl-1-methylureido)pyrid-2-yl]phenyl}propanoate in 5 ml of 9/1 tetrahydrofuran/methanol. The reaction mixture is stirred for two hours at room temperature. The reaction is stopped by adding 20 mL of water and 3 mL of acetic acid and is then extracted with ethyl acetate. The organic phases are combined and dried over sodium sulfate. The solvents are evaporated off and the residue is then crystallized from pentane. 143 mg of 2-[4-(2-carboxyethyl)phenyl)-4-(3-heptyl-1-methylureido)pyridinium acetate are obtained.
Yield = 59%. m.p. = 71-73°C.
**¹H NMR** (CDCl₃, 400 MHz): 0.88 (t, *J* = 7.2 Hz, 3H); 1.32 (m, 8H); 1.58 (m, 2H); 2.11 (s, 3H); 2.70 (t, *J* = 6.9 Hz, 2H); 3.00 (t, *J* = 6.9 Hz, 2H); 3.29 (q, *J* = 6.3 Hz, 2H); 3.38 (s, 3H); 6.14 (m, 1H); 7.12 (d, *J* = 5.6 Hz, 1H); 7.31 (d, *J* = 7.6 Hz, 2H); 7.51 (s, 1H); 7.79 (d, *J* = 7.6 Hz, 2H); 8.43 (d, *J* = 5.5 Hz, 1H).

### Example 9: 3-{5-[3-(1-Methyl-3-pentylureido)phenyl]-pyrimidin-2-yl)acrylic acid

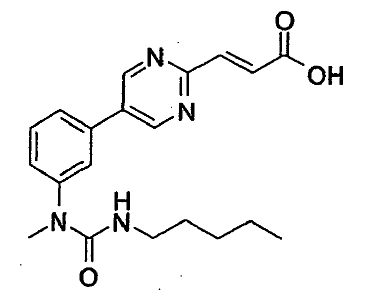

### a. 3-(N-Methyl-N-tert-butoxycarbonyl)phenylboronic acid

18.5 ml (46.2 mmol, 1.3 eq) of 2.5M butyllithium in hexane are added to a solution of 10 g (34.9 mmol, 1 eq) of tert-butyl (3-bromophenyl)methylcarbamate (obtained as in 7b) in 25 mL of tetrahydrofuran cooled to -78°C. The reaction mixture is stirred for 30 minutes at -78°C and 10 mL (41.9 mmol, 1.2 eq) of diisopropyl borate are then added dropwise. The reaction mixture is stirred at -78°C for 2 hours. The reaction is stopped by adding 70 ml of 1N hydrochloric acid at -10°C, and is then extracted with ethyl acetate. The organic phases are combined and dried over sodium sulfate. The solvents are evaporated off. 9.17 g of 3-(N-methyl-N-tert-butoxycarbonyl)phenylboronic acid are obtained in a crude yield of 100%.

### b. tert-Butyl [3-(2-bromopyrimidin-5-yl)phenyl]methyl-carbamate

22 ml of 2M potassium carbonate (43.9 mmol, 2.5 eq) are added dropwise to a solution of 3-(N-methyl-N-tert-butoxycarbonyl)phenylboronic acid (5.3 g, 21.06 mmol, 1.2 eq) and 5-bromo-2-iodopyrimidine (5 g, 17.6 mmol, 1 eq) in 50 ml of diethyl ether. The reaction medium is degassed with nitrogen for 30 minutes and tetrakis(triphenylphosphine)palladium (1.01 g, 0.88 mmol, 0.05 eq) is then added. The reaction mixture is stirred overnight at 100°C. The reaction is stopped by adding water and is then extracted with ethyl acetate. The solvents are evaporated off and the residue is then chromatographed on silica gel (90/10 heptane/ethyl acetate). 1.87 g of tert-butyl [3-(2-bromopyrimidin-5-yl)phenyl]methylcarbamate are obtained in the form of a beige-coloured solid in a yield of 30%.

### c. Methyl 3-{5-[3-(tert-butoxycarbonylmethylamino)-phenyl]pyrimidin-2-yl}acrylate

0.25 ml (2.75 mmol, 2 eq) of methyl acrylate, 9.3 mg (0.04 mmol, 0.3 eq) of palladium acetate and 25 mg (0.08 mmol, 0.06 eq) of tri-ortho-tolylphosphine are added to a solution of 0.5 g (1.37 mmol, 1 eq) of tert-butyl [3-(2-bromopyrimidin-5-yl)phenyl]methylcarbamate in 0.5 ml of dimethylformamide. The reaction mixture is heated at 80°C overnight. The reaction is stopped by adding 20 ml of water and is then extracted with ethyl acetate. The organic phases are combined and dried over sodium sulfate. The solvents are evaporated off and the residue is then chromatographed on silica gel (95/5 heptane/ethyl acetate). 377.7 mg of methyl 3-{5-[3-(tert-butoxycarbonylmethylamino)-phenyl]pyrimidin-2-yl}acrylate are obtained in the form of a beige-coloured solid. Yield = 75%.

### d. Methyl 3-(3'-methylaminobiphenyl-4-yl)acrylate

0.16 ml (2.04 mmol, 5 eq) of trifluoroacetic acid is added to a solution of 151 mg (0.4 mmol, 1 eq) of methyl 3-{5'-[3-(tert-butoxycarbonylmethylamino)-phenyl]pyrimidin-2-yl}acrylate in 3 ml of dichloromethane. The reaction mixture is stirred for eight hours at room temperature. The reaction is stopped by adding aqueous ammonia solution and is then extracted with ethyl acetate. The organic phases are combined and dried over sodium sulfate. The solvents are evaporated off and the residue is then taken up in heptane. 71.6 mg of methyl 3-(3'-methylaminobiphenyl-4-yl)acrylate are obtained in the form of a yellow solid. Yield = 65%.

### e. Methyl 3-{5-[3-(1-methyl-3-pentylureido)phenyl]-pyrimidine-2-acrylate

22 µL (0.53 mmol, 1 eq) of pentyl isocyanate are added dropwise to a solution of 71.6 mg (0.27 mmol, 1 eq) of methyl 3-[5-(3-methylaminophenyl)pyrimidin-2-yl]acrylate and 22 µL of triethylamine in 2 mL of dichloromethane. The reaction mixture is stirred for 2 days at room temperature. The reaction is stopped by adding 10 ml of water and is then extracted with ethyl acetate. The organic phases are combined and dried over sodium sulfate. The solvents are evaporated off and the residue is then chromatographed on silica gel (70/30 heptane/ethyl acetate). 37.6 mg of methyl 3-{5-[3-(1-methyl-3-pentylureido)phenyl]pyrimidine-2-acrylate are obtained in the form of white crystals. Yield = 38%.

### f. 3-{5-[3-(1-Methyl-3-pentylureido)phenyl]pyrimidin-2-yl}acrylic acid

10 mg (242.5 µmol, 2.5 eq) of sodium hydroxide pellets are added to a solution of 37.6 mg (97 µmol, 1 eq) of 3-{5-[3-(1-methyl-3-pentylureido)phenyl]pyrimidine-2-acrylate in 2 ml of tetrahydrofuran and 4 drops of water. The reaction mixture is stirred for 8 hours at room temperature. The reaction medium is concentrated and then diluted with 10 ml of water and washed with twice 10 ml of dichloromethane. The aqueous phase is acidified with 1N hydrochloric acid and then extracted with ethyl acetate. The organic phases are combined and dried over sodium sulfate. The solvents are evaporated off and the residue is then chromatographed on a preparative silica plate (95/5/0.5% dichloromethane/methanol/aqueous ammonia). 7.9 mg of 3-{5-[3-(1-methyl-3-pentylureido)phenyl]pyrimidin-2-yl}acrylic acid are obtained in the form of beige-coloured crystals.
Yield = 22%.
**¹H NMR** (CDCl₃, 400 MHz): 0.88 (t, *J* = 7 Hz, 3H); 1.27 (m, 4H); 1.42 (m, 2H); 3.18 (q, j = 6.8 Hz, 2H); 3.37 (s, 3H); 4.41 (t, *J* = 5.5 Hz, 1H); 6.67 (d, *J* = 16.2 Hz, 1H); 7.44 (d, *J* = 7.8 Hz, 1H); 7.58 (t, *J* = 7.8 Hz, 1H); 7.76 (d, *J* = 16.2 Hz, 1H); 8.42 (m, 2H); 8.99 (s, 2H).

### EXAMPLE 10: 3-{5-[3-(1-Methyl-3-pentylureido)phenyl]-pyrimidin-2-yl)propanoic acid

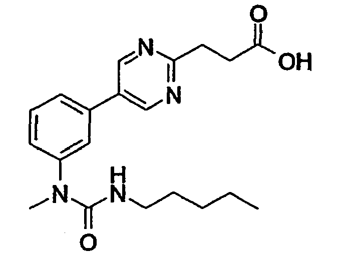

### a. Methyl 3-{5-[3-(tert-butoxycarbonylmethylamino)-phenyl]pyrimidin-2-yl}propanoate

30 mg of 10% Pd/C are added to a solution of 0.3 g (0.81 mmol, 1 eq) of methyl 3-{5-[3-(tert-butoxycarbonylmethylamino)phenyl]pyrimidin-2-yl}acrylate (obtained as in 9c) in 3 ml of methanol, in a PARR bomb. The reaction mixture is placed under a hydrogen pressure of 3 bar and is then heated at 50°C for 12 hours. The reaction medium is cooled to room temperature and then degassed with nitrogen and filtered through Celite. After evaporating off the solvents, the residue is chromatographed on silica gel (90/10 heptane/ethyl acetate). 118.2 mg of methyl 3-{5-[3-(tert-butoxycarbonylmethylamino)phenyl]pyrimidin-2-yl}propanoate are obtained in the form of a colourless oil. Yield = 40%.

### b. Methyl 3-[5-(3-methylaminophenyl)pyrimidin-2-yl]propanoate

0.12 ml (1.6 mmol, 5 eq) of trifluoroacetic acid is added to a solution of 118 mg (0.32 mmol, 1 eq) of methyl 3-{5-[3-(tert-butoxycarbonylmethylamino)-phenyl]pyrimidin-2-yl}propanoate in 4 ml of dichloromethane. The reaction mixture is stirred for 16 hours at room temperature. The reaction is stopped by adding 20 mL of 1N sodium hydroxide solution and is then extracted with ethyl acetate. The organic phases are combined and dried over sodium sulfate. The solvents are evaporated off and the residue is then chromatographed on silica gel (99/1 dichloromethane/methanol). 163 mg of methyl 3-[5-(3-methylaminophenyl)pyrimidin-2-yl]propanoate are obtained in the form of a colourless oil. Yield = 99%.

### c. Methyl 3-{5-[3-(1-methyl-3-pentylureido)phenyl]-pyrimidin-2-yl}propanoate

133 mg (0.66 mmol, 1.1 eq) of 4-nitrophenyl chloroformate are added to a solution, cooled to 0°C, of 163 mg (0.6 mmol, 1 eq) of 3-[5-(3-methylaminophenyl)pyrimidin-2-yl]propanoate and 0.1 ml (0.72 mmol, 1.2 eq) in 5 ml of dichloromethane. The reaction mixture is stirred for 12 hours at room temperature. The reaction is stopped by adding 20 ml of water, dried over sodium sulfate and filtered, and the solvents are evaporated off. The residue is taken up in 3 ml of dimethylformamide and 69 µl (0.6 mmol, 1 eq) of N-amylamine are then added. The reaction medium is heated at 80°C for 4 hours and then poured onto ice and extracted with ethyl acetate. The organic phases are combined and dried over sodium sulfate, the solvents are concentrated and the residue is then chromatographed on silica gel (60/40 heptane/ethyl acetate).

111.9 mg of methyl 3-{5-[3-(1-methyl-3-pentylureido)phenyl]pyrimidin-2-yl}propanoate are obtained in the form of a yellow oil. Yield = 48%.

### d. 3-{5-[3-(1-Methyl-3-pentylureido)phenyl]pyrimidin-2-yl}propanoic acid

1.44 ml (1.45 mmol, 5 eq) of lithium hydroxide are added to a solution of 111 mg (0.29 mmol, 1 eq) of methyl 3-{5-[3-(1-methyl-3-pentylureido)-phenyl]pyrimidin-2-yl}propanoate in 3 mL of tetrahydrofuran. The reaction mixture is stirred for four hours at room temperature. The reaction is stopped by adding 10 ml of water, washed with ether and then acidified with 1N hydrochloric acid solution, and then extracted with ethyl acetate. The organic phases are combined and dried over sodium sulfate. The solvents are evaporated off and the residue is then crystallized from ether. 66.7 mg of 3-{5-[3-(1-methyl-3-pentylureido)phenyl]pyrimidin-2-yl}propanoic acid are obtained in the form of white crystals. Yield = 48%.
**¹H NMR** (CDCl₃, 400 MHz): 0.84 (t, *J* = 7.0 Hz, 3H); 1.25 (m, 4H); 1.39 (m, 2H); 2.78 (t, *J* = 7.3 Hz, 2H); 3.03 (t, *J* = 7.1, 2H); 3.17 (q, *J* = 7.2 Hz, 2H); 3.19 (s, 3H); 4.41 (t, *J* = 5.4 Hz, 1H); 7.38 (m, 1H); 7.55 (t, *J* = 8 Hz, 1H); 8.35 (m, 2H); 8.75 (s, 2H).

### EXAMPLE 11: 2-[2-Butoxy-4-(2-carboxyethyl)phenyl]-6-(1-methyl-3-pentylureido)pyridinium hydrochloride

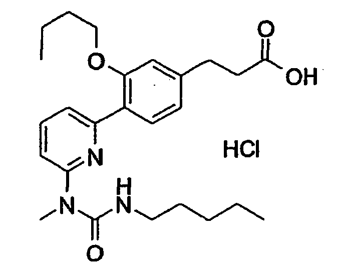

### a. 6-Methylamino-2-bromopyridine

30 g (0.13 mol) of 2,6-dibromopyridine are added to a solution of 225 ml (2.39 mol) of methylamine in ethanol (33% by weight, Aldrich) precooled to 0°C. The reaction is heated at 80°C with stirring for 20 hours, in a glass system equipped with a manometer. The reaction is monitored by TLC control. The reaction medium is cooled to 0°C and the assembly is opened. The slightly brown solution thus obtained is concentrated under vacuum to a volume of 60 ml, and water (240 ml) is then added, followed by addition of aqueous sodium carbonate solution (2N, 240 ml). The beige-coloured precipitate formed is filtered off, washed with water and dissolved in dichloromethane (200 ml). The solution is dried over magnesium sulfate and the solvent is evaporated off. The addition of heptane allows the precipitation of 17.5 g (74%) of 6-methylamino-2-bromopyridine in the form of a beige-coloured powder.

### b. 1-(6-Bromopyrid-2-yl)-1-methyl-3-pentylurea

2 g of 6-methylamino-2-bromopyridine are mixed with 3.0 mL of pentyl isocyanate. The mixture is heated for 12 hours at 100°C. The residue is chromatographed on silica (9/1 heptane/ethyl acetate). 1.8 g of 1-(6-bromopyrid-2-yl)-1-methyl-3-pentylurea are obtained. Yield = 56%.

### c. Methyl 3-butoxy-4-iodobenzoate

21.5 mL (0.189 mol, 1.5 eq) of 1-iodobutane are added to a solution of 35.03 g (0.126 mol, 1 eq) of methyl 3-hydroxy-3-iodobenzoate in 350 ml of methyl ethyl ketone in the presence of 52.24 g (0.378 mol, 3 eq) of potassium carbonate. The reaction medium is heated at 85°C for 2 hours. The solid is filtered off and the solvent is evaporated off. The solid obtained is washed with heptane to give 41.78 g of methyl 3-butoxy-4-iodobenzoate in the form of white crystals. Yield = 99%.

### d. (3-Butoxy-4-iodophenyl)methanol

8.17 g (0.375 mol, 3 eq) of lithium borohydride are added to a solution of 41.78 g (0.125 mol, 1 eq) of methyl 3-butoxy-4-iodobenzoate in 210 ml of tetrahydrofuran. The reaction medium is heated at 60°C for 2 hours and then hydrolysed cautiously with ice-cold saturated ammonium chloride solution. The reaction medium is neutralized with concentrated hydrochloric acid and then extracted with ethyl acetate. The organic phases are washed with water and dried over magnesium sulfate. The solvent is evaporated off to give 38.31 g of (3-butoxy-4-iodophenyl)methanol in the form of a whitish oil. Yield = 100%.

### e. 3-Butoxy-4-iodobenzaldehyde

89.5 g (0.875 mol, 7 eq) of manganese dioxide are added to a solution of 38.30 g (0.125 mol, 1 eq) of (3-butoxy-4-iodophenyl)methanol in 250 mL of dichloromethane. The reaction medium is stirred at room temperature for 18 hours and then filtered through silica gel. The solvent is evaporated off to give 29.61 g of 3-butoxy-4-iodobenzaldehyde in the form of an orange-coloured oil. Yield = 78%.

### f. Methyl (E)-3-(3-butoxy-4-iodophenyl)acrylate

65.08 g (0.195 mol, 2 eq) of methyl (triphenylphosphoranylidene)acetate are added to a solution of 29.60 g (0.097 mol, 1 eq) of 3-butoxy-4-iodobenzaldehyde in 360 ml of toluene. The reaction mixture is refluxed for 2 hours. The solvent is evaporated off and the oil obtained is chromatographed on silica gel (50/50 heptane/dichloromethane). 30.47 g of methyl (E)-3-(3-butoxy-4-iodophenyl)acrylate are obtained in the form of pale yellow crystals.
Yield = 87%.

### g. Methyl (E)-3-[3-Butoxy-4-(4,4,5,5-tetramethyl[1,3,2]dioxaborolan-2-yl)phenyl]acrylate

2.0 g of methyl(E)-3-(3-butoxy-4-iodophenyl)acrylate are dissolved in 10 mL of dimethylformamide, and 1.8 g of pinacoldiborane are added, along with 226 mg of diphenylphosphinoferrocenepalladium dichloride and 1.6 g of potassium acetate. The mixture is stirred for 3 hours at 90°C. The reaction is hydrolysed and extracted with ethyl acetate. After evaporation of the solvents and chromatography on silica gel (eluent = 7/3 heptane/ethyl acetate), 1.10 g of methyl(E)-3-[3-butoxy-4-(4,4,5,5-tetramethyl[1,3,2]dioxaborolan-2-yl)phenyl]acrylate are obtained. Yield = 55%.

### h. Methyl (E)-3-{3-butoxy-4-[6-(1-methyl-3-pentylureido)pyrid-2-yl]phenyl}acrylate

496 mg of methyl(E)-3-[3-butoxy-4-(4,4,5,5-tetramethyl[1,3,2]dioxaborolan-2-yl)phenyl]acrylate are dissolved in 5 mL of dimethylformamide and 1 mL of 2N potassium phosphate solution. 350 mg of 1-(6-bromopyrid-2-yl)-1-methyl-3-phenylurea are added, along with 5 mg of palladium acetate and 16 mg of dicyclohexylbiphenylphosphine. The mixture is stirred for 3 hours at 90°C and is then hydrolysed and extracted with ethyl acetate. After evaporation of the solvents and chromatography on silica gel (eluent = 7/3 heptane/ethyl acetate), 430 mg of methyl (E)-3-{3-butoxy-4-[6-(1-methyl-3-pentylureido)pyrid-2-yl]phenyl}acrylate are obtained. Yield = 82%.

### i. Methyl 3-{3-butoxy-4-[6-(1-methyl-3-pentylureido)pyrid-2-yl]phenyl}propionate

A solution of 430 mg of methyl(E)-3-{3-butoxy-4-[6-(1-methyl-3-pentylureido)pyrid-2-yl]phenyl}acrylate in 5 mL of methanol is stirred for 3 hours at room temperature in the presence of 100 mg of palladium-on-charcoal, under a hydrogen atmosphere. After filtering off the palladium and evaporating off the solvents, 370 mg of methyl 3-{3-butoxy-4-[6-(1-methyl-3-pentylureido)pyrid-2-yl]phenyl}propionate are obtained. Yield = 86%.

### j. 2-[2-Butoxy-4-(2-carboxyethyl)phenyl]-6-(1-methyl-3-pentylureido)pyridinium hydrochloride

300 mg of sodium hydroxide are added to a solution of 370 mg of methyl 3-{3-butoxy-4-[6-(1-methyl-3-pentylureido)pyrid-2-yl]phenyl}propionate dissolved in 15 ml of an 8/2 tetrahydrofuran/methanol mixture. After stirring for 4 hours at room temperature, the reaction is stopped with water and acetic acid and is then extracted with ethyl acetate. The solvents are evaporated off. The residual oil is chromatographed on silica gel (7/3 heptane/ethyl acetate). 5 mL of a solution of hydrogen chloride in ethyl acetate are added and the hydrochloride is precipitated by addition of isopropyl ether. After filtration, 205 mg of 2-[2-butoxy-4-(2-carboxyethyl)phenyl]-6-(1-methyl-3-pentylureido)pyridinium hydrochloride are obtained.
Yield = 53%.
**¹H NMR** (DMSO, 400 MHz): 0.80(m, 3H); 0.93 (m, 3H); 1.24 (m, 4H); 1.44 (m, 4H); 1.74 (m, 2H); 2.64 (m, 2H); 2.92 (m, 2H); 3.39 (s, 3H); 4.10 (m, 2H); 6.98 (d, *J* = 8 Hz, 1H); 7.13 (s, 1H); 7.24 (d, *J* = 8 Hz, 1H); 7.56 (m, 2H); 7.97 (m, 1H); 9.70 (s, 1H).

### EXAMPLE 12: 2-[2-Butoxy-4-(2-carboxyethyl)phenyl]-6-(1-methyl-3-heptylureido)pyridinium hydrochloride

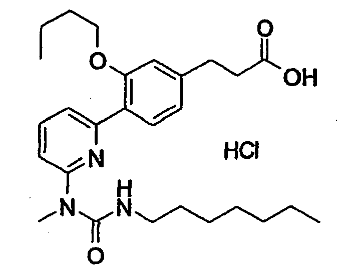

### a. (6-Bromopyrid-2-yl)-1-methyl-3-heptylurea

2 g of 6-methylamino-2-brompyridine (*prepared according to the procedure of Example 11, step a*) are mixed with 3.0 mL of heptyl isocyanate. The mixture is heated for 12 hours at 100°C. The residue is chromatographed on silica gel (9/1 heptane/ethyl acetate). 2.45 g of 1-(6-bromopyrid-2-yl)-1-methyl-3-heptylurea are obtained. Yield = 71%.

### b. 2-[2-Butoxy-4-(2-carboxyethyl)phenyl]-6-(1-methyl-3-heptylureido)pyrdinium hydrochloride

300 mg of sodium hydroxide are added to a solution of 377 mg of methyl 3-{3-butoxy-4-[6-(1-methyl-3-heptylureido)pyrid-2-yl]phenyl}propionate (*prepared according to the procedure of Example 11, steps c to i, replacing the 1-(6-bromopyrid-2-yl)-1-methyl-3-pentylurea with 1-(6-bromopyrid-2-yl)-1-methyl-3-heptylurea in step h*) dissolved in 15 mL of an 8/2 tetrahydrofuran/methanol mixture. After stirring for 4 hours at room temperature, the reaction is stopped with water and acetic acid and is then extracted with ethyl acetate. The solvents are evaporated off. The residual oil is chromatographed on silica gel (7/3 heptane/ethyl acetate). 5 mL of a solution of hydrogen chloride in ethyl acetate are added, and the hydrochloride is precipitated by addition of isopropyl ether. After filtration, 220 mg of 2-[2-butoxy-4-(2-carboxyethyl)phenyl]-6-(1-methyl-3-heptylureido)pyridinium hydrochloride are obtained.
Yield = 55%.
**¹H NMR** (DMSO, 400 MHz): 0.80 (m, 3H); 0.88 (m, 3H); 1.16 (m, 8H); 1.37 (m, 4H); 1.67 (m, 2H); 2.50 (m, 2H); 2.85 (m, 2H); 3.16 (m, 2H); 3.33 (s, 3H); 4.04 (m, 2H); 6.92 (d, J = 8 Hz, 1H); 7.07 (s, 1H); 7.17 (d, *J* = 8 Hz, 1H); 7.50 (m, 2H); 7.91 (m, 1H); 9.70 (s, 1H).

### EXAMPLE 13: 2-[4-(2-Carboxyethyl)-2-ethoxyphenyl]-6-(3-heptyl-1-methylureido)pyridinium hydrochloride

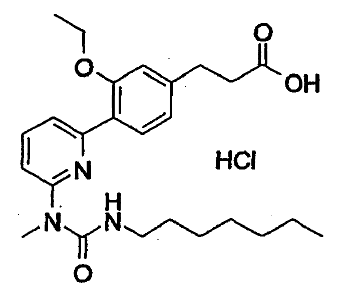

### a. 3-Ethoxy-4-(4,4,5,5-tetramethyl[1,3,2]dioxaborolan-2-yl)benzaldehyde

2.0 g of ethylvanillin are dissolved in 10 mL of dichloromethane and 3 mL of triethylamine are then added. 2.36 mL of triflic anhydride are added slowly at 0°C. After stirring for 1 hour at 0°C, the reaction is hydrolysed with sodium hydrogen carbonate solution and then extracted with ethyl acetate. After evaporating off the solvents, the oil obtained is dissolved in 20 ml of dimethylformamide, and 3.96 g of pinacoldiborane are added, along with 489 mg of diphenylphosphinoferrocenepalladium dichloride and 3.53 g of potassium acetate. The mixture is stirred for 3 hours at 90°C. The reaction is hydrolysed and then extracted with ethyl acetate. The solvents are evaporated off and the residue obtained is chromatographed on silica gel (eluent = 7/3 heptane/ethyl acetate). 1.62 g of 3-ethoxy-4-(4,4,5,5-tetramethyl[1,3,2]dioxaborolan-2-yl)benzaldehyde are obtained. Yield = 49%.

### b. tert-Butyl 6-(2-ethoxy-4-formylphenyl)pyrid-2-yl]methylcarbamate

1.62 g of 3-ethoxy-4-(4,4,5,5-tetramethyl[1,3,2]dioxaborolan-2-yl)benzaldehyde are dissolved in 15 mL of dimethylformamide, and 3 mL of 2M potassium phosphate solution and 2.52 g of tert-butyl (6-bromopyrid-2-yl)methylcarbamate are added, along with 64 mg of palladium acetate and 200 mg of dicyclohexylbiphenylphosphine. The mixture is stirred for 3 hours at 90°C. The reaction is hydrolysed and then extracted with ethyl acetate. The solvents are evaporated off and the residue obtained is chromatographed on silica gel (eluent = 7/3 heptane/ethyl acetate). 1.1 g of tert-butyl 6-(2-ethoxy-4-formylphenyl)pyrid-2-yl]methylcarbamate are obtained. Yield = 53%.

### c. Methyl (E)-3-{4-[6-(tert-butoxycarbonylmethylamino)-pyrid-2-yl]-3-ethoxyphenyl}acrylate

1.1 g of tert-butyl 6-(2-ethoxy-4-formylphenyl)pyrid-2-yl]methylcarbamate are dissolved in 10 ml of toluene and 1.55 g of methyl triphenylphosphoranylideneacetate are then added. The reaction mixture is stirred for 1 hour at 90°C. After evaporating off the solvent, the residual oil obtained is chromatographed on silica gel (8/2 heptane/ethyl acetate). 1.0 g of methyl (E)-3-{4-[6-(tert-butoxycarbonylmethylamino)pyrid-2-yl]-3-ethoxyphenyl}acrylate is obtained. Yield = 78%.

### d. Methyl 3-{4-[6-(tert-butoxycarbonylmethylamino)-pyrid-2-yl]-3-ethoxyphenyl}propionate

A solution of 1.0 g of methyl(E)-3-{4-[6-(tert-butoxycarbonylmethylamino)pyrid-2-yl]-3-ethoxyphenyl}acrylate in 10 mL of methanol is stirred for 3 hours at room temperature in the presence of 200 mg of 10% palladium-on-charcoal under a hydrogen atmosphere. After filtering off the palladium and evaporating off the solvents, 1.0 g of methyl 3-{4-[6-(tert-butoxycarbonylmethylamino)pyrid-2-yl]-3-ethoxyphenyl}propionate is obtained. Yield = 100%.

### e. Methyl 3-[3-ethoxy-4-(6-methylaminopyrid-2-yl)phenyl]propionate

1.0 g of methyl 3-{4-[6-(tert-butoxycarbonylmethylamino)pyrid-2-yl]-3-ethoxyphenyl}propionate is dissolved in 10 mL of dichloromethane and 4 mL of trifluoroacetic acid are then added. The mixture is stirred for 48 hours at room temperature. After hydrolysis of the reaction with sodium hydrogen carbonate solution and then extraction with ethyl acetate, the solvents are evaporated off. 615 mg of methyl 3-[3-ethoxy-4-(6-methylaminopyrid-2-yl)phenyl]propionate are obtained. Yield = 81%.

### f. Methyl 3-{3-Ethoxy-4-[6-(3-heptyl-1-methylureido)pyrid-2-yl]phenyl}propionate

615 mg of methyl 3-[3-ethoxy-4-(6-methylaminopyrid-2-yl)phenyl]propionate are mixed with 1.0 mL of heptyl isocyanate. After microwave irradiation for one hour (T = 100°C), the mixture is chromatographed on silica gel (9/1 heptane/ethyl acetate). 735 mg of methyl 3-{3-ethoxy-4-[6-(3-heptyl-1-methylureido)pyrid-2-yl]phenyl}propionate are obtained. Yield = 82%.

### g. 2-[4-(2-Carboxyethyl)-2-ethoxyohenyl]-6-(3-heptyl-1-methylureido)pyridinium hydrochloride

500 mg of sodium hydroxide are added to a solution of 735 mg of methyl 3-{3-ethoxy-4-[6-(3-heptyl-1-methylureido)pyrid-2-yl]phenyl}propionate dissolved in 15 ml of an 8/2 tetrahydrofuran/methanol mixture. After stirring for 4 hours at room temperature, the reaction is stopped with water and acetic acid and then extracted with ethyl acetate. The solvents are evaporated off. The residual oil is chromatographed on silica gel (7/3 heptane/ethyl acetate). 5 mL of a solution of hydrogen chloride in ethyl acetate are added to the chromatographed product and the hydrochloride is precipitated by addition of isopropyl ether. After filtration, 441 mg of 2-[4-(2-carboxyethyl)-2-ethoxyphenyl]-6-(3-heptyl-1-methylureido)pyridinium hydrochloride are obtained.
Yield = 57%.
**¹H NMR** (DMSO, 400 MHz): 0.85 (m, 3H); 1.18 (m, 8H); 1.37 (m, 3H); 1.48 (m, 2H); 2.64 (m, 2H); 2.92 (m, 2H); 3.25 (s, 3H); 4.20 (m, 2H); 6.98 (d, J = 8 Hz, 1H); 7.12 (s, 1H); 7.25 (d, J = 8 Hz, 1H); 7.58 (d, J = 8 Hz, 2H); 7.99 (m, 1H); 9.70 (s, 1H).

### EXAMPLE 14: 3-(3-Butoxy-4-{5-[(methyloctanoylamino)-methyl]thiophen-3-yl}phenyl)propanoic acid

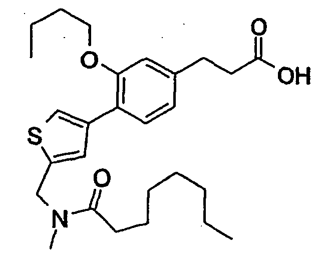

### a. (4-Bromothiophen-2-ylmethyl)methylamine

10.6 g of 4-bromothiophene-2-carbaldehyde are dissolved in 100 mL of acetonitrile, and 11.8 g of methylamine hydrochloride and 6.28 g of sodium cyanoborohydride are added. The reaction mixture is stirred overnight at room temperature. The reaction is stopped with 1M sodium hydroxide solution. After evaporating off the solvents, the residue is chromatographed on silica gel (7/3 heptane/ethyl acetate). 8.19 g of (4-bromothiophen-2-ylmethyl)methylamine are obtained. Yield = 79%.

### b. tert-Butyl (4-bromothiophen-2-ylmethyl)methylcarbamate

8.18 g of (4-bromothiophen-2-ylmethyl)methylamine are dissolved in 80 ml of dichloromethane in the presence of 6.65 ml of triethylamine. At 0°C, a solution of 9.53 g of di-tert-butyl dicarbonate in 10 ml of dichloromethane is added slowly. The reaction mixture is stirred for 2 hours at room temperature. The reaction is stopped with water and is then extracted with ethyl acetate. After evaporating off the solvents, the residue is chromatographed on silica gel (7/3 heptane/ethyl acetate). 5.93 g of tert-butyl (4-bromothiophen-2-ylmethyl)methylcarbamate are obtained. Yield = 49%.

### c. tert-Butyl methyl-[4-(4,4,5,5-tetramethyl[1,3,2]-dioxaborolan-2-yl)thiophen-2-ylmethyl]carbamate

5.92 g of tert-butyl (4-bromothiophen-2-ylmethyl)methylcarbamate are dissolved in 70 ml of dimethylformamide, and 5.89 g of pinacoldiborane are added along with 776 mg of diphenylphosphinoferrocenepalladium dichloride and 5.59 g of potassium acetate. The mixture is stirred for 3 hours at 90°C. The reaction is stopped with water and then extracted with ethyl acetate. The solvents are evaporated off. The residue is chromatographed on silica (eluent = 7/3 heptane/ethyl acetate). 4.57 g of tert-butyl methyl-[4-(4,4,5,5-tetramethyl[1,3,2]dioxaborolan-2-yl)thiophen-2-ylmethyl]carbamate are obtained. Yield = 63%.

### d. Methyl (E)-3-(3-butoxy-4-{5-[(tert-butoxycarbonyl-methylamino)methyl]thiophen-3-yl}phenyl)acrylate

2.50 g of tert-butyl methyl-[4-(4,4,5,5-tetramethyl[1,3,2]dioxaborolan-2-yl)thiophen-2-ylmethyl]carbamate are dissolved in 27 ml of dimethylformamide, and 5.5 mL of 2M potassium phosphate solution and 1.96 g of methyl (E)-3-(3-butoxy-4-iodophenyl)acrylate *(prepared according to the procedure of Example 11, step f),* are added, along with 12 mg of palladium acetate and 38 mg of dicyclohexylbiphenylphosphine. The mixture is stirred for 1 hour at 90°C. The reaction is stopped with water and then extracted with ethyl acetate. The solvents are evaporated off. The residue is chromatographed on silica (eluent = 7/3 heptane/ethyl acetate). 1.98 g of methyl (E)-3-(3-butoxy-4-{5-[(tert-butoxycarbonyl-methylamino)methyl]thiophen-3-yl}phenyl)acrylate are obtained. Yield = 79%.

### e. Methyl (E)-3-(3-butoxy-4-{5-[(tert-butoxycarbonyl-methylamino)methyl]thiophen-3-yl}phenyl)propionate

1.98 g of methyl (E)-3-(3-butoxy-4-{5-[(tert-butoxycarbonylmethylamino)methyl]thiophen-3-yl}phenyl)acrylate are dissolved in 20 ml of methanol and stirred for 12 hours at room temperature in the presence of 1.0 g of palladium-on-charcoal under a hydrogen atmosphere. After filtering off the palladium and evaporating off the solvents, 1.37 g of methyl (E)-3-(3-butoxy-4-{5-[(tert-butoxycarbonyl-methylamino)methyl]thiophen-3-yl}phenyl)propionate are obtained. Yield = 69%.

### f. Methyl 3-[3-butoxy-4-(5-methylaminomethylthiophen-3-yl)phenyl]propionate

1.36 g of methyl (E)-3-(3-butoxy-4-{5-[(tert-butoxycarbonylmethylamino)methyl]thiophen-3-yl}phenyl)propionate are dissolved in 15 ml of dichloromethane and 2.2 ml of trifluoroacetic acid are then added. The mixture is stirred for 2 hours at room temperature. After hydrolysing the reaction with sodium hydrogen carbonate solution and then extracting with ethyl acetate and evaporating off the solvents, 1.15 g of methyl 3-[3-butoxy-4-(5-methylaminomethylthiophen-3-yl)phenyl]propionate are obtained. Yield = 100%.

### g. Methyl 3-(3-butoxy-4-{5-[(methyloctanoylamino)-methyl]thiophen-3-yl}phenyl)propionate

1.07 g of methyl 3-[3-butoxy-4-(5-methylaminomethylthiophen-3-yl)phenyl]propionate are dissolved in 20 mL of dichloromethane, and 1.25 mL of triethylamine are added, along with 36 mg of dimethylaminopyridine. 318 mg of octanoyl chloride are added. The reaction mixture is stirred for 12 hours at room temperature. After hydrolysing the reaction with sodium hydrogen carbonate solution and then extracting with ethyl acetate and evaporating off the solvents, a crude product is obtained, which is chromatographed on silica gel (eluent = 7/3 heptane/ethyl acetate). 432 mg of methyl 3-(3-butoxy-4-{5-[(methyloctanoylamino)-methyl]thiophen-3-yl}phenyl)propionate are obtained. Yield = 81%.

### h. 3-(3-Butoxy-4-{5-[(methyloctanoylamino)methyl]thio-phen-3-yl}phenyl)propanoic acid

350 mg of sodium hydroxide are added to a solution of 427 mg of methyl 3-(3-butoxy-4-{5-[(methyloctanoylamino)methyl]thiophen-3-yl}phenyl)propionate dissolved in 10 mL of methanol. After stirring for 2 hours at 40°C, the reaction is hydrolysed with hydrochloric acid solution and then extracted with ethyl acetate. The solvents are evaporated off. The residual oil is crystallized from a heptane/ethyl ether mixture.

318 mg of 3-(3-butoxy-4-{5-[(methyloctanoylamino)methyl]thiophen-3-yl}phenyl)propanoic acid are obtained. Yield = 77%.
**¹H NMR** (CDCl₃, 400 MHz): 0.85 (m, 3H); 1.28 (m, 8H); 1.49 (m, 2H); 1.60 (m, 2H); 1.79 (m, 2H); 2.33&2.48 (2m(rotamers), 2H); 2.65 (m, 2H); 2.95 (m, 2H); 2.99 (s, 3H); 4.00 (m, 2H); 4.65&4.72 (2s(rotamers), 2H); 6.80 (m, 2H); 7.27 (m, 2H); 7.37 (m, 1H); 7.49 (m, 1H).

### EXAMPLE 15: 3-[3-Butoxy-4-(5-{[(4-methoxybenzoyl)-methylamino]methyl}thiophen-3-yl)phenyl]propanoic acid

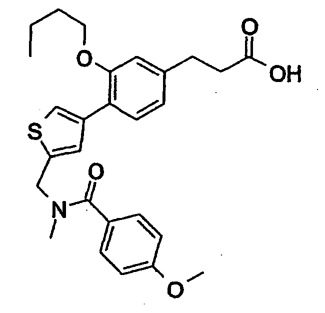

### a. Methyl 3-[3-butoxy-4-(5-{[(4-methoxybenzoyl)-methylamino]methyl}thiophen-3-yl)phenyl]propionate

1.07 g of methyl 3-[3-butoxy-4-(5-methylaminomethylthiophen-3-yl)phenyl]propionate *(prepared according to the procedure of Example 14, steps a to f)* are dissolved in 20 ml of dichloromethane, and 1.25 ml of triethylamine are added, along with 36 mg of dimethylaminopyridine. 270 mg of 4-methoxybenzoyl chloride are added. The reaction mixture is stirred for 12 hours at room temperature. The reaction is stopped with sodium hydrogen carbonate solution and then extracted with ethyl acetate. The solvents are evaporated off. The residue is chromatographed on silica (eluent = 7/3 heptane/ethyl acetate).

388 mg of methyl 3-[3-butoxy-4-(5-{[(4-methoxybenzoyl)methylamino]methyl}thiophen-3-yl)phenyl]propionate are obtained. Yield = 71%.

### b. 3-[3-Butoxy-4-(5-{[(4-methoxybenzoyl)methylamino]-methy}thiophen-3-yl)phenyl]prapanoic acid

308 mg of sodium hydroxide are added to a solution of 382 mg of methyl 3-[3-butoxy-4-(5-{[(4-methoxybenzoyl)methylamino]methyl}thiophen-3-yl)phenyl]propionate dissolved in 10 mL of methanol. After stirring for 2 hours at 40°C, the reaction is hydrolysed with hydrochloric acid solution and then extracted with ethyl acetate. The solvents are evaporated off. The residual oil is crystallized from a heptane/ethyl ether mixture.

300 mg of 3-[3-butoxy-4-(5-{[(4-methoxybenzoyl)methylamino]methyl}thiophen-3-yl)phenyl]propanoic acid are obtained. Yield = 81%.
**¹H NMR** (CDCl₃, 400 MHz): 0.95 (m, 3H); 1.47 (m, 2H); 1.79 (m, 2H); 2.70 (m, 2H); 2.96 (m, 2H); 3.09 (s, 3H); 3.82 (s, 3H); 4.00 (m, 2H); 4.79 (s, 2H); 6.81 (m, 2H); 6.88 (m, 3H); 7.24-7.52 (m, 4H).

### EXAMPLE 16: 3-[3-Butoxy-4-(5-{[(3-methoxybenzoyl)-methylamino]methyl}thiophen-3-yl)phenyl]propanoic acid

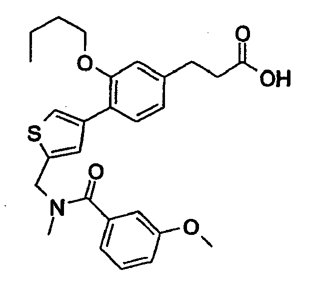

### a. Methyl 3-[3-butoxy-4-(5-{[(3-methoxybenzoyl)methyl-amino]methyl}thiophen-3-yl)phenyl]propionate

1.07 g of methyl 3-[3-butoxy-4-(5-methylaminomethylthiophen-3-yl)phenyl]propionate *(prepared according to the procedure of Example 14, steps a to f)* are dissolved in 20 mL of dichloromethane, and 1.25 ml of triethylamine are added, along with 36 mg of dimethylaminopyridine. 270 mg of 3-methoxybenzoyl chloride are added. The reaction mixture is stirred for 12 hours at room temperature. The reaction is stopped with sodium hydrogen carbonate solution and then extracted with ethyl acetate. The solvents are evaporated off. The residue is chromatographed on silica (eluent = 7/3 heptane/ethyl acetate).

380 mg of methyl 3-[3-butoxy-4-(5-{[(3-methoxybenzoyl)methylamino]methyl}thiophen-3-yl)phenyl]propionate are obtained. Yield = 70%.

### b. 3-[3-Butoxy-4-(5-{[(3-methoxybenzoyl)methylamino]-methyl}thiophen-3-yl)phenyl]propanoic acid

302 mg of sodium hydroxide are added to a solution of 374 mg of methyl 3-[3-butoxy-4-(5-{[(3-methoxybenzoyl)methylamino]methyl}thiophen-3-yl)phenyl]propionate dissolved in 10 mL of methanol. After stirring for 2 hours at 40°C, the reaction is hydrolysed with hydrochloric acid solution and then extracted with ethyl acetate. The solvents are evaporated off. The residual oil is crystallized from a heptane/ethyl ether mixture.

307 mg of 3-[3-butoxy-4-(5-{[(3-methoxybenzoyl)methylamino]methyl}thiophen-3-yl)phenyl]propanoic acid are obtained. Yield = 84%.
**¹H NMR** (CDCl₃, 400 MHz): 0.95 (m, 3H); 1.47 (m, 2H); 1.79 (m, 2H); 2.69 (m, 2H); 2.96 (m, 2H); 3.10 (s, 3H); 3.81 (s, 3H); 4.00 (m, 2H); 4.62&4.88 (2s(rotamers), 2H); 6.81 (m, 2H); 6.96 (m, 3H); 7.26-7.52 (m, 4H).

### EXAMPLE 17 - CROSSOVER-CURVE PPAR TRANSACTIVATION TESTS

The activation of PPAR receptors with an agonist (activator) in HeLN cells leads to the expression of a reporter gene, luciferase, which, in the presence of a substrate, generates light. The modulation of the PPAR receptors is measured by quantifying the luminescence produced after incubating the cells in the presence of a reference agonist. The ligands displace the agonist from its site. The measurement of the activity is performed by quantifying the light produced. This measurement makes it possible to determine the modulatory activity of the compounds according to the invention by determining the constant that represents the affinity of the molecule for the PPAR receptor. Since this value can fluctuate depending on the basal activity and the expression of the receptor, it is referred to as Kd apparent (KdApp in nM).

To determine this constant, "crossover curves" of the test product against a reference agonist are performed in a 96-well plate: 10 concentrations of the test product plus a concentration 0 are arranged in a line, and 7 concentrations of the agonist plus a concentration 0 are arranged in a column. This represents 88 measurement points for 1 product and 1 receptor. The remaining 8 wells are used for repeatability controls.

In each well, the cells are in contact with a concentration of the test product and a concentration of the reference agonist, 2-(4-{2-[3-(2,4-difluorophenyl)-1-heptylureido]ethyl}phenylsulfanyl)-2-methyl-propionic acid for PPARα, {2-methyl-4-[4-methyl-2-(4-trifluoromethylphenyl)thiazol-5-ylmethylsulfanyl]-phenoxy}acetic acid for PPARδ and 5-{4-[2-(methylpyrid-2-ylamino)ethoxy]benzyl}thiazolidine-2,4-dione for PPARγ. Measurements are also taken for total agonist controls with the same products.

The HeLN cell lines used are stable transfectants containing the plasmids ERE-βGlob-Luc-SV-Neo (reporter gene) and PPAR (α, δ, γ) Gal-hPPAR. These cells are inoculated into 96-well plates at a rate of 10 000 cells per well in 100 µl of DMEM medium without phenol red and supplemented with 10% of defatted calf serum. The plates are then incubated at 37°C and 7% CO₂ for 16 hours.

The various dilutions of the test products and of the reference ligand are added at a rate of 5 µl per well. The plates are then incubated for 18 hours at 37°C and 7% CO₂ The culture medium is removed by turning over and 100 µl of a 1:1 PBS/luciferine mixture are added to each well. After 5 minutes, the plates are read by the luminescence detector.

These crossed curves make it possible to determine the AC50 values (concentration at which 50% activation is observed) of the reference ligand at various concentrations of test product. These AC50 values are used to calculate the Schild regression by plotting a straight line corresponding to the Schild equation ("quantitation in receptor pharmacology" Terry P. Kenakin, Receptors and Channels, 2001, 7, 371-385) which allows the Kd app values (in nM) to be obtained.

### Transactivation results:

| Compounds | PPARα Kd app | PPARδ Kd app | PPARγ Kd app |
|---|---|---|---|
| | (nM) | (in nM) | (in nM) |
| Reference 1: 2-(4-{2-[3-(2,4-difluorophenyl)-1-heptylureido]-ethyl}phenylsulfanyl)-2-methyl-propanoic acid | 200 | n.a. | n.a. |
| Reference 2: {2-methyl-4-[4-methyl-2-(4-trifluoromethyl-phenyl)thiazol-5-ylmethylsulf-anyl]phenoxy}acetic acid | n.a. | 10 | n.a. |
| Reference 3: 5-{4-[2-(methylpyrid-2-ylamino)ethoxy]benzyl}thiazoli-dine-2,4-dione | n.a. | n.a. | 30 |
| Compound of Example 1 | n.a. | n.a. | 1000 |
| Compound of Example 6 | n.a. | 4000 | 250 |
| Compound of Example 2 | n.a. | n.a. | 500 |
| Compound of Example 7 | n.a. | n.a. | 60 |
| Compound of Example 8 | n.a. | n.a. | 1000 |
| Compound of Example 11 | 2000 | n.a. | 120 |
| Compound of Example 12 | 4000 | n.a. | 15 |
| Compound of Example 13 | 4000 | 8000 | 120 |
| Compound of Example 14 | n.a. | n.a. | 2 |
| Compound of Example 15 | n.a. | n.a. | 30 |
| Compound of Example 16 | n.a. | n.a. | 30 |

| | | | |
|---|---|---|---|
| n.a. means not active | | | |

These results show the affinity of the compounds for PPARγ and more particularly the specificity of the affinity of the compounds of the invention for the PPARγ subtype, compared with the affinity of the compounds for the PPARα subtype or for the PPARδ subtype.

### EXAMPLE 18 - COMPOSITIONS

Various concrete formulations based on the compounds according to the invention are illustrated in this example.

### A- ORAL ROUTE

| (a) 0.2 g tablet | | |
|---|---|---|
| - Compound of Example 1 | | 0.001 g |
| - Starch | | 0.114 g |
| - Dicalcium phosphate | | 0.020 g |
| - Silica | | 0.020 g |
| - Lactose | | 0.030 g |
| - Talc | | 0.010 g |
| - Magnesium stearate | | 0.005 g |

| (b) Drinkable suspension in 5 ml ampules | | |
|---|---|---|
| - Compound of Example 5 | | 0.001 g |
| - Glycerol | | 0.500 g |
| - 70% Sorbitol | | 0.500 g |
| - Sodium saccharinate | | 0.010 g |
| - Methyl para-hydroxybenzoate | | 0.040 g |
| - Flavouring | | qs |
| - Purified water | | qs 5 ml |

| (c) 0.8 g tablet | | |
|---|---|---|
| - Compound of Example 2 | | 0.500 g |
| - Pregelatinized starch | | 0.100 g |
| - Microcrystalline cellulose | | 0.115 g |

| | | |
|---|---|---|
| - Lactose | | 0.075 g |
| - Magnesium stearate | | 0.010 g |

| (d) Drinkable suspension in 10 ml ampules | | |
|---|---|---|
| - Compound of Example 4 | | 0.200 g |
| - Glycerol | | 1.000 g |
| - 70% Sorbitol | | 1.000 g |
| - Sodium saccharinate | | 0.010 g |
| - Methyl para-hydroxybenzoate | | 0.080 g |
| - Flavouring | | qs |
| - Purified water | qs | 10 ml |

### B- TOPICAL ROUTE

| (a) Ointment | | |
|---|---|---|
| - Compound of Example 6 | | 0.020 g |
| - Isopropyl myristate | | 81.700 g |
| - Liquid petroleum jelly fluid | | 9.100 g |
| - Silica ("Aerosil 200" sold by Degussa) | | 9.180 g |

| (b) Ointment | | |
|---|---|---|
| - Compound of Example 2 | | 0.150 g |
| - Compound of Example 4 | | 0.150 g |
| - White petroleum jelly codex | qs | 100 g |

| (c) Nonionic water-in-oil cream | | |
|---|---|---|
| - Compound of Example 1 | | 0.100 g |
| - Mixture of emulsifying lanolin alcohols, waxes and oils ("Anhydrous Eucerin" sold by BDF) | | 39.900 g |
| - Methyl para-hydroxybenzoate | | 0.075 g |
| - Propyl para-hydroxybenzoate | | 0.075 g |
| - Sterile demineralized water | qs | 100 g |

| (d) Lotion | | |
|---|---|---|
| - Compound of Example 3 | | 0.100 g |
| - Polyethylene glycol (PEG 400) | | 69.900 g |
| - 95% Ethanol | | 30.000 g |

| (e) Hydrophobic ointment | | |
|---|---|---|
| - Compound of Example 5 | | 0.300 g |
| - Isopropyl myristate | | 36.400 g |
| - Silicone oil ("Rhodorsil 47 V 300" sold by Rhone-Poulenc) | | 36.400 g |
| - Beeswax | | 13.600 g |
| - Silicone oil ("Abil 300,000 cst" sold by Goldschmidt) | qs | 100 g |

| (f) Nonionic oil-in-water cream | | |
|---|---|---|
| - Compound of Example 2 | | 1.000 g |
| - Cetyl alcohol | | 4.000 g |
| - Glyceryl monostearate | | 2.500 g |
| - PEG-50 stearate | | 2.500 g |
| - Karite butter | | 9.200 g |
| - Propylene glycol | | 2.000 g |
| - Methyl para-hydroxybenzoate | | 0.075 g |
| - Propyl para-hydroxybenzoate | | 0.075 g |
| - Sterile demineralized water | qs | 100 g |

## Claims

1. Compounds **characterized in that** they correspond to formula (I) below: 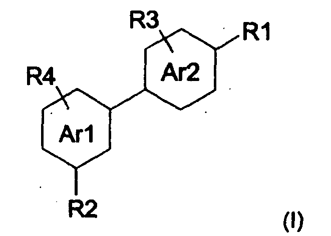 in which:
- **R1** represents a radical of formula (a) or (b) below: 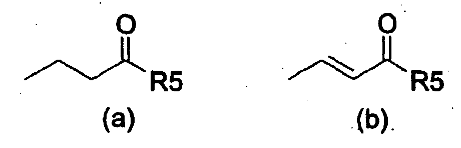 R5 having the meanings given below,
- **R2** represents a radical of formula (CH₂)ₘ-NR₆-CQ-(NH)ₙR₇; Q, R6, R7, m and n having the meanings given below,
- **R3** and **R4,** which may be identical or different, represent a hydrogen atom, a halogen atom, a linear or cyclic alkyl radical of 1 to 12 carbon atoms that may be interrupted with oxygen, fluorine or nitrogen atoms, a hydroxyl radical, an alkoxy radical containing from 1 to 7 carbon atoms, a polyether radical, an aralkyl radical or an aryloxy radical;
- **R5** represents a hydroxyl radical, a radical OR8 or a hydroxylamine radical;
R8 being defined below,
- **R6** represents a lower alkyl radical containing from 1 to 4 carbon atoms;
- **R7** represents an alkyl radical containing from 1 to 12 carbon atoms, an aryl radical, an aralkyl radical, a heteroaryl radical or a heterocyclic radical;
- **R8** represents an alkyl, aryl or aralkyl radical;
- **m** and **n** may take the values 0 or 1;
- **Q** represents an oxygen or sulfur atom;
- **Ar1** and **Ar2** may be identical or different and represent an aromatic radical of formula: 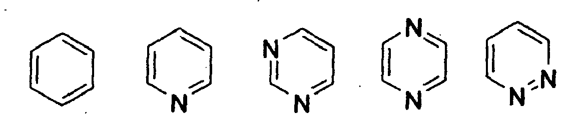 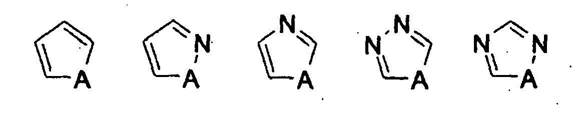
which, when it is an aryl radical, may be mono- or disubstituted with a halogen atom, a CF₃ radical, an alkyl radical containing from 1 to 12 carbon atoms, an alkoxy radical containing from 1 to 7 carbon atoms, a nitro function, a polyether radical, an aryl radical, a benzoyl radical, an alkyl ester group, a carboxylic acid, a hydroxyl radical optionally protected with an acetyl or benzoyl group or an amino function optionally protected with an acetyl or benzoyl group or optionally substituted with at least one alkyl containing from 1. to 12 carbon atoms;
and which, when it is an heteroaryl radical,is optionally substituted with at least one halogen, an alkyl containing from 1 to 12 carbon atoms, an alkoxy containing from 1 to 7 carbon atoms, an aryl radical, a nitro function, a polyether radical, a heteroaryl radical, a benzoyl radical, an alkyl ester group, a carboxylic acid, a hydroxyl optionally protected with an acetyl or benzoyl group or an amino function optionally protected with an acetyl or benzoyl group or optionally substituted with at least one alkyl containing from 1 to 12 carbon atoms
- **A** represents a sulfur or oxygen atom or a radical N-R9;
- **R9** represents a hydrogen atom or an alkyl radical containing from 1 to 12 carbon atoms;
it being understood that when Ar1 or Ar2 is an aryl radical, then Ar2 or Ar1 is necessarily a heteroaryl radical,
and the optical and geometrical isomers of the said compounds of formula (I).

2. Compounds according to Claim 1, **characterized in that** they are in the form of salts of an alkali metal or of an alkaline-earth metal or salts of an organic amine.

3. Compounds according to either of Claims 1 and 2, **characterized in that** the halogen atom is chosen from fluorine, chlorine and bromine atoms.

4. Compounds according to either of Claims 1 and 2, **characterized in that** the alkyl radical containing from 1 to 12 carbon atoms is chosen from methyl, ethyl, isopropyl, butyl, isobutyl, tert-butyl, hexyl, heptyl, octyl, decyl, cyclopentyl, cyclohexyl and methylenecyclopropyl radicals.

5. Compounds according to either of Claims 1 and 2, **characterized in that** the lower alkyl radical containing from 1 to 4 carbon atoms is chosen from methyl, ethyl, n-propyl, i-propyl, c-propyl, methylcyclopropyl, n-butyl, i-butyl and t-butyl radicals.

6. Compounds according to either of Claims 1 and 2, **characterized in that** the alkoxy radical containing from 1 to 7 carbon atoms is chosen from methoxy, ethoxy, isopropyloxy, methylcyclopropyloxy, isobutoxy, tert-butoxy, pentyloxy, isopentyloxy, hexyloxy, heptyloxy, benzyloxy, aryloxy or phenoxy radicals, which may be optionally substituted with an alkyl radical containing from 1 to 12 carbon atoms or an alkoxy radical containing from 1 to 5 carbon atoms.

7. Compounds according to either of Claims 1 and 2, **characterized in that** the polyether radical is chosen from methoxymethoxy, ethoxymethoxy and methoxyethoxymethoxy radicals.

8. Compounds according to either of Claims 1 and 2, **characterized in that** the aralkyl radical is chosen from benzyl, phenethyl or 2-naphthylmethyl radicals, which may be mono- or disubstituted with a halogen atom, a CF₃ radical, an, alkyl radical containing from 1 to 12 carbon atoms, an alkoxy radical containing from 1 to 7 carbon atoms, a nitro function, a polyether radical, an aryl radical, a benzoyl radical, an alkyl ester group, a carboxylic acid, a hydroxyl radical optionally protected with an acetyl or benzoyl, group or an amino function optionally protected with an acetyl or benzoyl group or optionally substituted with at least one alkyl containing from 1 to 12 carbon atoms.

9. Compounds according to either of Claims 1 and 2, **characterized in that** the aryl radical is chosen from phenyl, biphenyl, cinnamyl or naphthyl radicals, which may be mono- or disubstituted with a halogen atom, a CF₃ radical, an alkyl radical containing from 1 to 12 carbon atoms, an alkoxy radical containing from 1 to 7 carbon atoms, a nitro function, a polyether radical, an aryl radical, a benzoyl radical, an alkyl ester group, a carboxylic acid, a hydroxyl radical optionally protected with an acetyl or benzoyl group or an amino function optionally protected with an acetyl or benzoyl group or optionally substituted with at least one alkyl containing from 1 to 12 carbon atoms.

10. Compounds according to either of Claims 1 and 2, **characterized in that** the heteroaryl radical is chosen from the group consisting of a pyridyl, furyl, thienyl, isoxazolyl, oxadiazolyl, oxazolyl, isothiazolyl, quinazolinyl, benzothiadiazolyl, benzimidazolyl, quinoxalyl, indolyl or benzofuryl radical, optionally substituted with at least one halogen, an alkyl containing from 1 to 12 carbon atoms, an alkoxy containing from 1 to 7 carbon atoms, an aryl radical, a nitro function, a polyether radical, a heteroaryl radical, a benzoyl radical, an alkyl ester group, a carboxylic acid, a hydroxyl optionally protected with an acetyl or benzoyl group or an amino function optionally protected with an acetyl or benzoyl group or optionally substituted with at least one alkyl containing from 1 to 12 carbon atoms.

11. Compounds according to either of Claims 1 and 2, **characterized in that** the heterocyclic radical is chosen from a morpholino, piperidino, piperazino, 2-oxo-1-piperidyl or 2-oxo-1-pyrrolidinyl radical, optionally substituted with at least one alkyl containing from 1 to 12 carbon atoms, an alkoxy containing from 1 to 7 carbon atoms, an aryl radical, a nitro function, a polyether radical, a heteroaryl radical, a benzoyl radical, an alkyl ester group, a carboxylic acid, a hydroxyl optionally protected with an acetyl or benzoyl group or an amino function optionally protected with an acetyl or benzoyl group or optionally substituted with at least one alkyl, containing from 1 to 12 carbon atoms.

12. Compounds according to Claim 1, **characterized in that** they are taken, alone or as mixtures, from the group consisting of:
1. 3-(5-{3-[(Methyloctanoylamino)methyl]phenyl}thiophen-2-yl)acrylic acid
2. 3-(5-{3-[(Methyloctanoylamino)methyl]phenyl}-thiophen-2-yl)propanoic acid
3. 3-(5-{3-[(Methyloctanoylamino)methyl]phenyl}furan-2-yl)acrylic acid
4. 3-(4-{5-[(Methyloctanoylamino)methyl]thiophen-3-yl}phenyl)acrylic acid
5. 3-(4-{5-[(Methyloctanoylamino)methyl]thiophen-3-yl}phenyl)propanoic acid
6. 3-{4-[6-(3-Heptyl-1-methylureido)pyrid-2-yl]phenyl}propanoic acid
7. 3-{6-[3-(3-Heptyl-1-methylureido)phenyl]pyrid-3-yl}propanoic acid
8. 2-[4-(2-Carboxyethyl)phenyl]-4-(3-heptyl-1-methyl-ureido)pyridinium acetate
9. 3-{5-[3-(3-Pentyl-1-methylureido)phenyl]pyrimidin-2-yl}acrylic acid
10. 3-{5-[3-(3-Pentyl-1-methylureido)phenyl]pyrimidin-2-yl}propanoic acid
11. 2-[2-Butoxy-4-(2-carboxyethyl)phenyl]-6-(1-methyl-3-pentylureido)pyridinium hydrochloride
12. 2-[2-Butoxy-4-(2-carboxyethyl)phenyl]-6-(1-methyl-3-heptylureido)pyridinium hydrochloride
13. 2-[4-(2-Carboxyethyl)-2-ethoxyphenyl]-6-(3-heptyl-1-methylureido)pyridinium hydrochloride
14. 3-(3-Butoxy-4-{5-[(methyloctanoylamino)methyl]-thiophen-3-yl}phenyl)propanoic acid
15. 3-(3-Butoxy-4-(5-{[(4-methoxybenzoyl)methylamino]-methyl}thiophen-3-yl)phenyl]propanoic acid
16. 3-(3-Butoxy-4-(5-{[(3-methoxybenzoyl)methylamino]-methyl}thiophen-3-yl)phenyl]propanoic acid
17. 3-{5-[4-(3-Heptyl-1-methylureido)pyrid-2-yl]furan-2-yl}propanoic acid
18. 3-[5-[3-(3-Heptyl-1-methylureido)phenyl]-4-(2,2,2-trifluoroethoxy)-furan-2-yl]propanoic acid
19. 3-(5-{3-[3-(3,5-Dimethoxyphenyl)-1-ethylureido]-phenyl}-3-methylfuran-2-yl)propanoic acid
20. 3-(5-{6-[3-(4-Ethoxyphenyl)-1-ethylureido]pyrid-2-yl}furan-2-yl)propanoic acid
21. 3-(2-Methyl-4-{6-[1-methyl-3-(4-methylpentyl)ureido]pyrid-2-yl}phenyl)propanoic acid
22. Methyl 3-{4-[6-(1-ethyl-3-naphthalen-2-ylureido)-pyrid-2-yl]-2-fluorophenyl}propanoate
23. 3-(4-{6-[3-(4-Butoxyphenyl)-1-methylureido]pyrid-2-yl}phenyl)-N-hydroxypropionamide
24. 3-{3-Cyclopropylmethoxy-4-[6-(1-methyl-3-pentylureido)pyrid-2-yl]phenyl}propanoic acid
25. 3-{4-[6-(1-Ethyl-3-phenylthioureido)pyrid-2-yl]-3-propoxyphenyl}propanoic acid.
26. 3-{3-Ethoxy-4-[6-(3-heptyl-1-methylureido)pyrid-2-yl]phenyl}propanoic acid
27. 3-{4-[6-(3-Hexyl-1-methylureido)pyrid-2-yl]-3-isopropoxyphenyl}propanoic acid
28. 3-[4-[6-(3-Heptyl-1-methylureido)pyrid-2-yl]-3-(4,4,4-trifluorobutoxy)phenyl]propanoic acid
29. 3-{3-(2-Dimethylaminoethoxy)-4-[6-(1-methyl-3-pentylureido)pyrid-2-yl]phenyl}propanoic acid
30. 3-{3-(3-Hydroxypropoxy)-4-[6-(1-methyl-3-pentylureido)pyrid-2-yl]phenyl}propanoic acid
31. 3-{4-[6-(1-Ethyl-3-phenylthioureido)pyrid-2-yl]-3-fluorophenyl}acrylic acid
32. 3-{4-[6-(3-Hexyl-1-methylureido)pyrid-2-yl]-3-trifluoromethylphenyl}propanoic acid
33. 3-[6'-(1-Methyl-3-phenethylureido)-[2,2']bipyridinyl-5-yl]propanoic acid
34. 3-{2-[6-(3-Hexyl-1-methylureido)pyrid-2-yl]thiazol-4-yl}propanoic acid
35. Ethyl 3-{2-[6-(3-hexyl-1-methylureido)pyrid-2-yl]-thiazol-5-yl}propanoate
36. 3-(3-{6-[1-Methyl-3-(6-methylheptyl)ureidolpyrid-2-yl}isoxazol-5-yl)propanoic acid
37. 3-{4-[2-(3-Heptyl-1-methylureido)pyrimidin-4-yl]phenyl}propanoic acid
38. 3-{3-Cyclopropylmethoxy-4-[2-(3-heptyl-1-methylureido)pyrimidin-4-yl]phenyl}propanoic acid
39. 3-(4-{2-[1-Ethyl-3-(4-propoxyphenyl)ureido]-pyrimidin-4-yl}-3-fluorophenyl)propanoic acid
40. 3-{2-Fluoro-4-[2-(1-methyl-3-naphthalen-2-ylureido)pyrid-4-yl]phenyl}-N-hydroxypropionamide
41. 3-[2'-(3-Hexyl-1-methylthioureido)[2,4']-bipyridinyl-5-yl]propanoic acid
42. 3-{4-[2-(3-Hexyl-1-methylureido)pyrid-4-yl]-3-propoxyphenyl}propanoic acid
43. 3-(3-Benzyloxy-4-{4-[1-methyl-3-(5-methylhexyl)ureido]pyrid-2-yl}phenyl)propanoic acid
44. 3-{2-[4-(3-Hexyl-1-methylureido)pyrid-2-yl]thiazol-5-yl}acrylic acid
45. 3-{5-[3-(3-Hexyl-1-methylureido)phenyl]pyrid-2-yl}-acrylic acid
46. 3-{5-[5-(3-Heptyl-1-methylthioureido)thiophen-3-yl]pyrid-2-yl}acrylic acid
47. 3-{4-[2-(3-Heptyl-1-methylureido)thiazol-4-yl]-3-propoxyphenyl}propanoic acid
48. 3-(2-Fluoro-4-{5-[(heptanoylmethylamino)methyl]thiophen-3-yl}phenyl)propanoic acid
49. 3-(4-{5-[(Heptanoylmethylamino)methyl]thiophen-3-yl}-3-isobutoxyphenyl)acrylic acid
50. 3-(3-(2-Cyclopentylethoxy)-4-{5-[(methyloctanoylamino)methyl]thiophen-3-yl}phenyl)propanoic acid
51. Methyl 3-(3-isobutoxy-4-{5-[(methylnonanoylamino)-methyl]thiophen-3-yl}phenyl)propanoate
52. 3-{6-[5-({Ethyl-[2-(2-pentylphenyl)acetyl]amino}-methyl)thiophen-3-yl]pyrid-3-yl}propanoic acid
53. 3-(4-{4-[(Methylnonanoylamino)methyl]thiazol-2-yl}-3-propoxyphenyl)propanoic acid.
54. 3-(2-Chloro-4-{4-[(methylnonanoylamino)methyl]-thiophen-2-yl}phenyl)propanoic acid
55. 3-(2-Fluoro-4-{4-[(methylnonanoylamino)methyl]-thiophen-2-yl}phenyl)acrylic acid
56. 3-(4-{4-[(Heptanoylmethylamino)methyl]thiophen-2-yl}-1-methyl-1H-pyrrol-2-yl)propanoic acid
57. 3-(4-{4-[(Heptanoylmethylamino)methyl]thiophen-2-yl}furan-2-yl)propanoic acid
58. 3-{5'-[(Heptanoylmethylamino)methyl]-[3,3']bithiophenyl-5-yl}propanoic acid
59. Phenyl 3-{5'-[(heptanoylmethylamino)methyl]-3-propyl[2,3']bithiophenyl-5-yl}propanoate
60. 3-(5-{5-[(Heptanoylmethylamino)methyl]thiophen-3-yl}-4-propylfuran-2-yl)acrylic acid
61. 3-{3-Butoxy-4-[6-(3-heptyl-1-methylureido)pyrid-2-yl]phenyl}propanoic acid
62. 3-{3-Benzyloxy-4-[6-(3-heptyl-1-methylureido)pyrid-2-yl]phenyl}propanoic acid
63. 3-{3-Benzyloxy-4-[2-((3-heptyl-1-methylureido)pyrimidin-4-yl]phenyl}propanoic acid
64. 3-{3-Butyloxy-4-[2-(3-heptyl-1-methylureido)pyrimidin-4-yl]phenyl}propanoic acid
65. 3-{3-Butoxy-4-[4-(3-heptyl-1-methylureido)pyrid-2-yl]phenyl}propanoic acid
66. 3-{3-Benzyloxy-4-[4-(3-heptyl-1-methylureido)pyrid-2-yl]phenyl}propanoic acid
67. 3-{3-Benzyloxy-4-[5-(3-heptyl-1-methylureido)pyrid-3-yl]phenyl}propanoic acid
68. 3-{3-Butoxy-4-[5-(3-heptyl-1-methylureido)pyrid-3-yl]phenyl}propanoic acid
69. 3-(5-{3-[(Methyloctanoylamino)methyl]phenyl}-4-propylthiophen-2-yl)propanoic acid
70. 3-(3-Benzyloxy-4-{5-[(methyloctanoylamino)methyl]-thiophen-3-yl}phenyl}propanoic acid
71. 3-(4-Benzyl-5-{3-[(hexanoylmethylamino)methyl]-phenyl}thiophen-2-yl)propanoic acid
72. 3-{4-Cyclopropylmethyl-5-[3-(1-methyl-3-pentylureido)phenyl]thiophen-2-yl}propanoic acid
73. 3-{5-[3-(1-Methyl-3-pentylureido)-4-trifluoromethylphenyl]thiophen-2-yl}propanoic acid
74. 3-(5-{3-[3-(4-Butoxyphenyl)-1-ethylureido]phenyl}-thiophen-2-yl)propanoic acid
75. 3-{5-[3-(3-Heptyl-1-methylureido)-4-trifluoromethylphenyl]furan-2-yl}propanoic acid
76. 3-{2-Butoxy-4-[6-(1-methyl-3-pentylureido)pyrid-2-yl]phenyl}propanoic acid
77. 3-{2-(4-Methoxybenzyloxy)-4-[6-(1-methyl-3-pentyl-ureido)pyrid-2-yl]phenyl}propanoic acid
78. 3-{2-(3-Methoxybenzyloxy)-4-[6-(1-methyl-3-pentyl-ureido)pyrid-2-yl]phenyl}propanoic acid
79. 3-[2-Cyclopropylmethoxy-4-(6-{3-[2-(4-methoxyphenyl)-ethyl]-1-methylureido}pyrid-2-yl)phenyl]propanoic acid

13. Compounds according to Claim 1 or 2, **characterized in that** they have at least one of the following characteristics:
- R3 is an alkoxy radical of 1 to 7 carbon atoms;
- R5 corresponds to a hydroxyl radical;
- the sequence -(CH₂)ₘ-NR₆-CQ(NH)ₙR₇ has m = 0, n = 1;
- Q is an oxygen atom;
- R7 represents an alkyl radical of 1 to 8 carbon atoms;
- at least Ar1 or Ar2 is a group of pyridine type.

14. Compounds according to Claim 13, **characterized in that** they have all of the following characteristics:
- R3 is an alkoxy radical of 1 to 7 carbon atoms;
- R5 corresponds to a hydroxyl radical;
- the sequence -(CH₂)ₘ-NR₆-CQ(NH)ₙR₇ has m = 0, n = 1;
- Q is an oxygen atom;
- R7 represents an alkyl radical of 1 to 8 carbon atoms;
- at least Ar1 or Ar2 is a group of pyridine type.

15. Cosmetic composition, **characterized in that** it comprises, in a physiologically acceptable support, at least one of the compounds as defined in any one of Claims 1 to 14.

16. Composition according to Claim 15, **characterized in that** the concentration of compound(s) according to one of Claims 1 to 14 is between 0.0001% and 2% by weight relative to the total weight of the composition.

17. Cosmetic use of a composition as defined in either of Claims 15 and 16 for body or hair hygiene.

18. Compounds according to any one of Claims 1 to 14, as medicaments.

19. Use of a compound according to any one of Claims 1 to 14 in the manufacture of a composition for regulating and/or restoring skin lipid metabolism.

20. Use of a compound according to any one of Claims 1 to 14 in the manufacture of a composition for treating:
dermatological complaints associated with a keratinization disorder relating to differentiation and to proliferation, in particular for treating common acne, comedones, polymorphs, rosacea, nodulocystic acne, acne conglobata, senile acne and secondary acne such as solar, medicinal or occupational acne,
- ichthyosis, ichthyosiform conditions, Darter's disease, palmoplantar keratoderma, leukoplakia and leukoplakiform conditions, and cutaneous or mucous (oral) lichen,
- dermatological complaints with an inflammatory immuno-allergic component, with or without a cellular proliferation disorder, and in particular cutaneous, mucous or ungual psoriasis, psoriatic arthritis, cutaneous atopy such as eczema, or respiratory atopy or gingival hypertrophy,
- benign or malignant dermal or epidermal proliferations, of viral or nonviral origin, in particular common warts, flat warts and epidermodysplasia verruciformis, oral or florid papillomatoses, T lymphoma,
- proliferations which may be induced by ultraviolet light, in particular basal cell and spinocellular epithelioma,
- precancerous skin lesions, especially keratoacanthomas,
- immune dermatitides, especially lupus erythematosus,
- bullous immune diseases,
- collagen diseases, especially scleroderma,
- dermatological or systemic complaints with an immunological component,
- skin disorders due to exposure to UV radiation, ageing of the skin, whether light-induced or chronological ageing, or actinic keratoses and pigmentations, or any pathology associated with chronological or actinic ageing, especially xerosis,
- sebaceous function disorders, especially the hyperseborrhoea of acne or simple seborrhoea,
- cicatrization disorders or stretch marks,
- pigmentation disorders, such as hyperpigmentation, melasma, hypopigmentation or vitiligo,
- lipid metabolism complaints, such as obesity, hyperlipidaemia or non-insulin-dependent diabetes,
- inflammatory complaints such as arthritis,
- cancerous or precancerous conditions,
- alopecia of various origins, especially alopecia caused by chemotherapy or radiation,
- immune system disorders, such as asthma, type I sugar diabetes, multiple sclerosis or other selective dysfunctions of the immune system,
- complaints of the cardiovascular system, such as arteriosclerosis or hypertension.

21. Pharmaceutical composition, **characterized in that** it comprises, in a physiologically acceptable support, at least one of the compounds as defined in any one of Claims 1 to 14.

22. Composition according to Claim 21, **characterized in that** the concentration of compound(s) according to one of Claims 1 to 14 is between 0.001% and 10% by weight relative to the total weight of the composition.

23. Composition according to Claim 22, **characterized in that** the concentration of compound(s) according to one of Claims 1 to 14 is between 0.01% and 1% by weight relative to the total weight of the composition.

## Patentansprüche

1. Verbindungen, **dadurch gekennzeichnet, dass** sie der folgenden Formel (I) entsprechen: 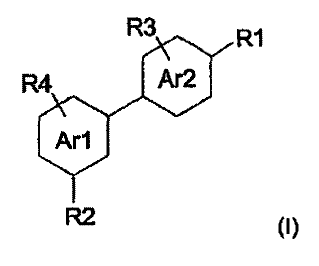 worin bedeuten:
· R1 ist eine Gruppe der folgenden Formeln (a) oder (b): 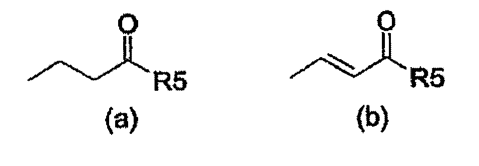 wobei R5 die unten angegebenen Bedeutungen aufweist,
· R2 bedeutet eine Gruppe der Formel (CH₂)ₘ-NR₆-CQ-(NH)ₙR₇; wobei Q, R₆, R₇, m and n die unten angegebenen Bedeutungen aufweisen,
· R3 und R4, die gleich oder verschieden sein können, bedeuten ein Wasserstoffatom, ein Halogenatom, eine lineare oder cyclische Alkylgruppe mit 1 bis 12 Kohlenstoffatomen, die durch Sauerstoff, Fluor oder Stickstoff unterbrochen sein kann, eine Hydroxygruppe, eine Alkoxygruppe mit 1 bis 7 Kohlenstoffatomen, eine Polyethergruppe, eine Aralkylgruppe oder eine Aryloxygruppe;
· R5 bedeutet eine Hydroxygruppe, eine Gruppe 0R8 oder eine Hydroxylamingruppe; wobei R8 nachstehend definiert ist,
· R6 bedeutet eine niedere Alkylgruppe mit 1 bis 4 Kohlenstoffatomen;
· R7 bedeutet eine Alkylgruppe mit 1 bis 12 Kohlenstoffatomen, eine Arylgruppe, eine Aralkylgruppe, eine Heteroarylgruppe oder eine heterocyclische Gruppe;
· R8 bedeutet eine Alkylgruppe, eine Arylgruppe oder eine Aralkylgruppe;
· m und n können die Werte 0 oder 1 annehmen;
· Q bedeutet ein Sauerstoff- oder Schwefelatom;
· Ar 1 und Ar2 können gleich oder verschieden sein und eine aromatische Gruppe der folgenden Formel bedeuten: 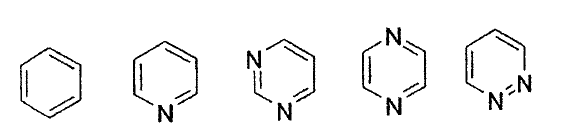 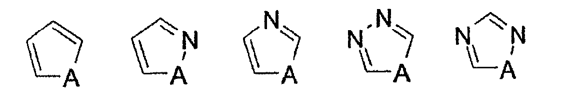 welche, wenn es sich um eine Arylgruppe handelt, ein- oder mehrfach substituiert sein kann mit einem Halogenatom, einer Gruppe CF₃, einer Alkylgruppe mit 1 bis 12 Kohlenstoffatomen, einer Alkoxygruppe mit 1 bis 7 Kohlenstoffatomen, einer Nitrofunktion, einer Polyethergruppe, einer Arylgruppe, einer Benzoylgruppe, einer Alkylestergruppe, einer Carbonsäure, einer Hydroxygruppe, die gegebenenfalls mit einer Acetyl- oder Benzoylgruppe geschützt ist, oder einer Aminogruppe, die gegebenenfalls mit einer Acetyl- oder Benzoylgruppe geschützt ist oder gegebenenfalls mit mindestens einer Alkylgruppe mit 1 bis 12 Kohlenstoffatomen substituiert ist; und welche, sie wenn es sich um eine Heteroarylgruppe handelt, gegebenenfalls substituiert ist mit mindestens einem Halogenatom, einer Alkylgruppe mit 1 bis 12 Kohlenstoffatomen, einer Alkoxygruppe mit 1 bis 7 Kohlenstoffatomen, einer Arylgruppe, einer Nitrofunktion, einer Polyethergruppe, einer Heteroarylgruppe, einer Benzoylgruppe, einer Alkylestergruppe, einer Carbonsäure, einer Hydroxygruppe, die gegebenenfalls mit einer Acetyl- oder Benzoylgruppe geschützt ist, oder einer Aminofunktion, die gegebenenfalls mit einer Acetyl- oder Benzoylgruppe geschützt ist oder gegebenenfalls mit mindestens einer Alkylgruppe mit 1 bis 12 Kohlenstoffatomen substituiert ist,
· A bedeutet ein Schwefelatom, ein Sauerstoffatom oder eine Gruppe N-R9;
· R9 bedeutet ein Wasserstoffatom oder eine Alkylgruppe mit 1 bis 12 Kohlenstoffatomen;
mit der Maßgabe, dass Ar2 oder Ar1 zwingend eine Heteroarylgruppe bedeutet, wenn Ar1 oder Ar2 eine Arylgruppe ist,
und die optischen und geometrischen Isomere der Verbindungen der Formel (I).

2. Verbindungen nach Anspruch 1, **dadurch gekennzeichnet, dass** sie in der Form von Salzen eines Alkalimetalls oder eines Erdalkalimetalls oder Salzen eines organischen Amins vorliegen.

3. Verbindungen nach Anspruch 1 oder 2, **dadurch gekennzeichnet, dass** das Halogenatom unter Fluor, Chlor und Brom ausgewählt ist.

4. Verbindungen nach Anspruch 1 oder 2, **dadurch gekennzeichnet, dass** die Alkylgruppe mit 1 bis 12 Kohlenstoffatomen unter den Gruppen Methyl, Ethyl, Isopropyl, Butyl, Isobutyl, tert-Butyl, Hexyl, Heptyl, Octyl, Decyl, Cyclopentyl, Cyclohexyl und Methylencyclopropyl ausgewählt ist.

5. Verbindungen nach Anspruch 1 oder 2, **dadurch gekennzeichnet, dass** die niedere Alkylgruppe mit 1 bis 4 Kohlenstoffatomen unter den Gruppen Methyl, Ethyl, n-Propyl, Isopropyl, Cyclopropyl, Methylcyclopropyl, n-Butyl, Isobutyl und tert-Butyl ausgewählt ist.

6. Verbindungen nach Anspruch 1 oder 2, **dadurch gekennzeichnet, dass** die Alkoxygruppe mit 1 bis 7 Kohlenstoffatomen unter den Gruppen Methoxy, Ethoxy, Isopropyloxy, Methylcyclopropyloxy, Isobutoxy, tert-Butoxy, Pentyloxy, Isopentyloxy, Hexyloxy, Heptyloxy, Benzyloxy, Aryloxy oder Phenoxy ausgewählt ist, die gegebenenfalls mit einer Alkylgruppe mit 1 bis 12 Kohlenstoffatomen oder einer Alkoxygruppe mit 1 bis 5 Kohlenstoffatomen substituiert sein kann.

7. Verbindungen nach Anspruch 1 oder 2, **dadurch gekennzeichnet, dass** die Polyethergruppe unter den Gruppen Methoxymethoxy, Ethoxymethoxy und Methoxyethoxymethoxy ausgewählt ist.

8. Verbindungen nach Anspruch 1 oder 2, **dadurch gekennzeichnet, dass** die Aralkylgruppe unter den Gruppen Benzyl, Phenethyl oder 2-Naphthylmethyl ausgewählt ist, die ein- oder zweifach substituiert ist mit einem Halogenatom, einer Gruppe CF₃, einer Alkylgruppe mit 1 bis 12 Kohlenstoffatomen, einer Alkoxygruppe mit 1 bis 7 Kohlenstoffatomen, einer Nitrofunktion, einer Polyethergruppe, einer Arylgruppe, einer Benzoylgruppe, einer Alkylestergruppe, einer Carbonsäure, einer Hydroxygruppe, die gegebenenfalls mit einer Acetyl- oder Benzoylgruppe geschützt ist, oder einer Aminogruppe, die gegebenenfalls mit einer Acetyl- oder Benzoylgruppe geschützt ist oder gegebenenfalls mit mindestens einer Alkylgruppe mit 1 bis 12 Kohlenstoffatomen substituiert ist.

9. Verbindungen nach Anspruch 1 oder 2, **dadurch gekennzeichnet, dass** die Arylgruppe unter den Gruppen Phenyl, Biphenyl, Cinnamyl oder Naphthyl ausgewählt ist, die ein- oder zweifach substituiert ist mit einem Halogenatom, einer Gruppe CF₃, einer Alkylgruppe mit 1 bis 12 Kohlenstoffatomen, einer Alkoxygruppe mit 1 bis 7 Kohlenstoffatomen, einer Nitrofunktion, einer Polyethergruppe, einer Arylgruppe, einer Benzoylgruppe, einer Alkylestergruppe, einer Carbonsäure, einer Hydroxygruppe, die gegebenenfalls mit einer Acetyl- oder Benzoylgruppe geschützt ist, oder einer Aminogruppe, die gegebenenfalls mit einer Acetyl- oder Benzoylgruppe geschützt ist oder gegebenenfalls mit mindestens einer Alkylgruppe mit 1 bis 12 Kohlenstoffatomen substituiert ist.

10. Verbindungen nach Anspruch 1 oder 2, **dadurch gekennzeichnet, dass** die Heteroarylgruppe unter den Gruppen Pyridyl, Furyl, Thienyl, Isoxazolyl, Oxadiazolyl, Oxazolyl, Isothiazolyl, Chinazolinyl, Benzothiadiazolyl, Benzimidazolyl, Chinoxalyl, Indolyl oder Benzofuryl ausgewählt ist, die gegebenenfalls substituiert sind mit mindestens einem Halogenatom, einer Alkylgruppe mit 1 bis 12 Kohlenstoffatomen, einer Alkoxygruppe mit 1 bis 7 Kohlenstoffatomen, einer Arylgruppe, einer Nitrofunktion, einer Polyethergruppe, einer Heteroarylgruppe, einer Benzoylgruppe, einer Alkylestergruppe, einer Carbonsäure, einer Hydroxygruppe, die gegebenenfalls mit einer Acetyl- oder Benzoylgruppe geschützt ist, oder einer Aminogruppe, die gegebenenfalls mit einer Acetyl- oder Benzoylgruppe geschützt ist oder gegebenenfalls mit mindestens einer Alkylgruppe mit 1 bis 12 Kohlenstoffatomen substituiert ist.

11. Verbindungen nach Anspruch 1 oder 2, **dadurch gekennzeichnet, dass** die heterocyclische Gruppe unter den Gruppen Morpholino, Piperidino, Piperazino, 2-Oxo-1-piperidyl oder 2-Oxo-1-pyrrolidinyl ausgewählt ist, die gegebenenfalls substituiert sind mit einer Alkylgruppe mit 1 bis 12 Kohlenstoffatomen oder einer Alkoxygruppe mit 1 bis 7 Kohlenstoffatomen, einer Arylgruppe, einer Nitrofunktion, einer Polyethergruppe, einer Heteroarylgruppe, einer Benzoylgruppe, einer Alkylestergruppe, einer Carbonsäure, einer Hydroxygruppe, die gegebenenfalls mit einer Acetyl- oder Benzoylgruppe geschützt ist, oder einer Aminogruppe, die gegebenenfalls mit einer Acetyl- oder Benzoylgruppe geschützt ist oder gegebenenfalls mit mindestens einer Alkylgruppe mit 1 bis 12 Kohlenstoffatomen substituiert ist.

12. Verbindungen nach Anspruch 1, **dadurch gekennzeichnet, dass** sie einzeln oder in Form von Gemischen ausgewählt sind unter:
1. 3-(5-{3-[(Methyloctanoylamino)methyl]phenyl}thiophen-2-yl)-acrylsäure
2. 3-(5-{3-[(Methyloctanoylamino)methyl]phenyl}-thiophen-2-yl)propansäure
3. 3-(5-{3-[(Methyloctanoylamino)methyl]phenyl}furan-2-yl)-acrylsäure
4. 3-(4-{5-[(Methyloctanoylamino)methyl]-thiophen-3-yl}phenyl) acrylsäure
5. 3-(4-{5-[(Methyloctanoylamino)methyl-thiophen-3-yl}phenyl)-propansäure
6. 3-{4-[6-(3-Heptyl-1-methylureido)pyrid-2-yl]phenyl)-propansäure
7. 3-{ 6-[3-(3-Heptyl-1-methylureido)phenyl]pyrid-3-yl}propansäure
8. 2-[4-(2-Carboxyethyl)phenyl]-4-(3-heptyl-1-methyl-ureido)-pyridiniumacetat
9. 3-{5-[3-(3-Pentyl-1-methylureido)phenyl]pyrimidin-2-yl}-acrylsäure
10. 3-{5-[3-(3-Pentyl-1-methylureido)phenyl]pyrimidin-2-yl}-propansäure
11. 2-[2-Butoxy-4-(2-carboxyethyl)phenyl]-6-(1-methyl-3-pentylureido)pyridinium-Hydrochlorid
12. 2-[2-Butoxy-4-(2-carboxyethyl)phenyl]-6-(1-methyl-3-heptylureido)pyridinium-Hydrochlorid
13. 2-[4-(2-Carboxyethyl)-2-ethoxyphenyl]-6-(3-heptyl-1-methylureido)pyridinium-Hydrochlorid
14. 3-(3-Butoxy-4-{5-[(methyloctanoylamino)methyl]-thiophen-3-yl}phenyl)propansäure
15. 3-(3-Butoxy-4-(5-{[(4-methoxybenzoyl)methylamino]-methyl}-thiophen-3-yl)phenyl]propansäure
16. 3-(3-Butoxy-4-(5-{[(3-methoxybenzoyl)methylamino]-methyl}-thiophen-3-yl)phenyl]propansäure
17. 3-{5-[4-(3-Heptyl-1-methylureido)-pyrid-2-yl] furan-2-yl}-propansäure
18. 3-[5-[3-(3-Heptyl-1-methylureido)phenyl]-4-(2,2,2-trifluor-ethoxy)-furan-2-yl]propansäure
19. 3-(5-{3-[3-(3, 5-Dimethoxyphenyl)-1-ethylureido]-phenyl}-3-methylfuran-2-yl)propansäure
20. 3-(5-{6-[3-(4-Ethoxyphenyl)-1-ethylureido]pyrid-2-yl}furan-2-yl)propansäure
21. 3-(2-Methyl-4-{6-[1-methyl-3-(4-methylpentyl)ureido]pyrid-2-yl}phenyl)propansäure
22. Methyl-3-{4-[6-(1-ethyl-3-naphthalin-2-ylureido)-pyrid-2-yl]-2-fluorphenyl}propanoat
23. 3-(4-{6-[3-(4-Butoxyphenyl)-1-methylureido]pyrid-2-yl}phenyl)-N-hydroxypropionamid
24. 3-{3-Cyclopropylmethoxy-4-[6-(1-methyl-3-pentylureido)pyrid-2-yl]phenyl}propansäure
25. 3-{4-[6-(1-Ethyl-3-phenylthioureido)pyrid-2-yl]-3-propoxyphenyl}propansäure
26. 3-{3-Ethoxy-4-[6-(3-heptyl-1-methylureido)pyrid-2-yl]phenyl}propansäure
27. 3-{4-[6-(3-Hexyl-1-methylureido)pyrid-2-yl]-3-isopropoxyphenyl}propansäure
28. 3-[4-[6-(3-Heptyl-1-methylureido)pyrid-2-yl]-3-(4,4, 4-trifluorbutoxy)phenyl]propansäure
29. 3-{3-(2-Dimethylaminoethoxy)-4-[6-(1-methyl-3-pentylureido)pyrid-2-yl]phenyl}propansäure
30. 3-{3-(3-Hydroxypropoxy)-4-[6-(1-methyl-3-pentylureido)pyrid-2-yl]phenyllpropansäure
31. 3-{4-[6-(1-Ethyl-3-phenylthioureido)pyrid-2-yl]-3-fluorphenyl}acrylsäure
32. 3-{4-[6-(3-Hexyl-1-methylureido)pyrid-2-yl]-3-trifluormethylphenyl}propansäure
33. 3-[6'-(1-Methyl-3-phenethylureido)-[2,2']bipyridinyl-5-yl]-propansäure
34. 3-{2-[6-(3-Hexyl-1-methylureido)pyrid-2-yl] thiazol-4-yl}-propansäure
35. Ethyl-3-{2-[6-(3-hexyl-1-methylureido)pyrid-2-yl]-thiazol-5-yl}propanoat
36. 3-(3-{ 6-[1-Methyl-3-(6-methylheptyl)ureido]pyrid-2-yl}isoxazol-5-yl)propansäure
37. 3-{4-[2-(3-Heptyl-1-methylureido)pyrimidin-4-yl]phenyl}-propansäure
38. 3-{3-Cyclopropylmethoxy-4-[2-(3-heptyl-1-methylureido)-pyrimidin-4-yl]phenyl}propansäure
39. 3-(4-{2-[1-Ethyl-3-(4-propoxyphenyl)ureido]-pyrimidin-4-yl}-3-fluorphenyl)propansäure
40. 3-{2-Fluor-4-[2-(1-methyl-3-naphthalin-2-ylureido)pyrid-4-yl]phenyl}-N-hydroxypropionamid
41. 3-[2'-(3-Hexyl-1-methylthioureido) [2, 4']-bipyridinyl-5-yl]propansäure
42. 3-{4-[2-(3-Hexyl-1-methylureido)pyrid-4-yl]-3-propoxyphenyl}propansäure
43. 3-(3-Benzyloxy-4-{4-[1-methyl-3-(5-methylhexyl) ureido]pyrid-2-yl}phenyl)propansäure
44. 3-{2-[4-(3-Hexyl-1-methylureido)-pyrid-2-yl]thiazol-5-yl}-acrylsäure
45. 3-{5-[3-(3-Hexyl-1-methylureido)-phenyl]pyrid-2-yl}-acrylsäure
46. 3-{5-[5-(3-Heptyl-1-methylthioureido)thiophen-3-yl]pyrid-2-yl}acrylsäure
47. 3-{4-[2-(3-Heptyl-1-methylureido)-thiazol-4-yl]-3-propoxy-phenyl}propansäure
48. 3-(2-Fluor-4-{ 5-[(heptanoylmethylamino) methyl] thiophen-3-yl}phenyl)propansäure
49. 3-(4-{5-[(Heptanoylmethylamino)methyl]-thiophen-3-yl }-3-isobutoxyphenyl)acrylsäure
50. 3-(3-(2-Cyclopentylethoxy)-4-{5-[(methyloctanoylamino)-methyl]-thiophen-3-yl}phenyl)propansäure
51. Methyl-3-(3-isobutoxy-4-{5-[(methylnonanoylamino)-methyl] thiophen-3-yl}phenyl)propanoat
52. 3-{6-[5-({Ethyl-[2-(2-pentylphenyl)acetyl]amino}-methyl)thiophen-3-yl]pyrid-3-yl}propansäure
53. 3-(4-{4-[(Methylnonanoylamino)methyl]thiazol-2-yl}-3-propoxyphenyl)propansäure
54. 3-(2-Chlor-4-{4-[(methylnonanoylamino)methyl]-thiophen-2-yl}phenyl)propansäure
55. 3-(2-Fluor-4-{4-[(methylnonanoylamino)methyl]-thiophen-2-yl}phenyl)acrylsäure
56. 3-(4-{4-[(Heptanoylmethylamino)methyl]-thiophen-2-yl}-1-methyl-1H-pyrrol-2-yl)propansäure
57. 3-(4-{4-[(Heptanoylmethylamino)methyl]-thiophen-2-yl}furan-2-yl)propansäure
58. 3-{5'-[(Heptanoylmethylamino)methyl]-[3,3']bithiophenyl-5-yl}propansäure
59. Phenyl-3-{5'-[(heptanoylmethylamino)methyl]-3-propyl [2,3']bithiophenyl-5-yl}propanoat
60. 3-(5-{5-[(Heptanoylmethylamino)methyl]-thiophen-3-yl}-4-propylfuran-2-yl)acrylsäure
61. 3-{3-Butoxy-4-[6-(3-heptyl-1-methylureido)pyrid-2-yl]phenyl}propansäure
62. 3-{3-Benzyloxy-4-[6-(3-heptyl-1-methylureido)pyrid-2-yl]phenyl}propansäure
63. 3-{3-Benzyloxy-4-[2-(3-heptyl-1-methylureido)pyrimidin-4-yl]phenyl}propansäure
64. 3-{3-Butyloxy-4-[2-(3-heptyl-1-methylureido)pyrimidin-4-yl]phenyl}propansäure
65. 3-{3-Butoxy-4-[4-(3-heptyl-1-methylureido)pyrid-2-yl]phenyl}propansäure
66. 3-{3-Benzyloxy-4-[4-(3-heptyl-1-methylureido)pyrid-2-yl]phenyl}propansäure
67. 3-{3-Benzyloxy-4-[5-(3-heptyl-1-methylureido)pyrid-3-yl]phenyl}propansäure
68. 3-{3-Butoxy-4-[5-(3-heptyl-1-methylureido)pyrid-3-yl]phenyl}propansäure
69. 3-(5-{3-[(Methyloctanoylamino)methyl]phenyl}-4-propylthiophen-2-yl)propansäure
70. 3-(3-Benzyloxy-4-{5-[(methyloctanoylamino)methyl]-thiophen-3-yl}phenyl}propansäure
71. 3-(4-Benzyl-5-{3-[(hexanoylmethylamino)methyl]-phenyl}thiophen-2-yl)propansäure
72. 3-{4-Cyclopropylmethyl-5-[3-(1-methyl-3-pentylureido)phenyl] thiophen-2-yl}propansäure
73. 3-{5-[3-(1-Methyl-3-pentylureido)-4-trifluormethylphenyl]thiophen-2-yl}propansäure
74. 3-(5-{3-[3-(4-Butoxyphenyl)-1-ethylureido]phenyl}-thiophen-2-yl)propansäure
75. 3-{5-[3-(3-Heptyl-1-methylureido)-4-trifluormethylphenyl]-furan-2-yl}propansäure
76. 3-{2-Butoxy-4-[6-(1-methyl-3-pentylureido)pyrid-2-yl]-phenyl)propansäure
77. 3-{2-(4-Methoxybenzyloxy)-4-[6-(1-methyl-3-pentyl-ureido)-pyrid-2-yl]phenyl}propansäure
78. 3-{2-(3-Methoxybenzyloxy)-4-[6-(1-methyl-3-pentyl-ureido)pyrid-2-yl]phenyl}propansäure
79. 3-[2-Cyclopropylmethoxy-4-(6-{3-[2-(4-methoxyphenyl)-ethyl]-1-methylureido}pyrid-2-yl)phenyl]propansäure.

13. Verbindungen nach Anspruch 1 oder 2, **dadurch gekennzeichnet, dass** sei mindestens einer der folgenden Eigenschaften aufweisen:
· R3 ist eine Alkoxygruppe mit 1 bis 7 Kohlenstoffatomen;
· R5 entspricht einer Hydroxygruppe;
· die Sequenz -(CH2)ₘ-NR₆-CQ(NH)ₙR₇ weist m = 0, n = 1 auf;
· Q ist ein Sauerstoffatom;
· R7 bedeutet eine Alkylgruppe mit 1 bis 8 Kohlenstoffatomen;
· zumindest Ar1 oder Ar2 ist eine Gruppe vom Pyridintyp.

14. Verbindungen nach Anspruch 13, **dadurch gekennzeichnet, dass** sie alle folgenden Eigenschaften aufweisen:
· R3 ist eine Alkoxygruppe mit 1 bis 7 Kohlenstoffatomen;
· R5 entspricht einer Hydroxygruppe;
· die Sequenz -(CH2)ₘ-NR₆-CQ(NH)ₙR₇ weist m = 0, n = 1 auf;
· Q ist ein Sauerstoffatom;
· R7 bedeutet eine Alkylgruppe mit 1 bis 8 Kohlenstoffatomen;
· zumindest Ar1 oder Ar2 ist eine Gruppe vom Pyridintyp.

15. Kosmetische Zusammensetzung, **dadurch gekennzeichnet, dass** sie in einem physiologisch akzeptablen Medium mindestens eine Verbindung enthält, wie sie in einem der Ansprüche 1 bis 14 definiert ist.

16. Zusammensetzung nach Anspruch 15, **dadurch gekennzeichnet, dass** die Konzentration der Verbindung(en) nach einem der Ansprüche 1 bis 14 im Bereich von 0,0001 bis 2 Gew.-%, bezogen auf das Gesamtgewicht der Zusammensetzung, liegt.

17. Kosmetische Verwendung einer Zusammensetzung, wie sie in Anspruch 15 oder 16 definiert ist, für die Körper- oder Haarpflege.

18. Verbindungen nach einem der Ansprüche 1 bis 14 als Arzneimittel.

19. Verwendung einer Verbindung nach einem der Ansprüche 1 bis 14 für die Herstellung einer Zusammensetzung zur Regulierung und/oder Wiederherstellung des Lipidmetabolismus der Haut.

20. Verwendung einer Verbindung nach einem der Ansprüche 1 bis 14 für die Herstellung einer Zusammensetzung zur Behandlung von:
- dermatologischen Erkrankungen, die mit einer Verhornungsstörung verbunden sind, die auf der Differenzierung und Proliferation der Zellen beruht, insbesondere zur Behandlung von Acne vulgaris, Acne comedonica, polymorpher Akne, Acne rosaceae, nodulocystischer Akne, Acne conglobata, Acne senilis, sekundären Akneformen wie Acne solaris, Acne medicamentosa oder Acne professionalis;
- Ichthyosis und ichthyosiformen Zuständen, der Darier-Krankheit, Palmoplantarkeratosen, Leukoplakien und leukoplasiformen Zuständen und Lichen der Haut oder der Schleimhäute (oral),
- dermatologischen Erkrankungen mit entzündlicher immunoallergischer Komponente, mit oder ohne Störungen der Zellproliferation, insbesondere aller Arten von Psoriasis der Haut, der Schleimhäute oder der Nägel, Psoriasis-Arthritis, Atopie der Haut, wie Ekzemen, respiratorischer Atopie oder gingivaler Hypertrophie,
- dermalen oder epidermalen Proliferationen, die gutartig oder bösartig und gegebenenfalls viralen Ursprungs sein können, insbesondere Verruca vulgaris, Verruca plana und verruciforme epidermale Dysplasie, orale oder floride Papillomatosis, T-Lymphomen,
- Proliferationen, die durch UV-Strahlung induziert sein können, insbesondere Epithelioma basocellulare und spinocellulare,
- präkanzerösen Hautläsionen, insbesondere Keratoakanthomen,
- Immundermatosen, insbesondere Lupus erythematodes,
- bullösen Immunerkrankungen,
- Collagenerkrankungen, insbesondere Sklerodermie,
- dermatologischen oder allgemeinen Erkrankungen mit immunologischer Komponente,
- Hautstörungen, die mit einer UV-Exposition zusammenhängen, Hautalterung, die lichtinduziert oder chronologisch sein kann, oder aktinischen Keratosen und Pigmentierungen oder beliebigen Erkrankungen, die mit einer altersbedingten oder aktinischen Alterung zusammenhängen, wie Xerose,
- Funktionsstörungen der Talgdrüsen, insbesondere hyperseborrhoeischer Akne oder Seborrhoe simplex,
- Wundheilungsstörungen oder Streifen,
- Pigmentierungsstörungen, wie Hyperpigmentierung, Melasmen, Hypopigmentierung oder Vitiligo,
- Erkrankungen des Lipidmetabolismus, wie Adipositas, Hyperlipidämie oder nicht-insulinpflichtigem Diabetes,
- entzündlichen Störungen, wie Arthritis,
- kanzerösen oder präkanzerösen Zuständen,
- Alopezie unterschiedlicher Ursache, insbesondere durch Chemotherapie oder Strahlung verursachter Alopezie,
- Störungen des Immunsystems, wie Asthma, Diabetes Typ I, multipler Sklerose oder anderen selektiven Funktionsstörungen des Immunsystems, oder
- Erkrankungen des kardiovaskulären Systems, wie Arteriosklerose oder Bluthochdruck.

21. Pharmazeutische Zusammensetzung, **dadurch gekennzeichnet, dass** sie in einem physiologisch akzeptablen Träger mindestens eine Verbindung enthält, wie sie in einem der Ansprüche 1 bis 14 definiert ist.

22. Zusammensetzung nach Anspruch 21, **dadurch gekennzeichnet, dass** die Konzentration der Verbindung(en) nach einem der Ansprüche 1 bis 14 im Bereich von 0,001 bis 10 Gew.-%, bezogen auf das Gesamtgewicht der Zusammensetzung, liegt.

23. Zusammensetzung nach Anspruch 22, **dadurch gekennzeichnet, dass** die Konzentration der Verbindung(en) nach einem der Ansprüche 1 bis 14 im Bereich von 0,01 bis 1 Gew.-%, bezogen auf das Gesamtgewicht der Zusammensetzung, liegt.

## Revendications

1. Composés **caractérisés en ce qu'**ils correspondent à la formule (I) ci-dessous : 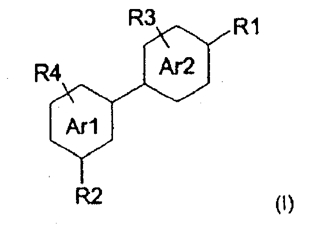 dans laquelle :
- **R1** représente un radical de formule (a) ou (b) ci-dessous : 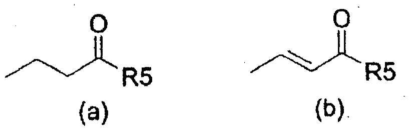 R5 ayant les significations données ci-dessous,
- **R2** représente un radical de formule (CH₂)ₘ-NR₆-CQ-(NH)ₙR₇ ; Q, R6, R7, m et n ayant les significations données ci-dessous,
- **R3** et **R4,** qui peuvent être identiques ou différents, représentent un atome d'hydrogène, un atome d'halogène, un radical alkyle linéaire ou cyclique de 1 à 12 atomes de carbone qui peuvent être interrompus par des atomes d'oxygène, de fluor ou d'azote, un radical hydroxy, un radical alcoxy contenant de 1 à 7 atomes de carbone, un radical polyéther, un radical aralkyle ou un radical aryloxy ;
- **R5** représente un radical hydroxy, un radical OR8 ou un radical hydroxylamine ;
R8 étant défini ci-dessous,
- **R6** représente un radical alkyle inférieur contenant de 1 à 4 atomes de carbone ;
- **R7** représente un radical alkyle contenant de 1 à 12 atomes de carbone, un radical aryle, un radical aralkyle, un radical hétéroaryle ou un radical hétérocyclique ;
- **R8** représente un radical alkyle, aryle ou aralkyle ;
- **m** et **n** peuvent prendre les valeurs 0 ou 1 ;
- **Q** représente un atome d'oxygène ou de souffre ;
- **Ar1** et **Ar2** peuvent être identiques ou différents et représentent un radical aromatique de formule : 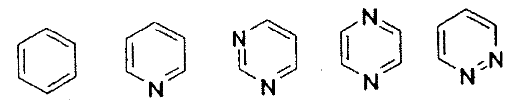 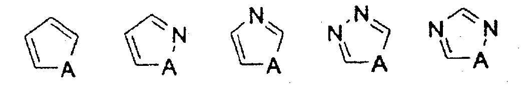
qui, lorsqu'il s'agit d'un radical aryle, peut être mono- ou disubstitué par un atome d'halogène, un radical CF₃, un radical alkyle contenant de 1 à 12 atomes de carbone, un radical alcoxy contenant de 1 à 7 atomes de carbone, une fonction nitro, un radical polyéther, un radical aryle, un radical benzoyle, un radical alkyl-ester, un acide carboxylique, un radical hydroxy éventuellement protégé par un groupement acétyle ou benzoyle ou une fonction amino protégée éventuellement par un groupement acétyle ou benzoyle ou éventuellement substituée par au moins un alkyle contenant de 1 à 12 atomes de carbone ;
et qui, lorsqu'il s'agit d'un radical hétéroaryle, est éventuellement substitué par au moins un halogène, un alkyle contenant de 1 à 12 atomes de carbone, un alcoxy contenant de 1 à 7 atomes de carbone, un radical aryle, une fonction nitro, un radical polyéther, un radical hétéroaryle, un radical benzoyle, un groupement alkyl-ester, un acide carboxylique, un hydroxy éventuellement protégé par un groupement acétyle ou benzoyle ou une fonction amino éventuellement protégée par un groupement acétyle ou benzoyle ou éventuellement substituée par au moins un alkyle contenant de 1 à 12 atomes de carbone
- **A** représente un atome de soufre ou d'oxygène ou un radical N-R9 ;
- **R9** représente un atome d'hydrogène ou un radical alkyle contenant de 1 à 12 atomes de carbone ;
étant entendu que lorsque Ar1 ou Ar2 est un radical aryle, alors Ar2 ou Ar1 est nécessairement un radical hétéroaryle,
et les isomères optiques et géométriques desdits composés de formule (I).

2. Composés selon la revendication 1, **caractérisés en ce qu'**ils sont sous forme de sels d'un métal alcalin ou d'un métal alcalino-terreux ou de sels d'une amine organique.

3. Composés selon l'une ou l'autre des revendications 1 et 2, **caractérisés en ce que** l'atome d'halogène est choisi parmi les atomes de fluor, de chlore et de brome.

4. Composés selon l'une ou l'autre des revendications 1 et 2, **caractérisés en ce que** le radical alkyle contenant de 1 à 12 atomes de carbone est choisi parmi les radicaux méthyle, éthyle, isopropyle, butyle, isobutyle, tert-butyle, hexyle, heptyle, octyle, décyle, cyclopentyle, cyclohexyle et méthylènecyclopropyle.

5. Composés selon l'une ou l'autre des revendications 1 et 2, **caractérisés en ce que** le radical alkyle inférieur contenant de 1 à 4 atomes de carbone est choisi parmi les radicaux méthyle, éthyle, n-propyle, i-propyle, c-propyle, méthylcyclopropyle, n-butyle, i-butyle et t-butyle.

6. Composés selon l'une ou l'autre des revendications 1 et 2, **caractérisés en ce que** le radical alcoxy contenant de 1 à 7 atomes de carbone est choisi parmi les radicaux méthoxy, éthoxy, isopropyloxy, méthylcyclopropyloxy, isobutoxy, tert-butoxy, pentyloxy, isopentyloxy, hexyloxy, heptyloxy, benzyloxy, aryloxy ou phénoxy, qui peuvent être éventuellement substitués par un radical alkyle contenant de 1 à 12 atomes de carbone ou un radical alcoxy contenant de 1 à 5 atomes de carbone.

7. Composés selon l'une ou l'autre des revendications 1 et 2, **caractérisés en ce que** le radical polyéther est choisi parmi les radicaux méthoxyméthoxy, éthoxyméthoxy et méthoxyéthoxyméthoxy.

8. Composés selon l'une ou l'autre des revendications 1 et 2, **caractérisés en ce que** le radical aralkyle est choisi parmi les radicaux benzyle, phénéthyle ou 2-naphtylméthyle, qui peuvent être mono- ou disubstitués avec un atome d'halogène, un radical CF₃, un radical alkyle contenant de 1 à 12 atomes de carbone, un radical alcoxy contenant de 1 à 7 atomes de carbone, une fonction nitro, un radical polyéther, un radical aryle, un radical benzoyle, un groupement alkyl-ester, un acide carboxylique, un radical hydroxy éventuellement protégé par un groupement acétyle ou benzoyle ou une fonction amino éventuellement protégée par un groupement acétyle ou benzoyle ou éventuellement substituée par au moins un alkyle contenant de 1 à 12 atomes de carbone.

9. Composés selon l'une ou l'autre des revendications 1 et 2, **caractérisés en ce que** le radical aryle est choisi parmi les radicaux phényle, biphényle, cinnamyle, ou naphtyle, qui peuvent être mono- ou disubstitués par un atome d'halogène, un radical CF₃, un radical alkyle contenant de 1 à 12 atomes de carbone, un radical alcoxy contenant de 1 à 7 atomes de carbone, une fonction nitro, un radical polyéther, un radical aryle, un radical benzoyle, un groupement alkyl-ester, un acide carboxylique, un radical hydroxy éventuellement protégé par un groupement acétyle ou benzoyle ou une fonction amino éventuellement protégée par un groupement acétyle ou benzoyle ou éventuellement substituée par au moins un alkyle contenant de 1 à 12 atomes de carbone.

10. Composés selon l'une ou l'autre des revendications 1 et 2, **caractérisés en ce que** le radical hétéroaryle est choisi dans le groupe constitué de radicaux pyridyle, furyle, thiényle, isoxazolyle, oxadiazolyle, oxazolyle, isothiazolyle, quinazolinyle, benzothiadiazolyle, benzimidazolyle, quinoxalyle, indolyle ou benzofuryle, éventuellement substitués par au moins un halogène, un alkyle contenant de 1 à 12 atomes de carbone, un alcoxy contenant de 1 à 7 atomes de carbone, un radical aryle, une fonction nitro, un radical polyéther, un radical hétéroaryle, un radical benzoyle, un groupement alkyl-ester, un acide carboxylique, un hydroxy éventuellement protégé par un groupement acétyle ou benzoyle ou une fonction amino éventuellement protégée par un groupement acétyle ou benzoyle ou éventuellement substituée par au moins un alkyle contenant de 1 à 12 atomes de carbone.

11. Composés selon l'une ou l'autre des revendications 1 et 2, **caractérisés en ce que** le radical hétérocyclique est choisi parmi un radical morpholino, pipéridino, pipérazino, 2-oxo-1-pipéridyle ou 2-oxo-1-pyrrolidinyle, éventuellement substitué par au moins un alkyle contenant de 1 à 12 atomes de carbone, un alcoxy contenant de 1 à 7 atomes de carbone, un radical aryle, une fonction nitro, un radical polyéther, un radical hétéroaryle, un radical benzoyle, un groupement alkyl-ester, un acide carboxylique, un hydroxy éventuellement protégé par un groupement acétyle ou benzoyle ou une fonction amino éventuellement protégée par un groupement acétyle ou benzoyle ou éventuellement substituée par au moins un alkyle contenant de 1 à 12 atomes de carbone.

12. Composés selon la revendication 1, **caractérisés en ce qu'**ils sont pris, seuls ou en mélanges, dans le groupe constitué de :
1. acide 3-(5-{3-[(méthyloctanoylamino)méthyl]phényl}thiophèn-2-yl)acrylique
2. acide 3-(5-{3-[(méthyloctanoylamino)méthyl]phényl}-thiophèn-2-yl)propanoïque
3. acide 3-(5-{3-[(méthyloctanoylamino)méthyl]phényl}furan-2-yl)acrylique
4. acide 3-(4-{5-[(méthyloctanoylamino)méthyl]thiophèn-3-yl}phényl)acrylique
5. acide 3-(4-{5-[(méthyloctanoylamino)méthyl]thiophèn-3-yl}phényl)propanoïque
6. acide 3-{4-[6-(3-heptyl-1-méthyluréido)pyrid-2-yl]phényl}propanoïque
7. acide 3-{6-[3-(3-heptyl-1-méthyluréido)phényl]pyrid-3-yl}propanoïque
8. acétate de 2-[4-(2-carboxyéthyl)phényl]-4-(3-heptyl-1-méthyl-uréido)pyridinium
9. acide 3-{5-[3-(3-pentyl-1-méthyluréido)phényl]pyrimidin-2-yl}acrylique
10. acide 3-{5-[3-(3-pentyl-1-méthyluréido)phényl]pyrimidin-2-yl}propanoïque
11. chlorhydrate de 2-[2-butoxy-4-(2-carboxyéthyl)phényl]-6-(1-méthyl-3-pentyluréido)pyridinium
12. chlorhydrate de 2-[2-butoxy-4-(2-carboxyéthyl)phényl)-6-(1-méthyl-3-heptyluréido)pyridinium
13. chlorhydrate de 2-[4-(2-carboxyéthyl)-2-éthoxyphényl]-6-(3-heptyl-1-méthyluréido)pyridinium
14. acide 3-(3-butoxy-4-{5-[(méthyloctanoylamino)méthyl]-thiophèn-3-yl}phényl)propanoïque
15. acide 3-(3-butoxy-4-(5-{[(4-méthoxybenzoyl)méthylamino]-méthyl}thiophèn-3-yl)phényl)propanoïque
16. acide 3-(3-butoxy-4-(5-{[(3-méthoxybenzoyl)méthylamino]-méthyl}thiophèn-3-yl)phényl)propanoïque
17. acide 3-{5-[4-(3-heptyl-1-méthyluréido)pyrid-2-yl]furan-2-yl}propanoïque
18. acide 3-[5-[3-(3-heptyl-1-méthyluréido)phényl]-4-(2,2,2-trifluoroéthoxy)furan-2-yl]propanoïque
19. acide 3-(5-{3-[3-(3,5-diméthoxyphényl)-1-éthyluréido]-phényl}-3-méthylfuran-2-yl)propanoïque
20. acide 3-(5-{6-[3-(4-éthoxyphényl)-1-éthyluréido]pyrid-2-yl}furan-2-yl)propanoïque
21. acide 3-(2-méthyl-4-{6-[1-méthyl-3-(4-méthylpentyl)uréido]pyrid-2-yl}phényl)propanoïque
22. 3-{4-[6-(1-éthyl-3-naphtalèn-2-yluréido)-pyrid-2-yl]-2-fluorophényl}propanoate de méthyle
23. 3-(4-{6-[3-(4-butoxyphényl)-1-méthyluréido]pyrid-2-yl}phényl)-N-hydroxypropionamide
24. acide 3-{3-cyclopropylméthoxy-4-[6-(1-méthyl-3-pentyluréido)pyrid-2-yl]phényl}propanoïque
25. acide 3-{4-[6-(1-éthyl-3-phénylthiouréido)pyrid-2-yl]-3-propoxyphényl}propanoïque
26. acide 3-{3-éthoxy-4-[6-(3-heptyl-1-méthyluréido)pyrid-2-yl]phényl}propanoïque
27. acide 3-{4-[6-(3-hexyl-1-méthyluréido)pyrid-2-yl]-3-isopropoxyphényl}propanoïque
28. acide 3-[4-[6-(3-heptyl-1-méthyluréido)pyrid-2-yl]-3-(4,4,4-trifluorobutoxy)phényl]propanoïque
29. acide 3-{3-(2-diméthylaminoéthoxy)-4-[6-(1-méthyl-3-pentyluréido)pyrid-2-yl]phényl}propanoïque
30. acide 3-{3-(3-hydroxypropoxy)-4-[6-(1-méthyl-3-pentyluréido)pyrid-2-yl]phényl}propanoïque
31. acide 3-{4-[6-(1-éthyl-3-phénylthiouréido)pyrid-2-yl]-3-fluorophényl}acrylique
32. acide 3-{4-[6-(3-hexyl-1-méthyluréido)pyrid-2-yl]-3-trifluorométhylphényl}propanoïque
33. acide 3-[6'-(1-méthyl-3-phénéthyluréido)-[2,2']bipyridinyl-5-yl]propanoïque
34. acide 3-{2-[6-(3-hexyl-1-méthyluréido)pyrid-2-yl]thiazol-4-yl}propanoïque
35. 3-{2-[6-(3-hexyl-1-méthyluréido)pyrid-2-yl]-thiazol-5-yl}propanoate d'éthyle
36. acide 3-(3-{6-[1-méthyl-3-(6-méthylheptyl)uréido]pyrid-2-yl}isoxazol-5-yl)propanoïque
37. acide 3-{4-[2-(3-heptyl-1-méthyluréido)pyrimidin-4-yl]phényl}propanoïque
38. acide 3-{3-cyclopropylméthoxy-4-[2-(3-heptyl-1-méthyluréido)pyrimidin-4-yl]phényl}propanoïque
39. acide 3-(4-{2-[1-éthyl-3-(4-propoxyphényl)uréido]-pyrimidin-4-yl}-3-fluorophényl)propanoïque
40. 3-{2-fluoro-4-[2-(1-méthyl-3-naphtalèn-2-yluréido)pyrid-4-yl]phényl}-N-hydroxypropionamide
41. acide 3-[2'-(3-hexyl-1-méthylthiouréido)[2,4']-bipyridinyl-5-yl]propanoïque
42. acide 3-{4-[2-(3-hexyl-1-méthyluréido)pyrid-4-yl]-3-propoxyphényl}propanoïque
43. acide 3-(3-benzyloxy-4-{4-[1-méthyl-3-(5-méthylhexyl)uréido]pyrid-2-yl}phényl)propanoïque
44. acide 3-{2-[4-(3-hexyl-1-méthyluréido)pyrid-2-yl]thiazol-5-yl}acrylique
45. acide 3-{5-[3-(3-hexyl-1-méthyluréido)phényl]pyrid-2-yl}-acrylique
46. acide 3-{5-[5-(3-heptyl-1-méthylthiouréido)thiophèn-3-yl]pyrid-2-yl}acrylique
47. acide 3-{4-[2-(3-heptyl-1-méthyluréido)thiazol-4-yl]-3-propoxyphényl}propanoïque
48. acide 3-(2-fluoro-4-{5-[(heptanoylméthylamino)méthyl]thiophèn-3-yl}phényl)propanoïque
49. acide 3-(4-{5-[(heptanoylméthylamino)méthyl]thiophèn-3-yl}-3-isobutoxyphényl)acrylique
50. acide 3-(3-(2-cyclopentyléthoxy)-4-{5-[(méthyloctanoylamino)méthyl]thiophèn-3-yl}phényl)propanoïque
51. 3-(3-isobutoxy-4-{5-[(méthylnonanoylamino)-méthyl]thiophèn-3-yl}phényl)propanoate de méthyle
52. acide 3-{6-[5-({éthyl-[2-(2-pentylphényl)acétyl]amino}-méthyl)thiophèn-3-yl]pyrid-3-yl}propanoïque
53. acide 3-(4-{4-[(méthylnonanoylamino)méthyl]thiazol-2-yl}-3-propoxyphényl)propanoïque
54. acide 3-(2-chloro-4-{4-[(méthylnonanoylamino)méthyl]-thiophèn-2-yl}phényl)propanoïque
55. acide 3-(2-fluoro-4-{4-[(méthylnonanoylamino)méthyl]-thiophèn-2-yl}phényl)acrylique
56. acide 3-(4-{4-[(heptanoylméthylamino)méthyl]thiophèn-2-yl}-1-méthyl-1H-pyrrol-2-yl)propanoïque
57. acide 3-(4-{4-[(heptanoylméthylamino)méthyl]thiophèn-2-yl}furan-2-yl)propanoïque
58. acide 3-{5'-[(heptanoylméthylamino)méthyl]-[3,3']bithiophényl-5-yl}propanoïque
59. 3-{5'-[(heptanoylméthylamino)méthyl]-3-propyl[2,3']bithiophényl-5-yl}propanoate de phényle
60. acide 3-(5-{5-[(heptanoylméthylamino)méthyl]thiophèn-3-yl}-4-propylfuran-2-yl)acrylique
61. acide 3-{3-butoxy-4-[6-(3-heptyl-1-méthyluréido)pyrid-2-yl]phényl}propanoïque
62. acide 3-{3-benzyloxy-4-[6-(3-heptyl-1-méthyluréido)pyrid-2-yl]phényl}propanoïque
63. acide 3-{3-benzyloxy-4-[2-(3-heptyl-1-méthyluréido)pyrimidin-4-yl]phényl}propanoïque
64. acide 3-{3-butyloxy-4-[2-(3-heptyl-1-méthyluréido)pyrimidin-4-yl]phényl}propanoïque
65. acide 3-{3-butoxy-4-[4-(3-heptyl-1-méthyluréido)pyrid-2-yl]phényl}propanoïque
66. acide 3-{3-benzyloxy-4-[4-(3-heptyl-1-méthyluréido)pyrid-2-yl]phényl}propanoïque
67. acide 3-{3-benzyloxy-4-[5-(3-heptyl-1-méthyluréido)pyrid-3-yl]phényl}propanoïque
68. acide 3-{3-butoxy-4-[5-(3-heptyl-1-méthyluréido)pyrid-3-yl]phényl}propanoïque
69. acide 3-(5-{3-[(méthyloctanoylamino)méthyl]phényl}-4-propylthiophèn-2-yl)propanoïque
70. acide 3-(3-benzyloxy-4-{5-[(méthyloctanoylamino)méthyl]-thiophèn-3-yl}phényl)propanoïque
71. acide 3-(4-benzyl-5-{3-[(hexanoylméthylamino)méthyl]-phényl}thiophèn-2-yl)propanoïque
72. acide 3-{4-cyclopropylméthyl-5-[3-(1-méthyl-3-pentyluréido)phényl]thiophèn-2-yl}propanoïque
73. acide 3-{5-[3-(1-méthyl-3-pentyluréido)-4-trifluorométhylphényl]thiophèn-2-yl}propanoïque
74. acide 3-(5-{3-[3-(4-butoxyphényl)-1-éthyluréido]phényl}-thiophèn-2-yl)propanoïque
75. acide 3-{5-[3-(3-heptyl-1-méthyluréido)-4-trifluorométhylphényl]furan-2-yl}propanoïque
76. acide 3-{2-butoxy-4-[6-(1-méthyl-3-pentyluréido)pyrid-2-yl]phényl}propanoïque
77. acide 3-{2-(4-méthoxybenzyloxy)-4-[6-(1-méthyl-3-pentyluréido)pyrid-2-yl]phényl}propanoïque
78. acide 3-{2-(3-méthoxybenzyloxy)-4-[6-(1-méthyl-3-pentyluréido)pyrid-2-yl]phényl}propanoïque
79. acide 3-[2-cyclopropylméthoxy-4-(6-{3-[2-(4-méthoxyphényl)-éthyl]-1-méthyluréido}pyrid-2-yl)phényl]propanoïque

13. Composés selon la revendication 1 ou 2, **caractérisés en ce qu'**ils ont au moins l'une des caractéristiques suivantes :
- R3 est un radical alcoxy de 1 à 7 atomes de carbone ;
- R5 correspond à un radical hydroxy ;
- la séquence -(CH₂)ₘ-NR₆-CQ(NH)nR₇ a m = 0 , n = 1 ;
- Q est un atome d'oxygène ;
- R7 représente un radical alkyle de 1 à 8 atomes de carbone ;
- au moins Ar1 ou Ar2 est un groupement de type pyridine.

14. Composés selon la revendication 13, **caractérisés en ce qu'**ils ont toutes les caractéristiques suivantes :
- R3 est un radical alcoxy de 1 à 7 atomes de carbone ;
- R5 correspond à un radical hydroxy ;
- la séquence -(CH₂)ₘ-NR₆-CQ(NH)ₙR₇ a m = 0, n = 1 ;
- Q est un atome d'oxygène ;
- R7 représente un radical alkyle de 1 à 8 atomes de carbone ;
- au moins Ar1 ou Ar2 est un groupement de type pyridine.

15. Composition cosmétique, **caractérisée en ce qu'**elle comprend, dans un support physiologiquement acceptable, au moins l'un des composés définis dans l'une quelconque des revendications 1 à 14.

16. Composition selon la revendication 15, **caractérisée en ce que** la concentration du ou des composés selon une des revendications 1 à 14 est comprise entre 0,0001% et 2% en poids par rapport au poids total de la composition.

17. Utilisation cosmétique d'une composition telle que définie dans l'une ou l'autre des revendications 15 et 16 pour l'hygiène corporelle ou capillaire.

18. Composés selon l'une quelconque des revendications 1 à 14, en tant que médicaments.

19. Utilisation d'un composé selon l'une quelconque des revendications 1 à 14, dans la fabrication d'une composition destinée à réguler et/ou restaurer le métabolisme des lipides cutanés.

20. Utilisation d'un composé selon l'une quelconque des revendications 1 à 14 dans la fabrication d'une composition destinée à traiter :
- les affections dermatologiques associées à un trouble de kératinisation lié à la différenciation et la prolifération, en particulier destinée à traiter l'acné vulgaire, comédonienne, polymorphe, rosacée, l'acné nodulokystique, l'acné conglobata, l'acné sénile et l'acné secondaire telle que l'acné solaire, médicamenteuse ou professionnelle,
- l'ichtyose, les affections ichtyosiformes, la maladie de Darier, la kératodermie palmoplantaire, la leucoplasie et les affections leucoplasiformes, et le lichen cutané ou muqueux (oral),
- les affections dermatologiques ayant une composante immuno-allergique inflammatoire, avec ou sans trouble de prolifération cellulaire, et en particulier le psoriasis cutané, muqueux ou unguéal, l'arthrite psoriasique, l'atopie cutanée telle que l'eczéma ou l'atopie respiratoire ou l'hypertrophie gingivale,
- les proliférations dermiques ou épidermiques bénignes ou malignes, d'origine virale ou non virale, en particulier les verrues vulgaires, les verrues planes et l'épidermodysplasie verruciforme, les papillomatoses orales ou florides, le lymphome T,
- les proliférations qui peuvent être induites par la lumière ultraviolette, en particulier l'épithélioma basocellulaire ou spinocellulaire,
- les lésions cutanées précancéreuses, notamment les kératoacanthomes,
- les dermatites immunes, notamment le lupus érythémateux,
- les maladies immunitaires bulleuses,
- les maladies du collagène, notamment la sclérodermie,
- les affections dermatologiques ou systémiques ayant une composante immunologique,
- les troubles cutanés dus à l'exposition aux radiations UV, au vieillissement de la peau, qu'il soit induit par la lumière ou le vieillissement chronologique, ou les kératoses et les pigmentations actiniques, ou toute pathologie associée au vieillissement chronologique ou actinique, notamment la xérose,
- les troubles de la fonction sébacée, notamment l'hyperséborrhée de l'acné ou la séborrhée simple,
- les troubles de cicatrisation ou les vergetures,
- les troubles de pigmentation, tels que l'hyperpigmentation, le mélasme, l'hypopigmentation ou le vitiligo,
- les affections du métabolisme des lipides, telles que l'obésité, l'hyperlipidémie ou le diabète non insulino-dépendant,
- les affections inflammatoires telles que l'arthrite,
- les affections cancéreuses ou précancéreuses,
- l'alopécie de différentes origines, notamment l'alopécie provoquée par la chimiothérapie ou les radiations,
- les troubles du système immunitaire, tels que l'asthme, le diabète sucré de type I, la sclérose en plaques ou d'autres dysfonctionnements sélectifs du système immunitaire,
- les affections du système cardiovasculaire, telles que l'artériosclérose ou l'hypertension.

21. Composition pharmaceutique, **caractérisée en ce qu'**elle comprend dans un support physiologiquement acceptable, au moins l'un des composés tels que définis dans l'une quelconque des revendications 1 à 14.

22. Composition selon la revendication 21, **caractérisée en ce que** la concentration de composé(s) selon l'une des revendications 1 à 14 est comprise entre 0,001% et 10% en poids par rapport au poids total de la composition.

23. Composition selon la revendication 22, **caractérisée en ce que** la concentration de composé(s) selon l'une des revendications 1 à 14 est comprise entre 0,01% et 1% en poids par rapport au poids total de la composition.
